# EUROPÄISCHE PATENTSCHRIFT

(11) **EP 2 723 743 B1**
(45) Veröffentlichungstag und Bekanntmachung des Hinweises auf die Patenterteilung: **14.08.2019**
(21) Anmeldenummer: 12735235.9
(22) Anmeldetag: 22.06.2012
(51) Int. Cl.: C07D 475/14, A61K 31/525, A61K 41/00, A61P 31/00

(54) **10H-BENZO[G]PTERIDIN-2,4-DION-DERIVATE, VERFAHREN ZUR HERSTELLUNG UND VERWENDUNG DERSELBEN**
10H-BENZO[G]PTERIDINE-2,4-DIONE DERIVATIVES, METHOD FOR THE PRODUCTION THEREOF, AND USE THEREOF
DÉRIVÉS DE 10H-BENZO[G]PTÉRIDINE-2,4-DIONE, PROCÉDÉ DE PRODUCTION ET D'UTILISATION DESDITS DÉRIVÉS

(30) Priorität: 22.06.2011 DE 102011105653
(43) Veröffentlichungstag der Anmeldung: 30.04.2014
(73) Patentinhaber: TriOptoTec GmbH, 93053 Regensburg (DE)
(72) Erfinder: MAISCH, Tim, 90425 Nürnberg (DE); SPÄTH, Andreas, 93073 Neutraubling (DE)
(74) Vertreter: Louis Pöhlau Lohrentz
(86) Internationale Anmeldenummer: PCT/EP2012/062173
(87) Internationale Veröffentlichungsnummer: WO 2012/175730

(56) Entgegenhaltungen:
- WO-A2-00/04930
- DE-A1- 2 224 371
- SEIJI SHINKAI ET AL: "Coenzyme models. Part 23. Formation and reactivity of the stable ?quinone form? of flavin in cationic polymer matrices", JOURNAL OF THE CHEMICAL SOCIETY, PERKIN TRANSACTIONS 1, 1. Januar 1980 (1980-01-01), Seite 1622, XP55034011, ISSN: 0300-922X, DOI: 10.1039/p19800001622
- RON BLONDER ET AL: "Application of a Nitrospiropyran-FAD-Reconstituted Glucose Oxidase and Charged Electron Mediators as Optobioelectronic Assemblies for the Amperometric Transduction of Recorded Optical Signals: Control of the "On"-"Off" Direction of the Photoswitch", JOURNAL OF THE AMERICAN CHEMICAL SOCIETY, Bd. 119, Nr. 49, 1. Dezember 1997 (1997-12-01), Seiten 11747-11757, XP55034326, ISSN: 0002-7863, DOI: 10.1021/ja972663v
- R. B. BARLOW ET AL: "144. A series of 9-dialkylaminoalkylisoalloxazines", JOURNAL OF THE CHEMICAL SOCIETY (RESUMED), 1. Januar 1950 (1950-01-01), Seite 713, XP55034329, ISSN: 0368-1769, DOI: 10.1039/jr9500000713
- DONALD B. MCCORMICK ET AL: "Syntheses of 8-bromo-5'-adenylate-containing nucleotides", JOURNAL OF MEDICINAL CHEMISTRY, Bd. 12, Nr. 2, 1. März 1969 (1969-03-01), Seiten 333-334, XP55034331, ISSN: 0022-2623, DOI: 10.1021/jm00302a037
- TAM-CHANG S-W ET AL: "Synthesis of Symmetrical and Unsymmetrical Alkyl Disulfides with Attached Flavin Analog for Formation of Self-Assembled Monolayers on Gold", TETRAHEDRON, ELSEVIER SCIENCE PUBLISHERS, AMSTERDAM, NL, Bd. 55, Nr. 47, 19. November 1999 (1999-11-19), Seiten 13333-13344, XP004188297, ISSN: 0040-4020, DOI: 10.1016/S0040-4020(99)00836-4
- TAKASHI HARAYAMA ET AL: "Reaction of 6-ethylamino-3-methyluracil with nitrobenzenes", JOURNAL OF HETEROCYCLIC CHEMISTRY, Bd. 23, Nr. 5, 1. September 1986 (1986-09-01), Seiten 1507-1509, XP55034208, ISSN: 0022-152X, DOI: 10.1002/jhet.5570230549
- CHRISTINA K. REMUCAL ET AL: "Photosensitized Amino Acid Degradation in the Presence of Riboflavin and Its Derivatives", ENVIRONMENTAL SCIENCE & TECHNOLOGY, Bd. 45, Nr. 12, 15. Juni 2011 (2011-06-15) , Seiten 5230-5237, XP55034035, ISSN: 0013-936X, DOI: 10.1021/es200411a
- COX C ET AL: "Strong hydrogene bonding to the amide nitrogen atom in an amide proton sponge consequences for structure and reactivity", ANGEWANDTE CHEMIE. INTERNATIONAL EDITION, WILEY VCH VERLAG, WEINHEIM, Bd. 38, Nr. 6, 1. Januar 1999 (1999-01-01) , Seiten 798-800, XP008154136, ISSN: 1433-7851
- SHRESTHA A R ET AL: "Synthesis, biological active molecular design, and molecular docking study of novel deazaflavin-cholestane hybrid compounds", BIOORGANIC & MEDICINAL CHEMISTRY, PERGAMON, GB, vol. 16, no. 18, 15 September 2008 (2008-09-15), pages 8685-8696, XP025427664, ISSN: 0968-0896, DOI: 10.1016/J.BMC.2008.07.089 [retrieved on 2008-08-22]

## Beschreibung

Die vorliegende Erfindung betrifft die Verwendung von 10H-Benzo[g]pteridin-2,4-dion-Derivaten und einen damit beschichteten Gegenstand.

Das aktive oder passive Eindringen, Anhaften und Vermehren von Krankheitserregern in einen Wirt wird als Infektion bezeichnet. Quellen für infektiöse Partikel treten überall auf. So wird beispielsweise der menschliche Körper von einer großen Anzahl von Mikroorganismen besiedelt, die im Regelfall durch den normalen Metabolismus und ein intaktes Immunsystem unter Kontrolle gehalten werden. Allerdings kann es, beispielsweise bei einer Schwächung des Immunsystems, zu einer starken Vermehrung der Krankheiterreger kommen und je nach Art des Erregers zu unterschiedlichen Krankheitssymptomen. Die Medizin hält für viele Erreger-bedingte Krankheiten spezifische Gegenmittel bereit, beispielsweise Antibiotika gegen Bakterien oder Antimikotika gegen Pilze oder Virustatika gegen Viren. Allerdings ist bei der Verwendung dieser Gegenmittel vermehrt das Auftreten von resistenten Krankheitserregern zu beobachten, die teilweise gleichzeitig gegen mehrere Gegenmittel Resistenzen aufweisen. Durch das Auftreten dieser resistenten oder multiresistenten Krankheitserreger hat sich die Therapie von Infektionserkrankungen zunehmend erschwert. Die klinische Konsequenz der Resistenz zeigt sich durch ein Versagen der Behandlung, vor allem bei immunsupprimierten Patienten.

Neue Ansatzpunkt zur Bekämpfung von resistenten bzw. multiresistenten Krankheitskeimen sind daher einerseits die Suche nach neuen Gegenmitteln, beispielsweise Antibiotika oder Antimikotika, und andererseits die Suche nach alternativen Inaktivierungsmöglichkeiten.

Als alternatives Verfahren hat sich die photodynamische Inaktivierung von Mikroorganismen bewährt. Bei der photodynamischen Inaktivierung von Mikroorganismen spielen zwei verschiedene photooxidative Prozesse eine entscheidende Rolle. Voraussetzung für den Ablauf einer photooxidativen Inaktivierung ist einerseits das Vorhandensein einer ausreichenden Menge Sauerstoff und andererseits die Lokalisierung eines so genannten Photosensibilisators, welcher durch Licht einer entsprechenden Wellenlänge angeregt wird. Der angeregte Photosensibilisator kann die Bildung von reaktiven Sauerstoffspezies (ROS) bewirken, wobei einerseits Radikale, beispielsweise Superoxidanionen, Wasserstoffperoxid oder Hydroxylradikale, und/oder andererseits angeregter molekularer Sauerstoff, beispielsweise Singulett-Sauerstoff, gebildet werden können.

Bei beiden Reaktionen steht die Photooxidation von spezifischen Biomolekülen, die sich in direkter Nachbarschaft zu den reaktiven Sauerstoffspezies (ROS) befindet, im Vordergrund. Dabei findet insbesondere eine Oxidation von Lipiden und Proteinen statt, die beispielsweise als Bestandteile der Zellmembran von Mikroorganismen vorkommen. Durch die Zerstörung der Zellmembran wiederum kommt es zur Inaktivierung der betreffenden Mikroorganismen. Für Viren und Pilze wird ein ähnlicher Eliminationsprozess angenommen.

Beispielsweise werden durch Singulett-Sauerstoff alle Moleküle angegriffen. Besonders anfällig für eine Schädigung sind allerdings ungesättigte Fettsäuren in den Membranen von Bakterien. Gesunde, körpereigene Zellen verfügen über eine zelluläre Abwehr gegen Angriffe von freien Radikalen, die so genannten Katalasen oder Superoxiddismutasen. Daher können gesunde, körpereigene Zellen eine Schädigung durch reaktive Sauerstoffspezies (ROS), beispielsweise Radikale oder Singulett-Sauerstoff, entgegen wirken.

Aus dem Stand der Technik sind zahlreiche Photosensibilisatoren bekannt, die beispielsweise aus der Gruppe der Porphyrine und deren Derivate oder Phthalocyanine und deren Derivate oder Fullerene und deren Derivate oder Derivate der Phenothiazininium-Struktur, wie beispielsweise Methylenblau oder Toluidinblau, oder Vertreter der Phenoxazinium-Reihe, wie beispielsweise Nile blue, stammen. Die Photodynamik von Methylenblau bzw. Toluidinblau gegenüber Bakterien wird beispielsweise bereits in der Zahnheilkunde eingesetzt.

Bei den aus dem Stand der Technik bekannten Photosensibilisatoren handelt es sich zumeist um Substanzen mit einer relativ komplexen Molekülstruktur und daher aufwendigen Herstellungsverfahren.

Es ist bekannt, dass 10-Methyl-10H-benzo[g]pteridine-2,4-dion-Derivate, Riboflavin und Tetraacetylriboflavin hohe Ausbeuten an Singulett-Sauerstoff haben, wobei jedoch ihre Affinität zu Mikroorganismen gering ist. Es ist weiterhin bekannt, dass Singulett-Sauerstoff lediglich über eine geringe Entfernung diffundieren kann, bevor er reagiert oder abgebaut wird. Daher ist die Inaktivierung von Mikroorganismen durch 10-Methyl-10H-benzo[g]ptendine-2,4-dion-Denvate, Riboflavin und Tetraacetylriboflavin unzureichend.

Weiterhin sind aus der WO 2010/019208 A1 und der WO 2011/008247 A1 zahlreiche Flavin-, Roseoflavin- und Riboflavin-Derivate bekannt, die an Flavinmononucleotid (FMN) Riboswiches binden können. Riboswiches sind RNA-Elemente in untranslatierten Regionen der mRNA von Prokaryoten, Pilzen und Pflanzen, die niedermolekulare Metabolite, beispielsweise FMN, binden und daraufhin die Genexpression regulieren. Beispielsweise wird nach Bindung von FMN an FMN-Riboswiches von Prokaryoten die Expression von Enzymen, die für die Riboflavin- und FMN-Biosynthese verantwortlich sind, reprimiert, wodurch die Riboflavin- und FMN-Biosynthese zum Erliegen kommt. Riboflavin nimmt im Stoffwechsel eine zentrale Rolle ein, da es als Vorstufe für Flavin-Koenzyme dient. Daher führt eine unterdrückte Riboflavin- und FMN-Biosynthese zu einer reduzierten Überlebensfähigkeit.

Allerdings kann es bei dieser Form der Bekämpfung von pathogenen Mikroorganismen ebenfalls zum Auftreten von Resistenzen kommen, die beispielsweise durch Mutationen der entsprechenden RNA-Elemente entstehen können.

Die WO 00/04930 A2 offenbart Verfahren und Vorrichtung zur Inaktivierung von biologischen Kontaminationen unter Verwendung von Photosensibilisatoren.

Ramucal, C.K. und McNeill, K. (Environ. Sci. Technol. 2011, 45(12), Seiten 5230 bis 5237) ist auf die photosensibilisierenden Aminosäure-Abbau in Gegenwart von Riboflavin und Derivaten davon gerichtet.

Aufgabe der vorliegenden Erfindung ist es daher, neue Photosensibilisatoren für eine Verwendung bei der Oberflächenreinigung oder Oberflächenbeschichtung, insbesondere von Medizinprodukten, Lebensmittelverpackungen oder Hygieneartikeln, bereitzustellen, die Mikroorganismen effizienter inaktivieren.

Die Aufgabe der der vorliegenden Erfindung wird gelöst durch die Verwendung nach Anspruch 1 einer Verbindung mit der Formel (1): 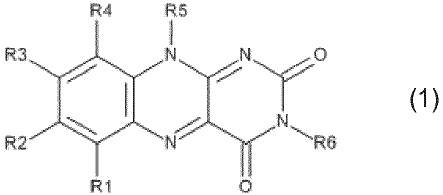 bei der Oberflächenreinigung, insbesondere von Medizinprodukten, Lebensmittelverpackungen oder Hygieneartikeln,
wobei A) nur 1 Rest R1, R2, R3 oder R4 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20 und k, l jeweils unabhängig voneinander eine ganze Zahl von 0 bis 6 bedeuten, wobei D und E jeweils unabhängig voneinander Wasserstoff, Halogen, vorzugsweise Chlor, Brom, lod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet, ist und
wobei die Reste R1, R2, R3 oder R4, die nicht -(C(D)(E))ₕ-X oder - (C(D)(E))ₖ-Aryl-(C(D)(E))ₗ-X sind, jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten und
wobei jeder der Reste R5 oder R6 jeweils unabhängig voneinander gleich oder voneinander verschieden ist und Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet, und wobei der Rest R5 ein nichtcyclischer Polyolrest der allgemeinen Formel -CH₂(CH(OH))_{g}CH₂OH oder ein Ether, Ester oder Acetal davon, wobei g eine ganze Zahl von 1 bis 10, vorzugsweise 1 bis 4, bedeutet, sein kann,
oder
wobei B) nur 1 Rest R5 oder R6 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20 und k, l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet, ist und
wobei der Rest R5 oder R6, der nicht -(C(D)(E))ₕ-X oder -(C(D)(E))ₖ-Aryl-(C(D)(E)ₗ-X ist, Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen,Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
wobei die Reste R1 bis R4 jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten, und wobei der Rest R5 ein nichtcyclischer Polyolrest der allgemeinen Formel -CH₂(CH(OH))_{g}CH₂OH oder ein Ether, Ester oder Acetal davon, wobei g eine ganze Zahl von 1 bis 10, vorzugsweise 1 bis 4, bedeutet, sein kann.

Die Aufgabe der der vorliegenden Erfindung wird weiterhin gelöst durch die Verwendung nach Anspruch 2 einer Verbindung mit der Formel (1): 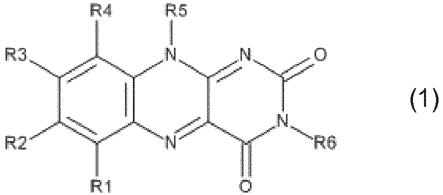 bei der Oberflächenbeschichtung von Medizinprodukten, Lebensmittelverpackungen oder Hygieneartikeln,
wobei A) nur 1 Rest R1, R2, R3 oder R4 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20 und k, l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet, ist und
wobei die Reste R1, R2, R3 oder R4, die nicht -(C(D)(E))ₕ-X oder - (C(D)(E))ₖ-Aryl-(C(D)(E))ₗ-X sind, jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten und
wobei jeder der Reste R5 oder R6 jeweils unabhängig voneinander gleich oder voneinander verschieden ist und Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet, und wobei der Rest R5 ein nichtcyclischer Polyolrest der allgemeinen Formel -CH₂(CH(OH))_{g}CH₂OH oder ein Ether, Ester oder Acetal davon, wobei g eine ganze Zahl von 1 bis 10, vorzugsweise 1 bis 4, bedeutet, sein kann,
oder
wobei B) nur 1 Rest R5 oder R6 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20 und k, l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet, ist und
wobei der Rest R5 oder R6, der nicht -(C(D)(E))ₕ-X oder -(C(D)(E))ₖ-Aryl-(C(D)(E)ₗ-X ist, Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen,Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
wobei die Reste R1 bis R4 jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten, und wobei der Rest R5 ein nichtcyclischer Polyolrest der allgemeinen Formel -CH₂(CH(OH))_{g}CH₂OH oder ein Ether, Ester oder Acetal davon, wobei g eine ganze Zahl von 1 bis 10, vorzugsweise 1 bis 4, bedeutet, sein kann.

Bei der erfindungsgemäß verwendeten Verbindung mit der Formel (1) handelt es sich um ein 10H-Benzo[g]pteridin-2,4-dion- oder Flavin-Derivat, das im Folgenden auch so bezeichnet wird.

Als Gegenion zum positiv geladenen quartären Stickstoffatom kann jedes geeignete Anion verwendet werden. Vorzugsweise werden als Gegenion zum positiv geladenen quartären Stickstoffatom Anionen verwendet, die die Bereitstellung eines pharmakologisch verträglichen Salzes ermöglichen.

Bei einer bevorzugten Ausführungsform der vorliegenden Erfindung ist X in der Verbindung mit der Formel (1) ein organischer Rest mit nur einem quartären Stickstoffatom, das als Gegenion Fluorid, Chlorid, Bromid, lodid, Sulfat, Hydrogensulfat, Phosphat, Dihydrogenphosphat, Hydrogenphosphat, Tosylat, Mesylat, Formiat, Acetat, Oxalat, Benzoat, Citrat und/oder Mischungen davon aufweist.

Die Aufgabe der der vorliegenden Erfindung wird ebenfalls gelöst durch die Bereitstellung eines beschichteten Gegenstands nach Anspruch 6, wobei der Gegenstand aus der Gruppe, die aus Medizinprodukten, Lebensmittelverpackungen und Hygieneartikeln besteht, ausgewählt ist und der Gegenstand mit einer Verbindung mit der Formel (1): 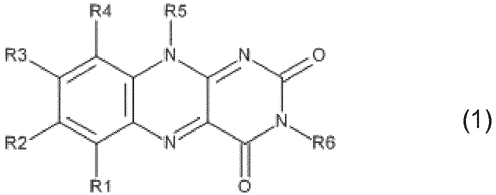 beschichtet ist,
wobei A) nur 1 Rest R1, R2, R3 oder R4 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20 und k, l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet, ist und
wobei die Reste R1, R2, R3 oder R4, die nicht -(C(D)(E))ₕ-X oder - (C(D)(E))ₖ-Aryl-(C(D)(E))ₗ-X sind, jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten und
wobei jeder der Reste R5 oder R6 jeweils unabhängig voneinander gleich oder voneinander verschieden ist und Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet, und wobei der Rest R5 ein nichtcyclischer Polyolrest der allgemeinen Formel -CH₂(CH(OH))_{g}CH₂OH oder ein Ether, Ester oder Acetal davon, wobei g eine ganze Zahl von 1 bis 10, vorzugsweise 1 bis 4, bedeutet, sein kann,
oder
wobei B) nur 1 Rest R5 oder R6 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20 und k, l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet, ist und
wobei der Rest R5 oder R6, der nicht -(C(D)(E))ₕ-X oder -(C(D)(E))ₖ-Aryl-(C(D)(E)ₗ-X ist, Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen,Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
wobei die Reste R1 bis R4 jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten, und wobei der Rest R5 ein nichtcyclischer Polyolrest der allgemeinen Formel -CH₂(CH(OH))_{g}CH₂OH oder ein Ether, Ester oder Acetal davon, wobei g eine ganze Zahl von 1 bis 10, vorzugsweise 1 bis 4, bedeutet, sein kann.

Weitere bevorzugte Ausführungsformen der vorliegenden Erfindung sind in den abhängigen Ansprüchen beschrieben.

Bei einer weiter bevorzugten Ausführungsform enthält das erfindungsgemäß zu verwendende 10H-Benzo[g]pteridin-2,4-dion-Derivats der Formel (1) keine basischen, vorzugsweise neutralen, protonierbaren und/oder positiv geladenen, Stickstoffatome und keine weiteren quartären Stickstoffatome.

Als "Photosensibilisator" werden erfindungsgemäß Verbindungen verstanden, die elektromagnetische Strahlung, vorzugsweise sichtbares Licht, UV-Licht und/oder Infrarotlicht, absorbieren und sodann reaktive Sauerstoffspezies (ROS), vorzugsweise freie Radikale und/oder Singulett-Sauerstoff, aus Triplett-Sauerstoff erzeugen.

Unter dem Begriff "Inaktivierung" wird erfindungsgemäß die Reduzierung der Lebensfähigkeit oder die Zerstörung eines Mikroorganismus, vorzugsweise dessen Zerstörung, verstanden. Eine lichtinduzierte Inaktivierung kann beispielsweise durch Verringerung der Anzahl von Mikroorganismen nach Bestrahlung einer definierten Ausgangsmenge dieser Mikroorganismen in Gegenwart wenigstens einer erfindungsgemäß zu verwendenden Verbindung mit der Formel (1) bestimmt werden.

Erfindungsgemäß wird unter einer Reduzierung der Lebensfähigkeit verstanden, dass die Anzahl der Mikroorganismen um wenigstens 99,0 %, vorzugsweise um wenigstens 99,9 %, weiter bevorzugt um wenigstens 99,99 %, weiter bevorzugt um wenigstens 99,999 %, noch weiter bevorzugt um wenigstens 99,9999 %, verringert wird. Äußerst bevorzugt wird die Anzahl der Mikroorganismen um mehr als 99,9 bis 100 %, vorzugsweise um mehr als 99,99 bis 100 %, verringert wird.

Vorzugsweise wird die Reduzierung der Anzahl der Mikroorganismen gemäß Boyce, J.M. und Pittet, D. ("Guidelines for hand hygiene in healthcare settings. Recommendations of the Healthcare Infection Control Practices Advisory Committee and the HIPAC/SHEA/APIC/IDSA Hand Hygiene Task Force", Am.J.Infect.Control 30 (8), 2002, Seite 1 - 46) als log₁₀-Reduktionsfaktor angegeben.

Erfindungsgemäß wird unter dem Begriff "log₁₀-Reduktionsfaktor" die Differenz zwischen dem dekadischen Logarithmus der Anzahl der Mikroorganismen vor und dem dekadischen Logarithmus der Anzahl der Mikroorganismen nach einer Bestrahlung dieser Mikroorganismen mit elektromagnetischer Strahlung in Gegenwart wenigstens einer erfindungsgemäß zu verwendenden Verbindung mit der Formel (1) verstanden.

Geeignete Methoden zur Bestimmung des log₁₀-Reduktionsfaktors sind beispielsweise in der DIN EN 14885:2007-01 "Chemische Desinfektionsmittel und Antiseptika - Anwendung Europäischer Normen für chemische Desinfektionsmittel und Antiseptika" oder in Rabenau, H.F. und Schwebke, I. ("Leitlinie der Deutschen Vereinigung zur Bekämpfung der Viruskrankheiten (DVV) e.V. und des Robert Koch-Instituts (RKI) zur Prüfung von chemischen Desinfektionsmitteln auf Wirksamkeit gegen Viren in der Humanmedizin" Bundesgesundheitsblatt, Gesundheitsforschung, Gesundheitsschutz 51(8), (2008), Seiten 937 - 945) beschrieben.

Vorzugsweise beträgt der log₁₀-Reduktionsfaktor nach einer Bestrahlung von Mikroorganismen mit elektromagnetischer Strahlung in Gegenwart wenigstens einer erfindungsgemäß zu verwendenden Verbindung mit der Formel (1) mindestens 2 log₁₀, vorzugsweise mindestens 3 log₁₀, weiter bevorzugt mindestens 4 log₁₀, weiter bevorzugt mindestens 4,5 log₁₀, weiter bevorzugt mindestens 5 log₁₀, weiter bevorzugt mindestens 6 log₁₀, noch weiter bevorzugt mindestens 7 log₁₀, noch weiter bevorzugt mindestens 7,5 log₁₀.

Beispielsweise bedeutet eine Reduktion der Anzahl der Mikroorganismen nach einer Bestrahlung dieser Mikroorganismen mit elektromagnetischer Strahlung in Gegenwart wenigstens einer erfindungsgemäß zu verwendenden Verbindung mit der Formel (1) um 2 Zehnerpotenzen, bezogen auf die Ausgangsmenge dieser Mikroorganismen, einen log₁₀-Reduktionsfaktor von 2 log₁₀.

Weiter bevorzugt wird die Anzahl der Mikroorganismen nach einer Bestrahlung dieser Mikroorganismen mit elektromagnetischer Strahlung in Gegenwart wenigstens einer erfindungsgemäß zu verwendenden Verbindung mit der Formel (1) um mindestens 1 Zehnerpotenz, weiter bevorzugt um mindestens 2 Zehnerpotenzen, vorzugsweise um mindestens 4 Zehnerpotenzen, weiter bevorzugt um mindestens 5 Zehnerpotenzen, weiter bevorzugt um mindestens 6 Zehnerpotenzen, noch weiter bevorzugt um mindestens 7 Zehnerpotenzen, jeweils bezogen auf die Ausgangsmenge dieser Mikroorganismen, verringert.

Unter dem Begriff "Mikroorganismen" werden im Sinne der Erfindung insbesondere Viren, Archäeen, prokaryote Mikroorganismen, wie Bakterien und Bakteriensporen, und eukaryote Mikroorganismen, wie Pilze, Protozoen, Pilzsporen, einzellige Algen, verstanden. Die Mikroorganismen können dabei einzellig oder mehrzellig, beispielsweise als Pilzmycel, auftreten.

Ein erfindungsgemäß zu verwendendes 10H-Benzo[g]pteridin-2,4-dion-Derivat weist die Formel (1) auf, 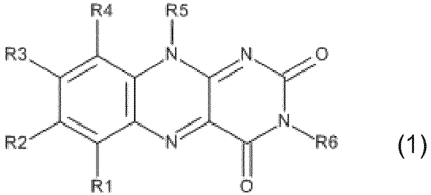 wobei entweder
A) nur 1 Rest R1, R2, R3 oder R4 ein organischer Rest der allgemeinen Formel -(C(D)(E))ₕ-X oder -(C(D)(E))ₖ-Aryl-(C(D)(E))ₗ-X ist, wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, wobei Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet, und wobei X ein organischer Rest mit nur einem quartären Stickstoffatom ist
   oder
B) nur 1 Rest R5 oder R6 ein organischer Rest der allgemeinen Formel -(C(D)(E))ₕ-X oder -(C(D)(E))ₖ-Aryl-(C(D)(E))ₗ-X ist, wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, wobei Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet, und wobei X ein organischer Rest mit nur einem quartären Stickstoffatom ist.

Bei einer bevorzugten Ausführungsform weist das erfindungsgemäß zu verwendende 10H-Benzo[g]pteridin-2,4-dion-Derivat der Formel (1) kein neutrales, protonierbares Stickstoffatom, das direkt an den Isoalloxazinring gebunden ist, beispielsweise als Amino-Rest, Methylamino-Rest oder Dimethylamino-Rest, und kein positiv geladenes Stickstoffatom, das direkt an den Isoalloxazinring gebunden ist, beispielsweise als Pyridin-1-ium-1-yl-Rest oder Trimethylammonio-Rest, auf.

Weiter bevorzugt enthält der organische Rest X keine basischen Stickstoffatome, vorzugsweise kein neutrales, protonierbares Stickstoffatom, und/oder kein protonierbares, positiv geladenes Stickstoffatom.

Bei Variante A) des erfindungsgemäß zu verwendenden 10H-Benzo[g]pteridin-2,4-dion-Derivats der Formel (1) ist nur 1 Rest R1, R2, R3 oder R4 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, wobei Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet, und wobei X ein organischer Rest mit nur einem quartären Stickstoffatom ist,
wobei die Reste R1, R2, R3 oder R4, die nicht -(C(D)(E))ₕ-X oder - (C(D)(E))ₖ-Aryl-(C(D)(E))ₗ-X sind, jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten und
wobei jeder der Reste R5 oder R6 jeweils unabhängig voneinander gleich oder voneinander verschieden ist und Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und wobei der Rest R5 ein nichtcyclischer Polyolrest der allgemeinen Formel -CH₂(CH(OH))_{g}CH₂OH oder ein Ether, Ester oder Acetal davon, wobei g eine ganze Zahl von 1 bis 10, vorzugsweise 1 bis 4, bedeutet, sein kann.

Bei Variante B) des erfindungsgemäß zu verwendenden 10H-Benzo[g]pteridin-2,4-dion-Derivats der Formel (1) ist nur 1 Rest R5 oder R6 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, wobei Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet, und wobei X ein organischer Rest mit nur einem quartären Stickstoffatom ist,
wobei der Rest R5 oder R6, der nicht -(C(D)(E))ₕ-X oder -(C(D)(E))ₖ-Aryl-(C(D)(E)ₗ-X ist, Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Ether mit 2 bis 20 C-Atomen, Thioether mit 2 bis 20 C-Atomen, Aryl mit 5 bis 20 C-Atomen oder Heteroaryl mit 4 bis 20 C-Atomen, das keine N-Atome enthält, bedeutet, und wobei der Rest R5 ein nichtcyclischer Polyolrest der allgemeinen Formel -CH₂(CH(OH))_{g}CH₂OH oder ein Ether, Ester oder Acetal davon, wobei g eine ganze Zahl von 1 bis 10, vorzugsweise 1 bis 4, bedeutet, sein kann, und
wobei die Reste R1 bis R4 jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten

Bei einer weiteren bevorzugten Ausführungsform des erfindungsgemäß zu verwendenden 10H-Benzo[g]pteridin-2,4-dion-Derivats der Formel (1) ist entweder
A) nur 1 Rest R1, R2, R3 oder R4 ein organischer Rest der allgemeinen Formel -(C(D)(E))ₕ-X oder -(C(D)(E))ₖ-Aryl-(C(D)(E))ₗ-X,
   wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, wobei X ein organischer Rest ist, der nur ein quartäres Stickstoffatom enthält, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet und
   wobei die Reste R1, R2, R3 oder R4, die nicht ein organischer Rest der allgemeinen Formel -(C(D)(E))ₕ-X oder -(C(D)(E))ₖ-Aryl-(C(D)(E))ₗ-X sind, jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten und
   wobei der Reste R5 ein nichtcyclischer Polyolrest der allgemeinen Formel -CH₂(CH(OH))_{g}CH₂OH ist oder ein Ether, Ester oder Acetal davon, wobei g eine ganze Zahl von 1 bis 10, vorzugsweise 1 bis 4, bedeutet und
   wobei R6 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet
   oder
B) nur der Rest R6 ein organischer Rest der allgemeinen Formel -(C(D)(E))ₕ-X oder -(C(D)(E))ₖ-Aryl-(C(D)(E))ₗ-X,
   wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, wobei X ein organischer Rest ist, der nur ein quartäres Stickstoffatom enthält, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
   wobei der Reste R5 ein nichtcyclischer Polyolrest der allgemeinen Formel -CH₂(CH(OH))_{g}CH₂OH ist oder ein Ether, Ester oder Acetal davon, wobei g eine ganze Zahl von 1 bis 10, vorzugsweise 1 bis 4, bedeutet und
   wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 1 bis 20 C-Atomen, S-Alkenyl mit 1 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 1 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten.

Weiter bevorzugt ist der Reste R5 ein nichtcyclischer Polyolrest, der aus der Gruppe, die aus Arabityl, Ribityl, Xylityl, Erythrityl, Threityl, Lactityl, Mannityl und Sorbityl, weiter bevorzugt D-Ribityl und D-Arabityl, besteht, ausgewählt wird oder ein Ether, Ester oder Acetal davon.

Bei einer weiteren bevorzugten Ausführungsform des erfindungsgemäß zu verwendenden 10H-Benzo[g]pteridin-2,4-dion-Derivats der Formel (1) ist nur der Reste R5 ein nichtcyclischer Polyolrest der allgemeinen Formel -CH₂(CH(OZ))_{f}CH₍₃₋ₑ₎(OZ)ₑ, wobei e 0, 1 oder 2 ist und f eine ganze Zahl von 1 bis 10, vorzugsweise 1 bis 4, bedeutet und wobei Z Wasserstoff, Alkyl mit 1 bis 20 C-Atomen, Carbonsäurester mit 1 bis 20 C-Atomen oder ein Rest X ist, wobei X ein organischer Rest mit nur einem quartären Stickstoffatom ist, mit der Maßgabe, dass nur 1 Rest Z ein organischer Rest X mit nur einem quartären Stickstoffatom ist,
wobei R6 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet und
wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 1 bis 20 C-Atomen, S-Alkenyl mit 1 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 1 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten.

Bei einer bevorzugten Ausführungsform des 10H-Benzo[g]pteridin-2,4-dion-Derivats der Formel (1) ist der organische Rest X mit nur einem quartären Stickstoffatom ein Rest der allgemeinen Formel (2): 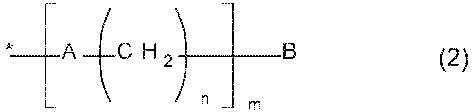 wobei A ein Sauerstoff- oder ein Schwefelatom ist und wobei n eine ganze Zahl von 1 bis 8 und m eine ganze Zahl von 0 bis 100, vorzugsweise 0-59, vorzugsweise 0-10, bedeuten und wobei B ein Rest der Formel (3), (4a), (4b), (5a) oder (5b): 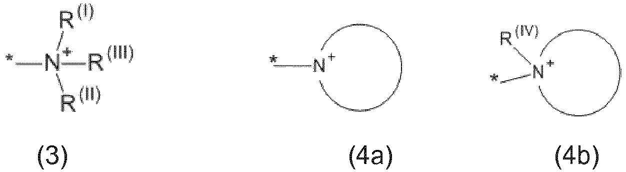 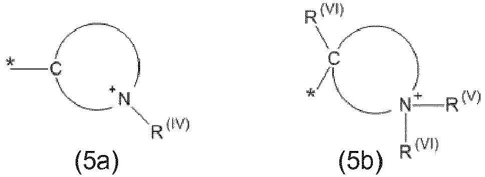 ist und wobei jeder der Reste R^{(I)}, R^{(II)}, R^{(III)}, R^{(IV)} und R^{(V)} unabhängig voneinander ein Arylrest mit 5 bis 20 C-Atomen, ein Alkylrest, der geradkettig oder verzweigt sein kann, mit 1 bis 20 C-Atomen, ein Alkenylrest, der geradkettig oder verzweigt sein kann, mit 2 bis 20 C-Atomen, ein Hydroxyalkylrest, der geradkettig oder verzweigt sein kann, mit 1 bis 20 C-Atomen, ein Etherrest, der geradkettig oder verzweigt sein kann, mit 2 bis 20 C-Atomen, oder ein Thioetherrest, der geradkettig oder verzweigt sein kann, mit 2 bis 20 C-Atomen ist und wobei der Rest R^{(VI)} Wasserstoff ein Arylrest mit 5 bis 20 C-Atomen, ein Alkylrest, der geradkettig oder verzweigt sein kann, mit 1 bis 20 C-Atomen, ein Alkenylrest, der geradkettig oder verzweigt sein kann, mit 2 bis 20 C-Atomen, ein Hydroxyalkylrest, der geradkettig oder verzweigt sein kann, mit 1 bis 20 C-Atomen, ein Etherrest, der geradkettig oder verzweigt sein kann, mit 2 bis 20 C-Atomen, oder ein Thioetherrest, der geradkettig oder verzweigt sein kann, mit 2 bis 20 C-Atomen ist und wobei der Rest mit der Formel (4a) und der Rest mit der Formel (5a): 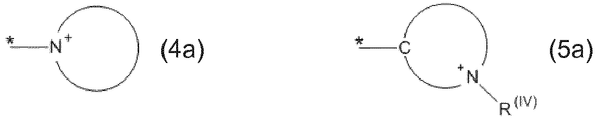 einen substituierten oder unsubstituierten heterocyclischen Rest mit 5 bis 7 Ringatomen, die 1 Stickstoffatom und mindestens 1 Kohlenstoffatom sowie optional 1 oder 2 Sauerstoff- oder Schwefelatom umfassen, wobei 1 Stickstoffatom eine Doppelbindung ausbildet, darstellt und wobei der Rest mit der Formel (4b) und der Rest mit der Formel (5b): 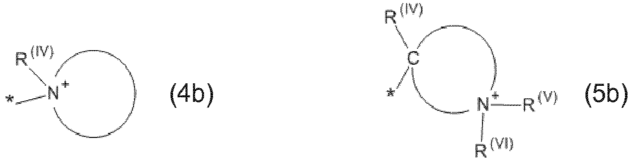 einen substituierten oder unsubstituierten heterocyclischen Rest mit 5 bis 7 Ringatomen, die 1 Stickstoffatom und mindestens 1 Kohlenstoffatom sowie optional 1 oder 2 Sauerstoff- oder Schwefelatom umfassen, darstellt.

Bei einer weiteren bevorzugten Ausführungsform wird der Rest der Formel (5a): aus der Gruppe, die aus Resten der Formeln (7a), (7b) und (7c): 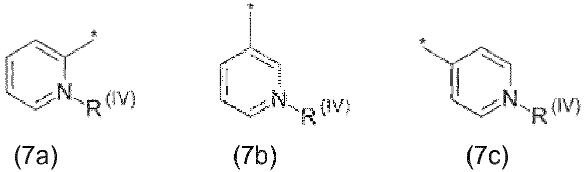 besteht, ausgewählt, wobei R^{(IV)} jeweils ein Arylrest mit 5 bis 20 C-Atomen, beispielsweise Phenyl oder Benzyl, Alkylrest, der geradkettig oder verzweigt sein kann, mit 1 bis 20 C-Atomen, beispielsweise Methyl, Ethyl, n-Propyl, i-Propyl, n-Butyl oder t-Butyl, Alkenylrest, der geradkettig oder verzweigt sein kann, mit 2 bis 20 C-Atomen, Hydroxyalkylrest, der geradkettig oder verzweigt sein kann, mit 1 bis 20 C-Atomen, beispielsweise Hydroxymethyl, 2-Hydroxyethyl, 3-Hydroxypropyl oder 1-Hydroxy-1-methyl-ethyl, Etherrest, der geradkettig oder verzweigt sein kann, mit 2 bis 20 C-Atomen, beispielsweise Methoxymethyl, Methoxyethyl, Methoxypropyl, Ethoxymethyl, Ethoxyethyl, Ethoxypropyl, Propoxymethyl, Propoxyethyl oder Propoxypropyl oder Thioetherrest, der geradkettig oder verzweigt sein kann, mit 2 bis 20 C-Atomen, beispielsweise Methylsulfanylmethyl, Ethylsulfanylmethyl, 2-Ethylsulfanylethyl oder 3-Methylsulfanylpropyl sein kann.

Bei einer weiteren bevorzugten Ausführungsform wird der Rest der Formel (4a) aus der Gruppe, die aus Resten der Formeln (8a), (8b) und (8c): 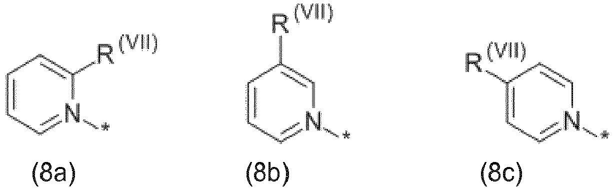 besteht, ausgewählt, wobei R^{(VII)} jeweils Wasserstoff, ein Arylrest mit 5 bis 20 C-Atomen, beispielsweise Phenyl oder Benzyl, ein Alkylrest, der geradkettig oder verzweigt sein kann, mit 1 bis 20 C-Atomen, beispielsweise Methyl, Ethyl, n-Propyl, i-Propyl, n-Butyl oder t-Butyl, ein Alkenylrest, der geradkettig oder verzweigt sein kann, mit 2 bis 20 C-Atomen, ein Hydroxyalkylrest, der geradkettig oder verzweigt sein kann, mit 1 bis 20 C-Atomen, beispielsweise Hydroxymethyl, 2-Hydroxyethyl, 3-Hydroxypropyl oder 1-Hydroxy-1-methyl-ethyl, ein Etherresten, die geradkettig oder verzweigt sein können, mit 2 bis 20 C-Atomen, beispielsweise Methoxymethyl, Methoxyethyl, Methoxypropyl, Ethoxymethyl, Ethoxyethyl, Ethoxypropyl, Propoxymethyl, Propoxyethyl oder Propoxypropyl, oder ein Thioetherrest, die geradkettig oder verzweigt sein kann, mit 2 bis 20 C-Atomen, beispielsweise Methylsulfanylmethyl, Ethylsulfanylmethyl, 2-Ethylsulfanylethyl oder 3-Methylsulfanylpropyl, sein kann.

Bei einer weiteren bevorzugten Ausführungsform ist der Rest der Formel (4b) 1-Methylpyrrolidin-1-ium-1-yl, 1-Methylpiperazin-1-ium-1-yl oder 4-Methylmorpholin-4-ium-4-yl.

Bei einer weiteren bevorzugten Ausführungsform werden die Reste R^{(I)}, R^{(II)}, R^{(III)}, R^{(IV)} und R^{(V)} unabhängig voneinander aus der Gruppe, die aus Wasserstoff und Alkylgruppen der allgemeinen Formel -(CH₂)ₙ-CH₃, wobei n eine ganze Zahl von 0 bis 19, vorzugsweise von 1 bis 17, ist, besteht, ausgewählt.

Bei einer weiteren bevorzugten Ausführungsform werden die Reste R^{(I)}, R^{(II)}, R^{(III)}, R^{(IV)} und R^{(V)} unabhängig voneinander ausgewählt aus der Gruppe, die aus Wasserstoff, Methyl, Ethyl, Prop-1-yl, Prop-2-yl, But-1-yl, But-2-yl, 2-Methylprop-1-yl, 2-Methyl-prop-2-yl, Pent-1-yl, Pent-2-yl, Pent-3-yl, 2-Methylbut-1-yl, 2-Methylbut-2-yl, 2-Methylbut-3-yl, 2-Methylbut-4-yl, 2,2-Dimethylprop-1-yl, Hex-1-yl, Hex-2-yl, Hex-3-yl, Hept-1-yl, Oct-1-yl, 2-Methylpent-1-yl, 2-Methylpent-2-yl, 2-Methylpent-3-yl, 2-Methylpent-4-yl, 2-Methylpent-5-yl, 3-Methylpent-1-yl, 3-Methylpent-2-yl, 3-Methylpent-3-yl, 2,2-Dimethylbut-1-yl, 2,2-dimethylbut-3-yl, 2,2-Dimethylbut-4-yl, 2,3-Dimethylbut-1-yl und 2,3-Dimethylbut-2-yl besteht, ausgewählt.

Bei einer besonders bevorzugten Ausführungsform werden die Reste R^{(I)}, R^{(II)}, R^{(III)}, R^{(IV)} und R^{(V)} unabhängig voneinander aus der Gruppe, die aus Methyl, Ethyl, Prop-1-yl, But-1-yl, Pent-1-yl, Hex-1-yl, Hept-1-und Oct-1-yl besteht, ausgewählt.

Bei einer weiteren bevorzugten Ausführungsform sind die Reste R^{(I)}, R^{(II)}, R^{(III)}, R^{(IV)} und R^{(V)} unabhängig voneinander Wasserstoff oder der Rest mit der Formel (10): 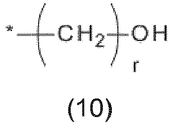 besteht, wobei r jeweils eine ganze Zahl von 1 bis 20, vorzugsweise von 1 bis 8, weiter bevorzugt von 1 bis 4, bedeutet, ausgewählt.

Bei einer weiteren bevorzugten Ausführungsform des erfindungsgemäß zu verwendenden 10H-Benzo[g]pteridin-2,4-dion-Derivats der Formel (1) wird der organischer Rest der allgemeinen Formel -(C(D)(E))ₕ-X aus der Gruppe, die aus den Resten der Formel (12a) bis (15b) besteht, 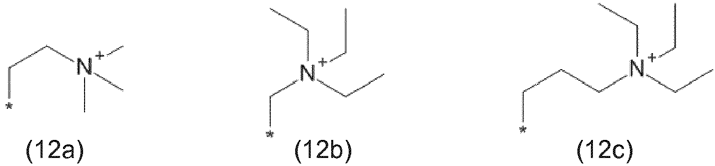 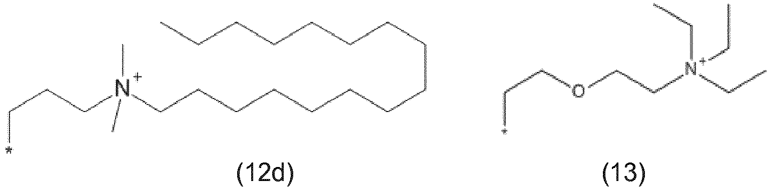 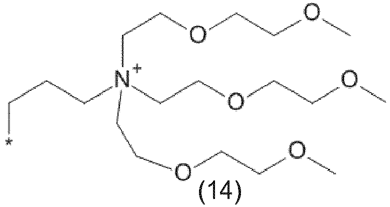 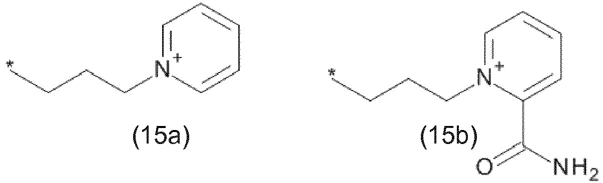 ausgewählt.

Bei einer weiter bevorzugten Ausführungsform sind die Reste R1 und R4, die jeweils unabhängig voneinander gleich oder voneinander verschieden sein können, Wasserstoff oder Methyl und nur 1 Rest R2, R3, R5 oder R6 ist ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20 und k, l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet, ist.

Bei einer weiteren bevorzugten Ausführungsform wird das erfindungsgemäß zu verwendende 10H-Benzo[g]pteridin-2,4-dion-Derivats der Formel (1) aus der Gruppe, die aus den Verbindungen mit den Formeln (30) bis (43) besteht, ausgewählt:

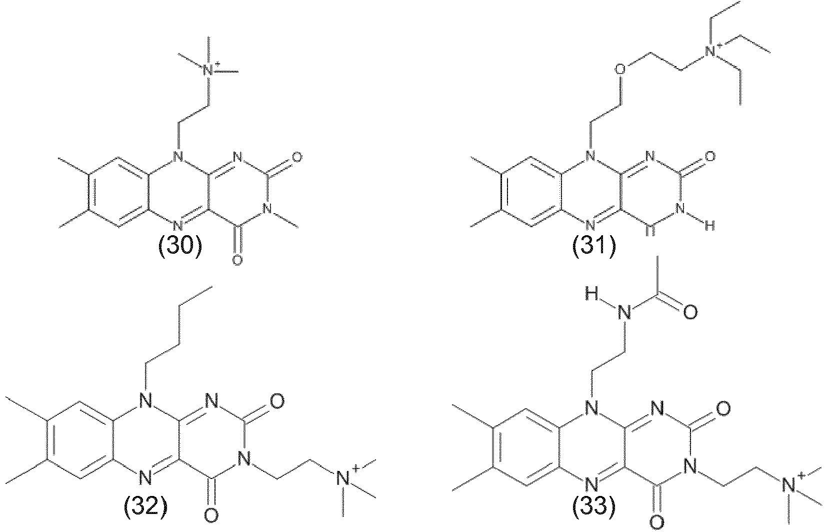

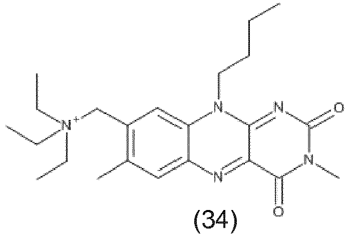

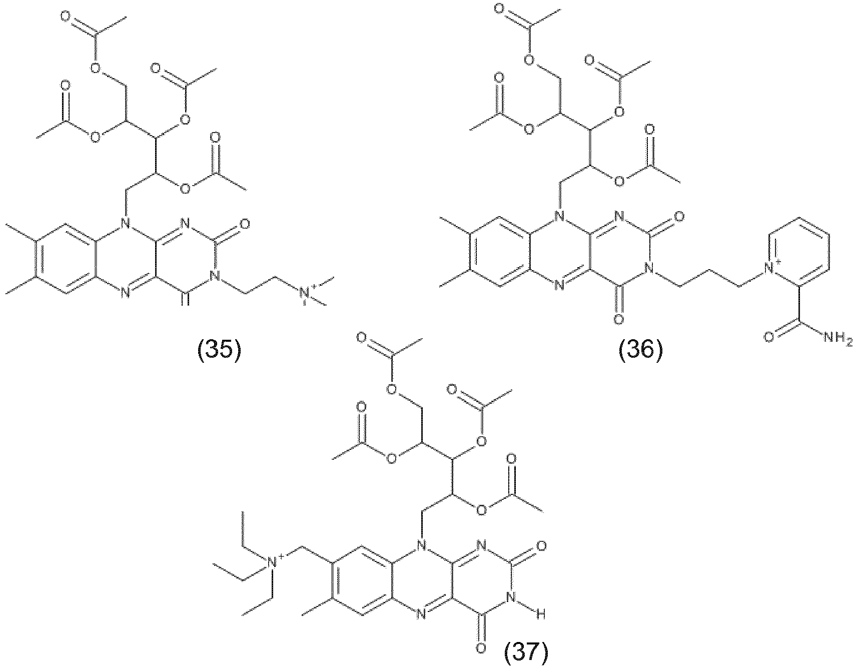

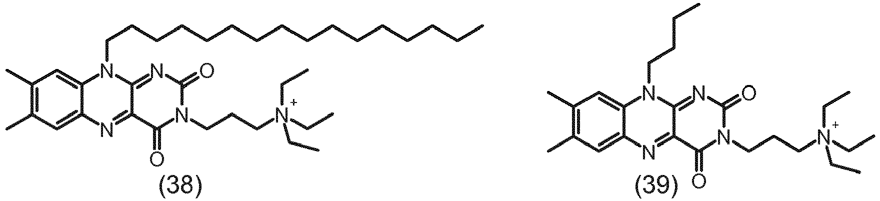

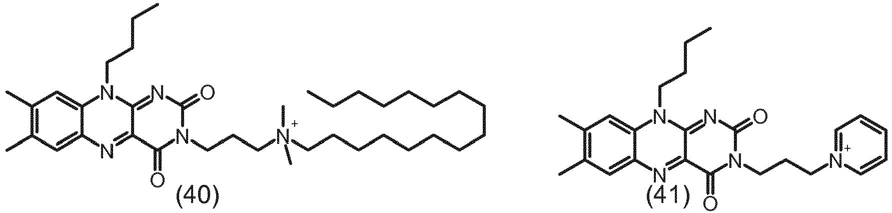

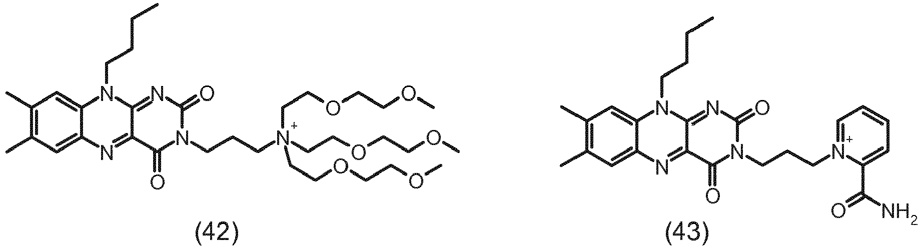

Beim erfindungsgemäß zu verwendenden 10H-Benzo[g]pteridin-2,4-dion-Derivat der Formel (1) können vorgenannte Aldehydreste, Ketonreste, Carbonsäurereste, Carbonsäureamidreste, Thioesterreste, Cycloalkylreste, Cycloalkenylreste, Alkylreste und Akenylreste geradkettig oder verzweigt, vorzugsweise geradkettig, sein und sowohl unsubstituiert oder mit wenigstens einem Rest, der Halogen, vorzugsweise Chlor, Brom, Iod oder Fluor, Thiol, Nitro, Hydroxy, Sulfanyl, Alkyloxy, vorzugsweise Methoxy, Ethoxy, n-Propyloxy, i-Propyloxy, n-Butyloxy oder n-Pentyloxy, Alkylsulfanyl, vorzugsweise Methylsulfanyl, Ethylsulfanyl, n-Propylsulfanyl, i-Propylsulfanyl, n-Butylsulfanyl oder n-Pentylsulfanyl oder Alkanoyloxy, vorzugsweise Formyloxy, Acetoxy oder n-Propanoyloxy ist, substituiert sein.

Bei einer weiter bevorzugten Ausführungsform werden die vorgenannte Alkylreste jeweils aus der Gruppe, die aus Methyl, Ethyl, n-Propyl, n-Butyl, n-Pentyl, n-Hexyl, n-Heptyl und n-Octyl besteht, ausgewählt.

Bei einer weiter bevorzugten Ausführungsform können vorgenannte Alkylreste nichtcyclische Polyolreste der allgemeinen Formel -CH₂(CH(OH))_{g}CH₂OH sein, wobei g eine ganze Zahl von 1 bis 10, vorzugsweise 1 bis 4, ist. Weiter bevorzugt werden die nichtcyclischen Polyolreste aus der Gruppe, die aus Arabityl, Ribityl, Xylityl, Erythrityl, Threityl, Lactityl, Mannityl und Sorbityl, weiter bevorzugt D-Ribityl und D-Arabityl, besteht, ausgewählt.

Beim erfindungsgemäß zu verwendenden 10H-Benzo[g]pteridin-2,4-dion-Derivats der Formel (1) können vorgenannte Cycloalkylreste und Cycloalkenylreste Sauerstoff- und/oder Schwefelatome, als Ringatome aufweisen und sowohl unsubstituiert oder mit wenigstens einem Rest, der aus Hydroxyl, Sulfanyl, Alkyloxy, vorzugsweise Methoxy, Ethoxy, n-Propyloxy, i-Propyloxy, n-Butyloxy oder n-Pentyloxy, Alkylsulfanyl, vorzugsweise Methylsulfanyl, Ethylsulfanyl, n-Propylsulfanyl, i-Propylsulfanyl, n-Butylsulfanyl oder n-Pentylsulfanyl oder Alkanoyloxy, vorzugsweise Formyloxy, Acetoxy oder n-Propanoyloxy ist, substituiert sein.

Bei einer weiter bevorzugten Ausführungsform werden die vorgenannten Cycloalkylreste und Cycloalkenylreste, die Sauerstoff- und/oder Schwefelatome, als Ringatome aufweisen, jeweils aus der Gruppe, die aus Tetrahydrofuranyl, Tetrahydrothiophenyl, Tetrahydropyranyl, Tetrahydrothiopyranyl, Dioxolanyl und Dioxanyl besteht, ausgewählt.

Bei einer bevorzugten Ausführungsform des erfindungsgemäß zu verwendenden 10H-Benzo[g]pteridin-2,4-dion-Derivats der Formel (1) weisen die Arylreste jeweils höchstens 4, weiter bevorzugt höchstens 3, weiter bevorzugt höchstens 2, anellierte Ringe auf. Noch weiter bevorzugt weisen die Arylreste jeweils 1 Ring auf.

Bei einer bevorzugten Ausführungsform des erfindungsgemäß zu verwendenden 10H-Benzo[g]pteridin-2,4-dion-Derivats der Formel (1) werden die vorgenannten Arylreste aus der Gruppe, die aus Phenyl, Benzyl, Naphthyl, Anthracenyl, Phenanthrenyl und Pyrenyl besteht, ausgewählt.

Bei einer bevorzugten Ausführungsform des erfindungsgemäß zu verwendenden 10H-Benzo[g]pteridin-2,4-dion-Derivats der Formel (1) weisen die vorgenannten Alkenylreste 2 bis 17 C-Atome, weiter bevorzugt 2 bis 13 C-Atome, weiter bevorzugt 2 bis 9 C-Atome, weiter bevorzugt 2 bis 5 C-Atome, auf. Bei einer weiter bevorzugten Ausführungsform werden die vorgenannten Alkenylreste aus der Gruppe, die aus Ethenyl, n-Propenyl und n-Butenyl besteht, ausgewählt.

Bei einer weiteren bevorzugten Ausführungsform des erfindungsgemäß zu verwendenden 10H-Benzo[g]pteridin-2,4-dion-Derivats der Formel (1) weisen die vorgenannten Aldehyde 1 bis 17 C-Atome, weiter bevorzugt 1 bis 13 C-Atome, weiter bevorzugt 1 bis 9 C-Atome, weiter bevorzugt 1 bis 5 C-Atome, auf. Bei einer weiter bevorzugten Ausführungsform werden die vorgenannten Aldehyde aus der Gruppe, die aus Methanal-1-yl (Formyl), Ethanal-1-yl (2-Oxoethyl), n-Propanal-1-yl (3-Oxopropyl) und n-Butanal-1-yl (4-Oxobutyl) besteht, ausgewählt.

Bei einer weiteren bevorzugten Ausführungsform des erfindungsgemäß zu verwendenden 10H-Benzo[g]pteridin-2,4-dion-Derivats der Formel (1) weisen die vorgenannten Ketone 2 bis 17 C-Atome, weiter bevorzugt 3 bis 14 C-Atome, weiter bevorzugt 3 bis 9 C-Atome, auf. Bei einer weiter bevorzugten Ausführungsform werden die vorgenannten Ketone aus der Gruppe, die aus Dimethylketyl, Methyl-Ethyl-ketyl, Ethyl-Methyl-ketyl, Diethylketyl, Methyl-Propyl-ketyl, Ethyl-Propyl-ketyl, Propyl-Methyl-ketyl, Propyl-Ethyl-ketyl und Dipropyl-ketyl, das geradkettig oder verzweigt sein kann, besteht, ausgewählt.

Bei einer weiter bevorzugten Ausführungsform können vorgenannte Aldehydreste und/oder Ketonreste Monosaccharidreste, vorzugsweise Pentose- oder Ketosereste, sein.

Vorzugsweise haben geeignete Monosaccharidreste 3 bis 7 Kohlenstoffatome, vorzugsweise 5 bis 6 Kohlenstoffatome, und weisen eine Carbonylgruppe, vorzugsweise Aldehydgruppe oder Ketogruppe, sowie mindestens eine Hydroxylgruppe auf und können offenkettig oder cyclisch, vorzugsweise als Furanose oder Pyranose, vorliegen.

Bevorzugt leiten sich geeignete Monosaccharidreste von Monosacchariden ab, die aus der Gruppe, die aus D-Glycerinaldehyd, L-Glycerinaldehyd, D-Erythrose, L-Erythrose, D-Threose, L-Threose, D-Ribose, L-Ribose, D-Arabinose, L-Arabinose, D-Xylose, L-Xylose, D-Lyxose, L-Lyxose, D-Allose, L-Allose, D-Altrose, L-Altrose, D-Glucose, L-Glucose, D-Mannose, L-Mannose, D-Gulose, L-Gulose, D-Idose, L-Idose, D-Galactose, L-Galactose, D-Talose, L-Talose, Dihydroxyaceton, D-Erythrulose, L-Erythrulose, D-Ribulose, L-Ribulose, D-Xylulose, L-Xylulose, D-Psicose, L-Psicose, D-Fructose, L-Fructose, D-Sorbose, L-Sorbose, D-Tagatose und L-Tagatose besteht, ausgewählt. Weiter bevorzugt werden geeignete Monosaccharide aus der Gruppe, die aus D-Ribose, L-Ribose, D-Arabinose, L-Arabinose, D-Xylose, L-Xylose, D-Lyxose, L-Lyxose, D-Allose, L-Allose, D-Altrose, L-Altrose, D-Glucose, L-Glucose, D-Mannose, L-Mannose, D-Gulose, L-Gulose, D-Idose, L-Idose, D-Galactose, L-Galactose, D-Talose, L-Talose, D-Ribulose, L-Ribulose, D-Xylulose, L-Xylulose, D-Psicose, L-Psicose, D-Fructose, L-Fructose, D-Sorbose, L-Sorbose, D-Tagatose und L-Tagatose besteht, ausgewählt werden.

Bei einer weiteren bevorzugten Ausführungsform des erfindungsgemäß zu verwendenden 10H-Benzo[g]pteridin-2,4-dion-Derivats der Formel (1) weisen die vorgenannten Carbonsäureester 1 bis 17 C-Atome, weiter bevorzugt 1 bis 15 C-Atome, weiter bevorzugt 1 bis 12 C-Atome, auf. Bei einer weiter bevorzugten Ausführungsform werden die vorgenannten Carbonsäureester aus der Gruppe, die aus Ethylester, n-Propylester, i-Propylester, n-Butylester, sec.-Butylester, tert.-Butylester und Benzylester besteht, ausgewählt.

Bei einer weiteren bevorzugten Ausführungsform des erfindungsgemäß zu verwendenden 10H-Benzo[g]pteridin-2,4-dion-Derivats der Formel (1) weisen die vorgenannten Carbonsäureamide 1 bis 17 C-Atome, weiter bevorzugt 1 bis 15 C-Atome, weiter bevorzugt 1 bis 12 C-Atome, auf. Bei einer weiter bevorzugten Ausführungsform werden die vorgenannten Carbonsäureamide aus der Gruppe, die aus Amid, N-Methylamid, N-Ethylamid, N-(n-Propyl)amid, N-(i-Propyl)amid, N-(n-Butyl)amid, N-(sec.-Butyl)amid, N-(tert.-Butyl)amid, N-Phenylamid, N-Benzylamid, N,N-Dimethylamid, N-Methyl-N-Ethyl-amid, N,N-Diethylamid, N-Methyl-N-(n-Propyl)amid, N-Methyl-N-(i-Propyl)amid, N-Methyl-N-(n-Butyl)amid, N-Methyl-N-(sec.-Butyl)amid, N-Methyl-N-(tert.-Butyl)amid, N-Ethyl-N-(n-Propyl)amid, N-Ethyl-N-(i-Propyl)amid, N-Ethyl-N-(n-Butyl)amid, N-Ethyl-N-(sec.-Butyl)amid, N-Ethyl-N-(tert.-Butyl)amid, N-(n-Propyl)-N-(n-Propyl)amid, N-(n-Propyl)-N-(i-Propyl)amid, N-(n-Propyl)-N-(n-Butyl)amid, N-(n-Propyl)-N-(sec.-Butyl)amid, N-(n-Propyl)-N-(tert.-Butyl)amid, N-(i-Propyl)-N-(n-Propyl)amid, N-(i-Propyl)-N-(i-Propyl)amid, N-(i-Propyl)-N-(n-Butyl)amid, N-(i-Propyl)-N-(sec.-Butyl)amid, N-(i-Propyl)-N-(tert.-Butyl)amid, N-(n-Butyl)-N-(n-Propyl)amid, N-(n-Butyl)-N-(i-Propyl)amid, N-(n-Butyl)-N-(n-Butyl)amid, N-(n-Butyl)-N-(sec.-Butyl)amid, N-(n-Butyl)-N-(tert.-Butyl)amid, N-(sec.-Butyl)-N-(n-Propyl)amid, N-(sec.-Butyl)-N-(i-Propyl)amid, N-(sec.-Butyl)-N-(n-Butyl)amid, N-( sec.-Butyl)-N-(sec.-Butyl)amid, N-(sec.-Butyl)-N-(tert.-Butyl)amid, N-(tert.-Butyl)-N-(n-Propyl)amid, N-(tert.-Butyl)-N-(i-Propyl)amid, N-(tert.-Butyl)-N-(n-Butyl)amid, N-( tert.-Butyl)-N-(sec.-Butyl)amid, N-(tert.-Butyl)-N-(tert.-Butyl)amid, N,N-Diphenylamid, N,N-Dibenzylamid, N-Phenyl-N-Benzylamid, N-Methyl-N-Phenylamid, N-Methyl-N-Benzylamid, N-Ethyl-N-Phenylamid, N-Ethyl-N-Benzylamid, N-Phenyl-N-(n-Propyl)amid, N-Phenyl-N-(i-Propyl)amid, N-Phenyl-N-(n-Butyl)amid, N-Phenyl-N-(sec.-Butyl)amid, N-Phenyl-N-(tert.-Butyl)amid, N-Benzyl-N-(n-Propyl)amid, N-Benzyl-N-(i-Propyl)amid, N-Benzyl-N-(n-Butyl)amid, N-Benzyl-N-(sec.-Butyl)amid und N-Benzyl-N-(tert.-Butyl)amid besteht, ausgewählt.

Bei einer weiteren bevorzugten Ausführungsform des erfindungsgemäß zu verwendenden 10H-Benzo[g]pteridin-2,4-dion-Derivats der Formel (1) wird der vorgenannten Heteroarylrest, der kein Stickstoffatom enthält, mit 4 bis 20 C-Atomen aus der Gruppe, die aus Thiophenyl, Furanyl, Benzothiofuranyl und Benzofuranyl besteht, ausgewählt.

Bei einer weiteren bevorzugten Ausführungsform des erfindungsgemäß zu verwendenden 10H-Benzo[g]pteridin-2,4-dion-Derivats der Formel (1) weisen die vorgenannten Etherreste 2 bis 17 C-Atome, weiter bevorzugt 2 bis 13 C-Atome, weiter bevorzugt 2 bis 9 C-Atome, auf. Bei einer weiter bevorzugten Ausführungsform werden die vorgenannten Etherreste beispielsweise aus der Gruppe, die aus Methoxymethyl, Methoxyethyl, Methoxy-n-propyl, Ethoxymethyl, n-Propoxymethyl, 2-Ethoxyethoxymethyl, 2-(2-Ethoxyethoxy)ethyl, i-Propoxymethyl, tert.-Butyloxymethyl, Dioxa-3,6-heptyl und Benzyloxymethyl besteht, ausgewählt. Bei einer weiter bevorzugten Ausführungsform können die vorgenannten Etherreste einfache Etherreste, Oligoetherreste, Polyetherreste oder Mischungen davon sein.

Bei einer weiteren bevorzugten Ausführungsform des erfindungsgemäß zu verwendenden 10H-Benzo[g]pteridin-2,4-dion-Derivats der Formel (1) weisen die vorgenannten Thioetherreste 2 bis 17 C-Atome, weiter bevorzugt 2 bis 13 C-Atome, weiter bevorzugt 2 bis 9 C-Atome, auf. Bei einer weiter bevorzugten Ausführungsform werden die vorgenannten Thioetherreste beispielsweise aus der Gruppe, Methylsulfanylmethyl, Methylsulfanylethyl, 3-Methylsulfanyl-n-propyl, Ethylsulfanylmethyl, n-Propylsulfanylmethyl, 2-Ethylsulfanylethylsulfanylmethyl, 2-(2-ethylsulfanylethylsulfanyl)ethyl, 2-Methylsulfanylpropyl, tert.-Butylsulfanymethyl und Benzylsulfanymethyl besteht, ausgewählt.

Bei einer weiteren bevorzugten Ausführungsform weist das erfindungsgemäß zu verwendende 10H-Benzo[g]pteridin-2,4-dion-Derivats der Formel (1) ein Molekulargewicht von weniger als 1300 g/mol, vorzugsweise weniger als 990 g/mol, weiter bevorzugt weniger als 810 g/mol, weiter bevorzugt weniger als 690 g/mol, noch weiter bevorzugt weniger als 610 g/mol, noch weiter bevorzugt weniger als 600 g/mol, noch weiter bevorzugt weniger als 570 g/mol, auf.

Chiralitätszentren können, wenn nicht anders angegeben, in der R- oder in der S-Konfiguration vorliegen. Die Erfindung betrifft sowohl die optisch reinen Verbindungen als auch Stereoisomerengemische, wie Enantiomerengemische und Diasteromerengemische, in jedem Verhältnis.

Die Erfindung betrifft vorzugsweise auch Mesomere und/oder Tautomere der Verbindung mit der Formel (1), sowohl die reinen Verbindungen als auch die Isomerengemische in jedem Verhältnis.

Bei einer weiteren bevorzugten Ausführungsform wird das erfindungsgemäß zu verwendende 10H-Benzo[g]pteridin-2,4-dion-Derivat der Formel (1) aus der Gruppe, die aus den Verbindungen mit den Formeln (30) bis (43) besteht, ausgewählt:

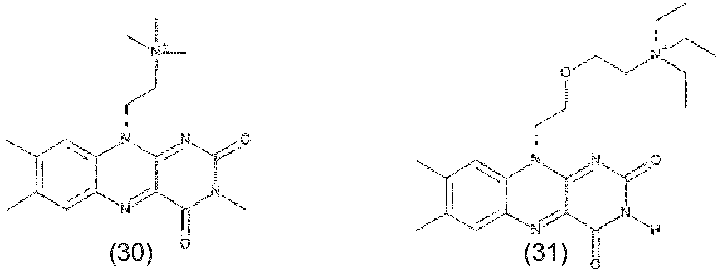

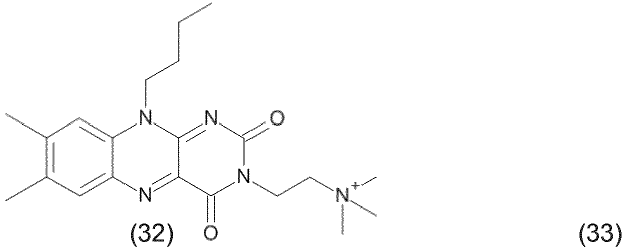

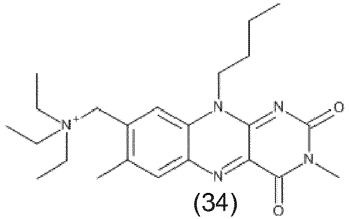

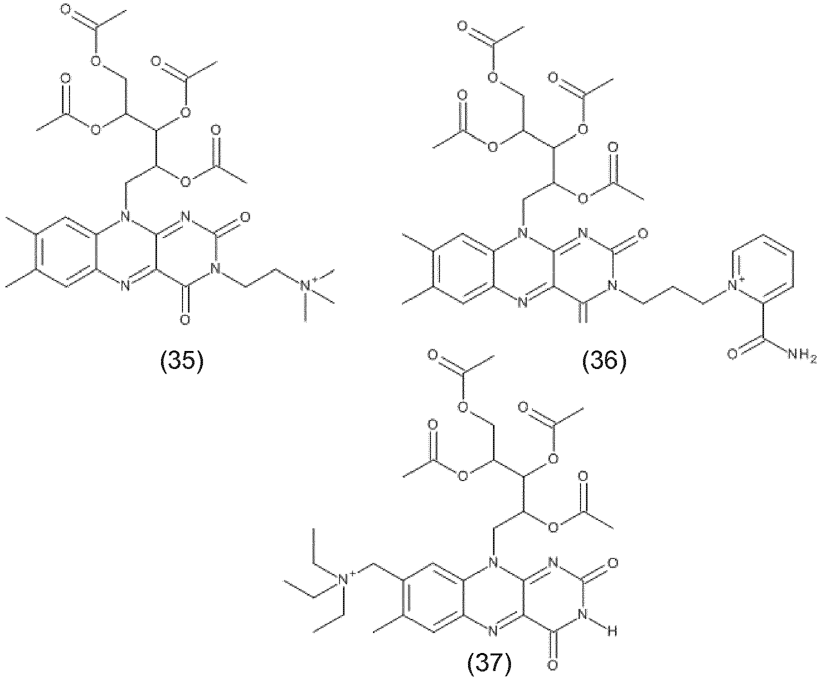

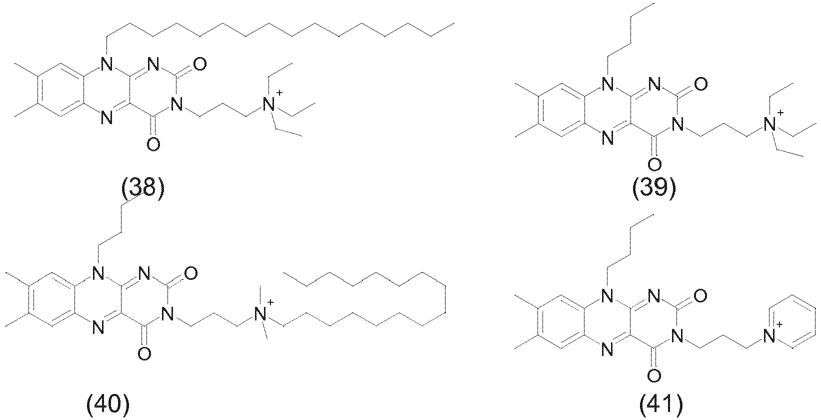

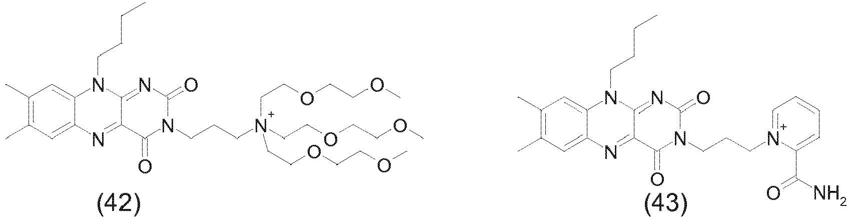

Eine Variante eines Verfahrens zur Herstellung eines 10H-Benzo[g]pteridin-2,4-dion-Derivats der Formel (1), umfasst folgende Schritte:
(A) Reduzieren eines substituierten Nitroanilins der Formel (22) zu einem substituierten o-Phenylendiamin der Formel (23), vorzugsweise durch Wasserstoff und Palladium auf Aktivkohle oder mit Zinn(II)chlorid, 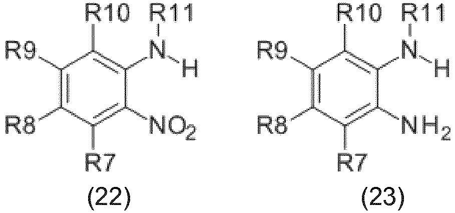 wobei jeder der Reste R7 bis R10, die jeweils unabhängig voneinander gleich oder voneinander verschieden sein können, Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das keine N-Atome enthält, oder ein organischen Rest der allgemeinen Formel -(C(D)(E))ₕ-OH, - (C(D)(E))ₖ-Aryl-(C(D)(E))ₗ-OH, -(C(D)(E))ₕ-X oder -(C(D)(E))ₖ-Aryl-(C(D)(E))ₗ-X bedeutet und
   wobei der Rest R11 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das keine N-Atome enthält, oder ein organischen Rest der allgemeinen Formel -(C(D)(E))ₕ-OH oder-(C(D)(E))ₖ-Aryl-(C(D)(E))ₗ-OH, -(C(D)(E))ₕ-X oder -(C(D)(E))ₖ-Aryl-(C(D)(E))ₗ-X bedeutet,
(B) Kondensieren des in Schritt (A) erhaltenen substituierten o-Phenylendiamin der Formel (23) mit Alloxan oder dessen Hydrat unter Erhalt einer Verbindung mit der Formel (24), optional in Gegenwart eines Katalysators, vorzugsweise Lewis-Säure oder Broenstedt-Säure, weiter bevorzugt Essigsäure in Gegenwart von Borsäure, 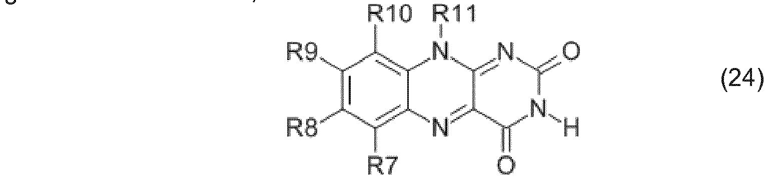
(C) Optional Umsetzen der in Schritt (B) erhaltenen Verbindung der Formel (24) mit einem Alkylierungsagens der allgemeinen Formel T-Alkyl, T-Alkenyl, T-Cycloalkyl, T-Cycloalkenyl, T-(C(D)(E))ₕ-OH, T-(C(D)(E))ₖ-Aryl-(C(D)(E))ₗ-OH, T-Aryl, T-(C(D)(E))ₕ-X oder T-(C(D)(E))ₖ-Aryl-(C(D)(E))ₗ-X, wobei der Rest T Wasserstoff, Chlor, Brom, Iod, p-Toluolsulfonyl (OTs), Methansulfonyl (OMs), OH oder R₂S⁺, wobei R jeweils unabhängig voneinander gleich oder verschieden sein kann und vorzugsweise Methyl, Ethyl, Propyl oder Butyl ist, bedeutet, unter Erhalt einer Verbindung mit der Formel (25): 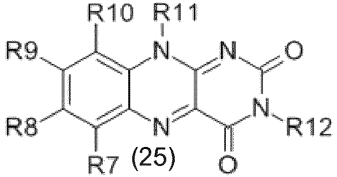
(D) Optional Umsetzen der in Schritt (B) erhaltenen Verbindung der Formel (24) oder der in Schritt (C) erhaltenen Verbindung der Formel (25) mit Tosylchlorid, Mesylchlorid oder Iod, optional in Gegenwart eines Katalysators, und nachfolgend mit einer organischen Verbindung, die wenigstens ein tertiäres Stickstoffatom enthält, wenn keiner der Reste R7 bis R12 ein organischer Rest der allgemeinen Formel - (C(D)(E))ₕ-X oder -(C(D)(E))ₖ-Aryl-(C(D)(E))ₗ-X ist und wenn 1 Rest R7 bis R12 ein organischer Rest der allgemeinen Formel -(C(D)(E))ₕ-OH oder -(C(D)(E))ₖ-Aryl-(C(D)(E))ₗ-OH ist, unter Erhalt des erfindungsgemäß zu verwendenden 10H-Benzo[g]pteridin-2,4-dion-Derivats der Formel (1),
   mit der Maßgabe, dass nur 1 Rest R1 bis R6 ein organischer Rest der allgemeinen Formel -(C(D)(E))ₕ-X oder -(C(D)(E))ₖ-Aryl-(C(D)(E))ₗ-X ist und wobei h jeweils eine ganze Zahl von 1 bis 20 ist und k, l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X jeweils ein organischer Rest mit nur einem quartären Stickstoffatom ist und Aryl jeweils einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet.

Eine weitere Variante eines Verfahrens zur Herstellung eines 10H-Benzo[g]pteridin-2,4-dion-Derivats der Formel (1), umfasst folgende Schritte:
(A) Herstellen eines substituierten Anilins mit der Formel (102a) oder (102b) durch (a) Peptidkupplung/Reduktion an ein Anilin mit der Formel (101) oder (b) reduktive Aminierung eines Anilins mit der Formel (101) mit Aldehyden oder (c) Pd-katalysierte Kupplung von einem Halogenid der Formel (104) an ein Amine der Formel R11-NH₂ oder (d) Pd-katalysierte Kupplung von einem Amin der Formel (100) an ein Halogenid der Formel R11-NH₂ 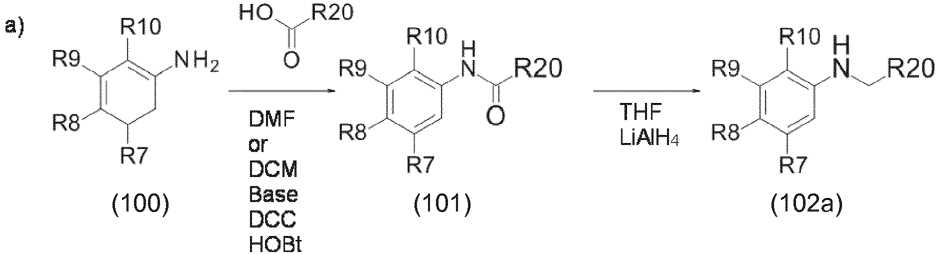 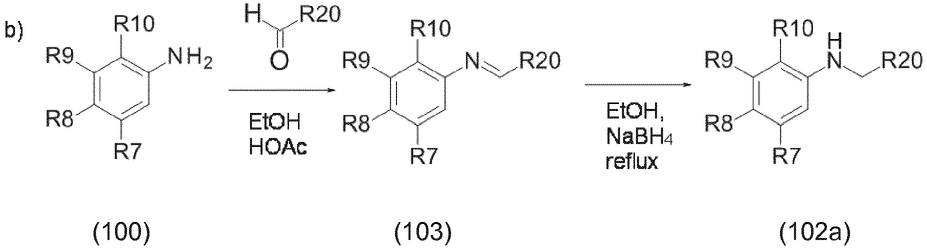 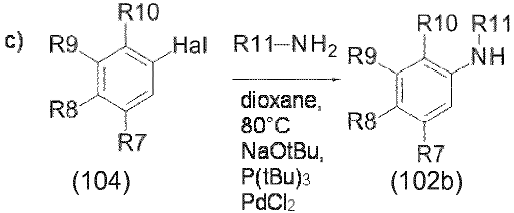 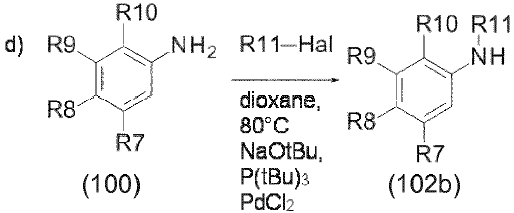 wobei jeder der Reste R7 bis R10, die jeweils unabhängig voneinander gleich oder voneinander verschieden sein können, Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das keine N-Atome enthält, oder ein organischen Rest der allgemeinen Formel -(C(D)(E))ₕ-OH, - (C(D)(E))ₖ-Aryl-(C(D)(E))ₗ-OH, -(C(D)(E))ₕ-X oder -(C(D)(E))ₖ-Aryl-(C(D)(E))ₗ-X bedeutet und
   wobei der Rest R11 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das keine N-Atome enthält, oder ein organischen Rest der allgemeinen Formel -(C(D)(E))ₕ-OH oder-(C(D)(E))ₖ-Aryl-(C(D)(E))ₗ-OH, -(C(D)(E))ₕ-X oder -(C(D)(E))ₖ-Aryl-(C(D)(E))ₗ-X bedeutet, und
   wobei der Rest R20 Wasserstoff, Alkyl mit 1 bis 19 C-Atomen, Alkenyl mit 2 bis 19 C-Atomen, Ether mit 1 bis 19 C-Atomen, Thioether mit 1 bis 19 C-Atomen, Cycloalkyl mit 3 bis 19 C-Atomen, Cycloalkenyl mit 3 bis 19 C-Atomen, Aryl mit 5 bis 19 C-Atomen, Heteroaryl mit 4 bis 19 C-Atomen, das keine N-Atome enthält, oder ein organischen Rest der allgemeinen Formel -(C(D)(E))ₕ₋₁-OH oder -(C(D)(E))ₖ₋₁-Aryl-(C(D)(E))ₗ₋₁-OH, -(C(D)(E))ₕ₋₁-X oder -(C(D)(E))ₖ₋₁-Aryl-(C(D)(E))ₗ₋₁-X bedeutet, und
   wobei der Rest Hal Fluor, Chlor, Brom oder Iod bedeutet,
(B) Umsetzen des in Schritt (A) erhaltenen substituierten Anilins mit der Formel (102a) mit Violursäure unter Erhalt einer Verbindung der Formel (24z): 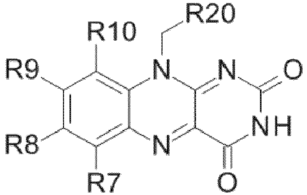 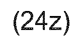 oder
   Umsetzen des in Schritt (A) erhaltenen substituierten Anilins mit der Formel (102b) mit Violursäure unter Erhalt einer Verbindung der Formel (24): 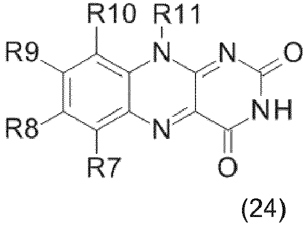
(C) Optional Umsetzen der in Schritt (B) erhaltenen Verbindung der Formel (24z) mit einem Alkylierungsagens der allgemeinen Formel T-Alkyl, T-Alkenyl, T-Cycloalkyl, T-Cycloalkenyl, T-(C(D)(E))ₕ-OH, T-(C(D)(E))ₖ-Aryl-(C(D)(E))ₗ-OH, T-Aryl, T-(C(D)(E))ₕ-X oder T-(C(D)(E))ₖ-Aryl-(C(D)(E))ₗ-X, wobei der Rest T Wasserstoff, Chlor, Brom, Iod, p-Toluolsulfonyl (OTs), Methansulfonyl (OMs), OH oder R₂S⁺, wobei R jeweils unabhängig voneinander gleich oder verschieden sein kann und vorzugsweise Methyl, Ethyl, Propyl oder Butyl ist, bedeutet, unter Erhalt einer Verbindung mit der Formel (25z): 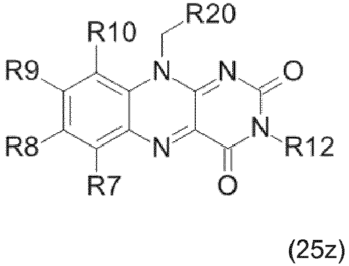 oder
   Umsetzen der in Schritt (B) erhaltenen Verbindung der Formel (24) mit einem Alkylierungsagens der allgemeinen Formel T-Alkyl, T-Alkenyl, T-Cycloalkyl, T-Cycloalkenyl, T-(C(D)(E))ₕ-OH, T-(C(D)(E))ₖ-Aryl-(C(D)(E))ₗ-OH, T-Aryl, T-(C(D)(E))ₕ-X oder T-(C(D)(E))ₖ-Aryl-(C(D)(E))ₗ-X, wobei der Rest T Wasserstoff, Chlor, Brom, Iod, p-Toluolsulfonyl (OTs), Methansulfonyl (OMs), OH oder R₂S⁺, wobei R jeweils unabhängig voneinander gleich oder verschieden sein kann und vorzugsweise Methyl, Ethyl, Propyl oder Butyl ist, bedeutet, unter Erhalt einer Verbindung mit der Formel (25),: 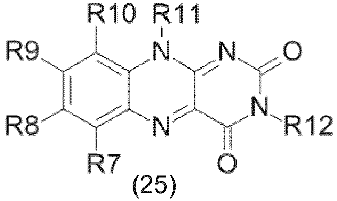
(D) Optional Umsetzen der in Schritt (B) erhaltenen Verbindung der Formel (24z) oder (24) oder der in Schritt (C) erhaltenen Verbindung der Formel (25z) oder (25) mit Tosylchlorid, Mesylchlorid oder Iod, optional in Gegenwart eines Katalysators, und nachfolgend mit einer organischen Verbindung, die wenigstens ein tertiäres Stickstoffatom enthält, wenn keiner der Reste R7 bis R12 bzw. R7 bis R11 und R20 ein organischer Rest der allgemeinen Formel -(C(D)(E))ₕ-X oder-(C(D)(E))ₖ-Aryl-(C(D)(E))ₗ-X ist und wenn 1 Rest R7 bis R12 bzw. R7 bis R11 oder R20 ein organischer Rest der allgemeinen Formel -(C(D)(E))ₕ-OH oder-(C(D)(E))ₖ-Aryl-(C(D)(E))ₗ-OH ist, unter Erhalt des erfindungsgemäß zu verwendenden 10H-Benzo[g]pteridin-2,4-dion-Derivats der Formel (1),
   mit der Maßgabe, dass nur 1 Rest R1 bis R6 ein organischer Rest der allgemeinen Formel -(C(D)(E))ₕ-X oder -(C(D)(E))ₖ-Aryl-(C(D)(E))ₗ-X ist und wobei h jeweils eine ganze Zahl von 1 bis 20 ist und k, l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X jeweils ein organischer Rest mit nur einem quartären Stickstoffatom ist und Aryl jeweils einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet.

Eine weitere Variante eines Verfahrens zur Herstellung eines 10H-Benzo[g]pteridin-2,4-dion-Derivats der Formel (1), umfasst folgende Schritte:
(A) Kondensieren eines Amins mit der Formel R11-NH₂ mit einem Chloruracil-Derivate der Formel (26), optional in Gegenwart eines Katalysators, vorzugsweise Lewis-Säure oder Broenstedt-Säure, unter Erhalt einer Verbindung mit der Formel (27): 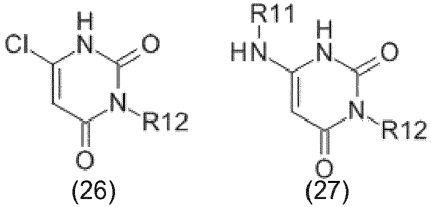 wobei jeder der Reste R11 oder R12 jeweils unabhängig voneinander gleich oder voneinander verschieden sein kann und Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das keine N-Atome enthält, oder ein organischen Rest der allgemeinen Formel -(C(D)(E))ₕ-OH oder - (C(D)(E))ₖ-Aryl-(C(D)(E))ₗ-OH, -(C(D)(E))ₕ-X oder -(C(D)(E))ₖ-Aryl-(C(D)(E))ₗ-X bedeutet,
(B) Umsetzen der in Schritt (A) erhaltenen Verbindung der Formel (27) mit einer Nitrosoverbindung der Formel (28) unter Erhalt einer Verbindung der Formel (25): 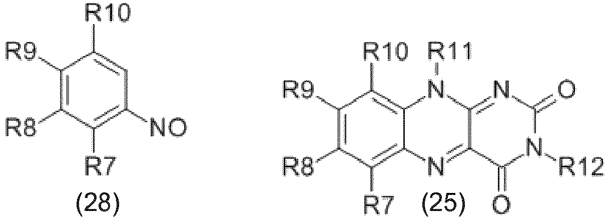 wobei jeder der Reste R7 bis R10 jeweils unabhängig voneinander gleich oder voneinander verschieden sein können und Wasserstoff, Halogen, Hydroxyl, Thiol, Nitro, Carboxy, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das keine N-Atome enthält, oder ein organischen Rest der allgemeinen Formel -(C(D)(E))ₕ-OH, - (C(D)(E))ₖ-Aryl-(C(D)(E))ₗ-OH, -(C(D)(E))ₕ-X oder -(C(D)(E))ₖ-Aryl-(C(D)(E))ₗ-X bedeutet, und
(C) Optional Umsetzen der in Schritt (B) erhaltenen Verbindung der Formel (25) mit Tosylchlorid, Mesylchlorid oder Iod, optional in Gegenwart eines Katalysators, und nachfolgend mit einer organischen Verbindung, die wenigstens ein tertiäres Stickstoffatom enthält, wenn keiner der Reste R7 bis R12 ein organischer Rest der allgemeinen Formel -(C(D)(E))ₕ-X oder -(C(D)(E))ₖ-Aryl-(C(D)(E))ₗ-X ist und wenn 1 Rest R7 bis R12 ein organischer Rest der allgemeinen Formel -(C(D)(E))ₕ-OH oder -(C(D)(E))ₖ-Aryl-(C(D)(E))ₗ-OH ist, unter Erhalt des erfindungsgemäß zu verwendenden 10H-Benzo[g]pteridin-2,4-dion-Derivats der Formel (1),
   mit der Maßgabe, dass nur 1 Rest R7 bis R12 ein organischer Rest der allgemeinen Formel -(C(D)(E))ₕ-X oder -(C(D)(E))ₖ-Aryl-(C(D)(E))ₗ-X ist und wobei h eine ganze Zahl von 1 bis 20 und k, l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet.

Bei einer möglichenAusführungsform der beiden Verfahrensvarianten ist keiner der Reste R7 bis R12 bzw. R7 bis R11 und R20 ein organischer Rest der allgemeinen Formel -(C(D)(E))ₕ-X oder -(C(D)(E))ₖ-Aryl-(C(D)(E))ₗ-X und 1 Rest R7 bis R10 ist Methyl wobei das Verfahren dann folgende Schritte umfassen kann,
(A) Radikalisches Halogenieren der Verbindung (24) oder (25) in Gegenwart eines Radikalstarters, vorzugsweise eines Peroxides oder einer AzoVerbindung, unter Erhalt einer Verbindung mit der Formel (24a-d) oder (25a-d): 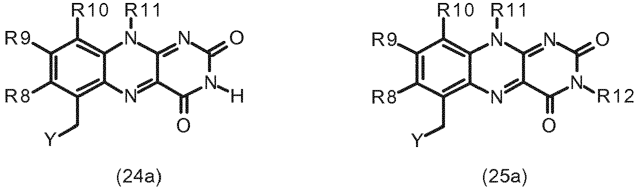 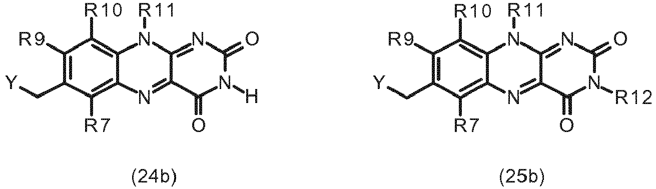 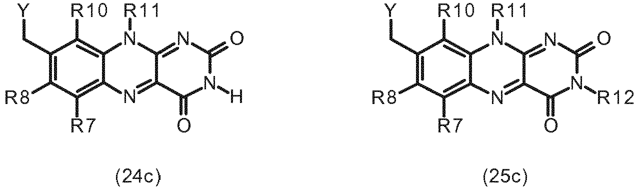 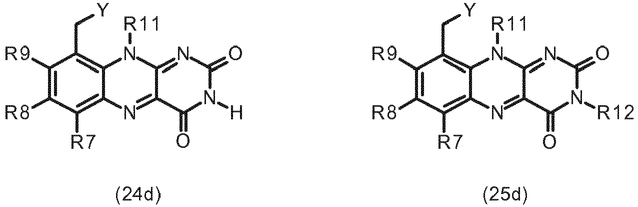 wobei der Rest Y Cl, Br oder I bedeutet, und
B) Umsetzen der in Schritt (A) erhaltenen Verbindung mit der Formel (24a-d) oder (25a-d) mit einer organischen Verbindung, die wenigstens ein tertiäres Stickstoffatom enthält, unter Erhalt des erfindungsgemäß zu verwendenden 10H-Benzo[g]pteridin-2,4-dion-Derivats der Formel (1).

Bei der Verwendung unterschiedlicher Amino-Schutzgruppe PG in einer Synthese, ergibt sich die Möglichkeit der orthogonalen Schutzgruppenstrategie, wobei unterschiedliche Amino-Funktionen eines Moleküls gezielt nacheinander freigesetzt können und zur Reaktion gebracht werden.

Geeignete Methoden zur Entfernung der Amino-Schutzgruppe PG sind aus dem Stand der Technik bekannt. Beispielsweise kann Benzyloxycarbonyl (Cbz) durch katalytische Hydrierung unter hydrogenolytischer Spaltung der Benzyl-Heteroatom-Bindung mit anschließender Decarboxylierung der so entstehenden instabilen Carbaminsäure oder Behandlung mit Säuren wieder entfernt werden. Di-tert-butyloxycarbonyl (Boc) kann beispielsweise durch saure Hydrolyse entfernt werden. Allyloxycarbonyl (Alloc) kann beispielsweise durch Einwirkung von Tetrakis(triphenylphosphan)palladium(0) und einem Nukleophil abgespalten werden.

Die Schritte (B) und/oder (C) können in Gegenwart eines oder mehrerer Lösungsmittel statt. Der Schritt (B) kann beispielsweise in Gegenwart von Dichlormethan (DCM) oder Tetrahydrofuran (THF) durchgeführt werden, vorzugsweise in Gegenwart einer Base, wie zum Beispiel Kaliumcarbonat. Alternativ kann der Schritt (B) beispielsweise in Gegenwart von Dimethylformamid (DMF) und Triphenylphosphin (PPh3) und/oder Kaliumiodid durchgeführt werden.

Der Schritt (C) kann beispielsweise in Gegenwart von Wasser/Dichlormethan oder Toluol/Tetrabutylammoiumiodid (TBAI) durchgeführt werden.

Einzellige oder mehrzellige Mikroorganismen können Auslöser von infektiösen Erkrankungen sein. Durch Applikation wenigstens eines Erreger-spezifischen Gegenmittels, beispielsweise Antibiotikum, Antimikotikum oder Virustatikum, kann die Anzahl der Erreger reduziert und/oder der Erreger inaktiviert werden. Die Applikation eines Erreger-spezifischen Gegenmittels kann systemisch und/oder topisch erfolgen.

Bei der systemischen Applikation wird das Erreger-spezifische Gegenmittel in das Blut- und/oder Lymphsystem des zu behandelnden Körpers übertragen und hierüber im gesamten Körper verteilt. Bei einer systemischen Aufnahme des Erreger-spezifischen Gegenmittels kann es zu einem Abbau des Gegenmittels und/oder zu Nebenwirkungen, beispielsweise durch eine biochemische Umwandlung (Metabolisierung) des Gegenmittels, kommen.

Bei der topischen Applikation des Erreger-spezifischen Gegenmittels erfolgt die Anwendung des Gegenmittels dort, wo es therapeutisch wirken soll, beispielsweise auf einer infizierten Hautpartie, während die gesunde Haut nicht belastet wird. Somit können systemische Nebenwirkungen weitgehend vermieden werden.

Oberflächliche Haut- oder Weichteilinfektionen müssen nicht notwendigerweise mit, einer systemischen Applikation eines Erreger-spezifischen Gegenmittels behandelt werden, da das Gegenmittel direkt auf die infizierte Hautpartien aufgetragen werden kann.

Die bisher bekannten Erreger-spezifischen Gegenmittel weisen sowohl bei systemischer als auch topischer Applikation teilweise starke Neben- und Wechselwirkungen auf. Darüber hinaus kann es auch bei topischer Applikation durch eine unzuverlässige Medikamenteneinnahme (Compliance) des Patienten, insbesondere bei Verwendung von Antibiotika, zur Resistenzbildung kommen.

Eine Alternative stellt hier die photodynamische Inaktivierung von Mikroorganismen dar, bei der Resistenzen gegenüber der photodynamischen Inaktivierung unbekannt sind. Unabhängig von der Art der zu bekämpfenden Mikroorganismen und der damit verbundenen infektiösen Erkrankungen wird die Anzahl der Erreger reduziert und/oder die Erreger werden abtötet. Beispielsweise können Mischungen aus verschieden Mikroorganismen, beispielsweise Pilze und Bakterien oder unterschiedliche Bakterienstämme, bekämpft werden.

Das erfindungsgemäß zu verwendende 10H-Benzo[g]pteridin-2,4-dion-Derivat der Formel (1) weist nach Bestrahlung mit elektromagnetischer Strahlung geeigneter Wellenlänge und Energiedichte eine hohe Ausbeute an Singulett-Sauerstoff auf.

Vorzugsweise liegt die elektromagnetische Strahlung im sichtbaren Spektralbereich, ultravioletten und/oder infraroten Bereich. Weiter bevorzugt weist die elektromagnetische Strahlung eine Wellenlänge aus einem Bereich von 280 bis 1000 nm, weiter bevorzugt von 380 bis 1000 nm, auf.

Weiter bevorzugt weist die elektromagnetische Strahlung eine Energiedichte aus einem Bereich von 1 µW/cm² bis 1 MW/cm², weiter bevorzugt von 1 mW/cm² bis 1 kW/cm², auf.

Die Bestrahlungszeit kann in Abhängigkeit von der Art der Mikroorganismen und/oder der Schwere der Infektion variiert werden. Vorzugsweise liegt die Bestrahlungszeit in einem Bereich von 1 µs bis 1 h, weiter bevorzugt von 1 ms bis 1000 s.

Vorzugsweise wird die elektromagnetische Strahlung durch eine Strahlungsquelle erzeugt, die aus der Gruppe, die aus Sonne und künstlichen Strahlungsquellen, beispielsweise UV-Lampe, IR-Lampe, Leuchtstofflampen, Leuchtdioden, Laser oder chemisches Licht, besteht, ausgewählt wird.

Darüber hinaus haben die Erfinder überraschenderweise festgestellt, dass das erfindungsgemäß zu verwendende 10H-Benzo[g]pteridin-2,4-dion-Derivat der Formel (1) oder pharmakologisch verträgliche Salze und/oder Ester und/oder Komplexe davon vorzugsweise eine hohe Affinität zu Mikroorganismen aufweist.

Auf Grund der Affinität kann das erfindungsgemäß zu verwendende 10H-Benzo[g]pteridin-2,4-dion-Derivats der Formel (1) an Mikroorganismen effektiv binden und lokal ausreichend Singulett-Sauerstoff erzeugen, um die Mikroorganismen zu inaktivieren, vorzugsweise abzutöten.

Bei dieser bevorzugten Verwendung als Photosensibilisator wird wenigstens ein erfindungsgemäß zu verwendendes 10H-Benzo[g]pteridin-2,4-dion-Derivat der Formel (1) von Mikroorganismen gebunden. Nach Bestrahlung mit elektromagnetischer Strahlung geeigneter Wellenlänge und Energiedichte werden die Mikroorganismen durch die entstandenen reaktiven Sauerstoffspezies (ROS), vorzugsweise Sauerstoffradikale und/oder Singulett-Sauerstoff, inaktiviert, vorzugsweise abgetötet.

Vorzugsweise erlaubt die Bindung wenigstens eines erfindungsgemäß zu verwendenden 10H-Benzo[g]pteridin-2,4-dion-Derivates der Formel (1) an Mikroorganismen ebenfalls eine Anfärbung oder Lokalisierung von Mikroorganismen. Dadurch kann vorzugsweise auch der Verlauf der Inaktivierung von Mikroorganismen oder der Dekolonisation verfolgt werden.

Erfindungsgemäß wird unter dem Begriff "Dekolonisation" das Entfernen, vorzugsweise vollständige Entfernen, von Mikroorganismen verstanden.

Bei einer weiteren bevorzugten Ausführungsform wird wenigstens ein erfindungsgemäß verwendendes 10H-Benzo[g]pteridin-2,4-dion-Derivat der Formel (1) oder ein pharmakologisch verträgliches Salz und/oder Ester und/oder Komplex davon bei der Inaktivierung von einzelligen oder mehrzelligen Mikroorganismen, die vorzugsweise aus der Gruppe, die aus Viren, Archäeen, Bakterien, Bakteriensporen, Pilzen, beispielsweise Myzelpilze und Hefen, Pilzsporen, Protozoen, Algen und blutübertragbaren Parasiten besteht, ausgewählt werden, verwendet.

Bei einer weiteren bevorzugten Ausführungsform wird wenigstens ein erfindungsgemäß zu verwendendes 10H-Benzo[g]pteridin-2,4-dion-Derivat der Formel (1) oder ein pharmakologisch verträgliches Salz und/oder Ester und/oder Komplex davon bzw. eine pharmazeutische Zubereitung enthaltend wenigstens ein erfindungsgemäß zu verwendendes 10H-Benzo[g]pteridin-2,4-dion-Derivat der Formel (1) oder ein pharmakologisch verträgliches Salz und/oder Ester und/oder Komplex davon bei der Reinigung von Zähnen, Zahnersatz und/oder Zahnspangen verwendet.

Wenigstens ein erfindungsgemäß zu verwendendes 10H-Benzo[g]pteridin-2,4-dion-Derivat der Formel (1) oder ein pharmakologisch verträgliches Salz und/oder Ester und/oder Komplex davon wird zur Inaktivierung von Mikroorganismen auf Oberflächen aller Art verwendet. Weiter bevorzugt wird das erfindungsgemäß zu verwendende 10H-Benzo[g]pteridin-2,4-dion-Derivat der Formel (1) oder ein pharmakologisch verträgliches Salz und/oder Ester und/oder Komplex davon bei der Oberflächenreinigung und/oder -beschichtung, vorzugsweise von Medizinprodukten, Lebensmittelverpackungen oder Hygieneartikeln, verwendet.

Weiter bevorzugt wird wenigstens ein erfindungsgemäß zu verwendendes 10H-Benzo[g]pteridin-2,4-dion-Derivat der Formel (1) oder ein pharmakologisch verträgliches Salz und/oder Ester und/oder Komplex davon auf Oberflächen aufgebracht und/oder eingebracht und, optional, nachfolgend mit einer geeigneten Strahlenquelle, die elektromagnetische Strahlung geeigneter Wellenlänge und Energiedichte erzeugt, bestrahlt. Vorzugsweise bewirkt das wenigstens eine erfindungsgemäß zu verwendende 10H-Benzo[g]pteridin-2,4-dion-Derivat der Formel (1) oder ein pharmakologisch verträgliches Salz und/oder Ester und/oder Komplex davon während der Bestrahlung eine "Selbstdesinfektion" der Oberfläche.

Die Bestrahlung kann dabei direkt nach der Behandlung der Oberfläche mit wenigstens einem erfindungsgemäß zu verwendenden 10H-Benzo[g]pteridin-2,4-dion-Derivat der Formel (1) oder ein pharmakologisch verträgliches Salz und/oder Ester und/oder Komplex davon, vorzugsweise nach dem Aufbringen des wenigstens einen erfindungsgemäß zu verwendenden 10H-Benzo[g]pteridin-2,4-dion-Derivates der Formel (1) oder ein pharmakologisch verträgliches Salz und/oder Ester und/oder Komplex davon auf die Oberfläche und/oder Einbringen des wenigstens einen erfindungsgemäß zu verwendenden 10H-Benzo[g]pteridin-2,4-dion-Derivates der Formel (1) oder ein pharmakologisch verträgliches Salz und/oder Ester und/oder Komplex davon in die Oberfläche, erfolgen und/oder zu einem späteren Zeitpunkt.

Weiter bevorzugt werden Gegenstände behandelt, die eine thermisch begrenzte Haltbarkeit aufweisen, beispielsweise Gegenstände aus thermoplastische Kunststoffen, oder von Desinfektionsmitteln angegriffen werden.

Gegenstände, die eine thermisch begrenzte Haltbarkeit aufweisen, können beispielsweise nur unzureichend sterilisiert werden, da sie bei höheren Temperaturen ihre Form verlieren oder spröde werden.

Darüber hinaus kann es bei einer unsachgemäßen und/oder übermäßigen Anwendung von Desinfektionsmitteln zu Resistenzbildung durch Selektion robuster Mikroorganismen kommen, wenn beispielsweise die Wirkstoffkonzentration und Einwirkzeit und damit die keimreduzierende Wirkung zu gering ist.

Bei einer weiter bevorzugten Ausführungsform wird wenigstens ein erfindungsgemäß zu verwendendes 10H-Benzo[g]pteridin-2,4-dion-Derivat der Formel (1) oder ein pharmakologisch verträgliches Salz und/oder Ester und/oder Komplex davon zur Inaktivierung von Mikroorganismen auf Oberflächen von Medizinprodukten, vorzugsweise invasiven medizinischen Hilfsmitteln wie etwa Kathetern, Hohlsonden, Schläuchen oder Nadeln, verwendet.

Vorzugsweise werden die Medizinprodukte aus Wundauflagen, Verbände, Kathetern, Hohlsonden, Schläuchen oder Nadeln ausgewählt.

Weiter bevorzugt werden unter Medizinprodukten auch zahnärztliche Abdrücke oder Zahnersatz, beispielsweise Prothesen, Kronen oder Implantate, verstanden.

Vorzugsweise wird durch eine Behandlung der Oberfläche von Medizinprodukten mit wenigstens einem 10H-Benzo[g]pteridin-2,4-dion-Derivat der Formel (1) oder ein pharmakologisch verträgliches Salz und/oder Ester und/oder Komplex davon und/oder Beschichtung und/oder Immobilisierung wenigstens eines erfindungsgemäß zu verwendenden 10H-Benzo[g]pteridin-2,4-dion-Derivates der Formel (1) oder ein pharmakologisch verträgliches Salz und/oder Ester und/oder Komplex davon auf der Oberfläche von Medizinprodukten und nachfolgender Bestrahlung mit elektromagnetische Strahlung geeigneter Wellenlänge und Energiedichte die Besiedelung von Mikroorganismen auf der behandelten Oberflächen reduziert, vorzugsweise verhindert.

Die Bestrahlung kann dabei direkt nach der Behandlung der Oberfläche mit wenigstens einem erfindungsgemäß zu verwendenden 10H-Benzo[g]pteridin-2,4-dion-Derivat der Formel (1) oder ein pharmakologisch verträgliches Salz und/oder Ester und/oder Komplex davon, vorzugsweise nach dem Aufbringen des wenigstens einen erfindungsgemäß zu verwendenden 10H-Benzo[g]pteridin-2,4-dion-Derivates der Formel (1) oder ein pharmakologisch verträgliches Salz und/oder Ester und/oder Komplex davon auf die Oberfläche und/oder Einbringen des wenigstens einen erfindungsgemäß zu verwendenden 10H-Benzo[g]pteridin-2,4-dion-Derivates der Formel (1) oder ein pharmakologisch verträgliches Salz und/oder Ester und/oder Komplex davon in die Oberfläche, erfolgen und/oder zu einem späteren Zeitpunkt, vor oder während der Verwendung des behandelten Medizinproduktes.

Bei einer weiter bevorzugten Verwendung des wenigstens einen erfindungsgemäß zu verwendenden 10H-Benzo[g]pteridin-2,4-dion-Derivates der Formel (1) oder eines pharmakologisch verträgliches Salzes und/oder Esters und/oder Komplexes davon in Wundauflagen und/oder Verbänden, beispielsweise Baumwollgaze, kann während oder nach dem Aufbringen einer Wundauflage und/oder Verbandes, die bzw. der wenigstens ein erfindungsgemäß zu verwendendes 10H-Benzo[g]pteridin-2,4-dion-Derivat der Formel (1) oder ein pharmakologisch verträgliches Salz und/oder Ester und/oder Komplex davon enthält, eine Bestrahlung mit elektromagnetische Strahlung geeigneter Wellenlänge und Energiedichte erfolgen wodurch es nachfolgend zu einer Reduzierung, vorzugsweise Inaktivierung, von Mikroorganismen im Wundbereich oder behandelten Hautpartien kommen.

Bei einer weiteren bevorzugten Ausführungsform umfasst die Wundauflage und/oder Verband neben wenigstens einem erfindungsgemäß zu verwendenden 10H-Benzo[g]pteridin-2,4-dion-Derivat der Formel (1) oder eines pharmakologisch verträgliches Salzes und/oder Esters und/oder Komplexes davon weitere Bestandteile, vorzugsweise Absorbtionsmittel, beispielsweise Calciumalginat oder Polyurethanschaum, oder weitere pharmazeutisch wirksame Substanzen.

Bei einer weiteren bevorzugten Ausführungsform wird wenigstens ein erfindungsgemäß zu verwendendes 10H-Benzo[g]pteridin-2,4-dion-Derivat der Formel (1) oder ein pharmakologisch verträgliches Salz und/oder Ester und/oder Komplex davon zur Inaktivierung von Mikroorganismen auf Oberflächen von Lebensmittelverpackungen verwendet.

Des Weiteren betrifft die vorliegende Erfindung einen beschichteten Gegenstand, der wenigstens ein erfindungsgemäß zu verwendendes 10H-Benzo[g]pteridin-2,4-dion-Derivat der Formel (1) oder ein pharmakologisch verträgliches Salz und/oder Ester und/oder Komplex davon enthält und/oder damit beschichtet ist.

Vorzugsweise weist die Oberfläche des beschichteten Gegenstands wenigstens ein erfindungsgemäß zuverwendendes 10H-Benzo[g]pteridin-2,4-dion-Derivat der Formel (1) oder ein pharmakologisch verträgliches Salz und/oder Ester und/oder Komplex davon auf.

Der beschichtete Gegenstand kann nachfolgend mit einer geeigneten Strahlenquelle, die elektromagnetische Strahlung geeigneter Wellenlänge und Energiedichte erzeugt, bestrahlt werden. Vorzugsweise bewirkt das erfindungsgemäß zu verwendende 10H-Benzo[g]pteridin-2,4-dion-Derivat der Formel (1) oder ein pharmakologisch verträgliches Salz und/oder Ester und/oder Komplex davon während der Bestrahlung eine "Selbst-desinfektion" der Oberfläche des beschichteten Gegenstandes.

Die Bestrahlung kann dabei direkt nach der Behandlung des beschichteten Gegenstandes mit wenigstens einem erfindungsgemäß zu verwendenden 10H-Benzo[g]pteridin-2,4-dion-Derivat der Formel (1) oder ein pharmakologisch verträgliches Salz und/oder Ester und/oder Komplex davon, vorzugsweise nach dem Aufbringen des wenigstens einen erfindungsgemäß zu verwendenden 10H-Benzo[g]pteridin-2,4-dion-Derivates der Formel (1) oder ein pharmakologisch verträgliches Salz und/oder Ester und/oder Komplex davon auf die Oberfläche des beschichteten Gegenstandes und/oder Einbringen des wenigstens einen erfindungsgemäß zu verwendenden 10H-Benzo[g]pteridin-2,4-dion-Derivates der Formel (1) oder ein pharmakologisch verträgliches Salz und/oder Ester und/oder Komplex davon in die Oberfläche des beschichteten Gegenstandes, erfolgen und/oder zu einem späteren Zeitpunkt, vorzugsweise vor oder während der Verwendung des beschichteten Gegenstandes.

Geeignete Gegenstände werden vorzugsweise aus der Gruppe, die aus Medizinprodukten, Lebensmittelverpackungen oder Hygieneartikeln, besteht, ausgewählt.

Bei einer weiteren bevorzugten Ausführungsform des beschichteten Gegenstandes handelt es sich um mit wenigstens einem erfindungsgemäß zu verwendenden 10H-Benzo[g]pteridin-2,4-dion-Derivat der Formel (1) oder ein pharmakologisch verträgliches Salz und/oder Ester und/oder Komplex davon beschichtete Partikel, beispielsweise anorganische oder organische Partikel.

Weiter bevorzugt umfassen die Partikel wenigstens ein erfindungsgemäß zu verwendendes 10H-Benzo[g]pteridin-2,4-dion-Derivat der Formel (1) oder ein pharmakologisch verträgliches Salz und/oder Ester und/oder Komplex davon, dass an die Partikel kovalent gebunden vorliegt.

Die Erfindung wird nachfolgend durch Figuren und Beispiele erläutert, ohne hierauf beschränkt zu sein.

Bei einer weiter bevorzugten Ausführungsform wird wenigstens ein erfindungsgemäßes 10H-Benzo[g]pteridin-2,4-dion-Derivat der Formel (1) oder ein pharmakologisch verträgliches Salz und/oder Ester und/oder Komplex davon zur Inaktivierung von Mikroorganismen auf Oberflächen von Medizinprodukten, vorzugsweise invasiven medizinischen Hilfsmitteln wie etwa Kathetern, Hohlsonden, Schläuchen oder Nadeln, verwendet.

Vorzugsweise werden die Medizinprodukte aus Wundauflagen, Verbände, Kathetern, Hohlsonden, Schläuchen oder Nadeln ausgewählt.

Weiter bevorzugt werden unter Medizinprodukten auch zahnärztliche Abdrücke oder Zahnersatz, beispielsweise Prothesen, Kronen oder Implantate, verstanden.

Vorzugsweise wird durch eine Behandlung der Oberfläche von Medizinprodukten mit wenigstens einem 10H-Benzo[g]pteridin-2,4-dion-Derivat der Formel (1) oder ein pharmakologisch verträgliches Salz und/oder Ester und/oder Komplex davon gemäß der Erfindung und/oder Beschichtung und/oder Immobilisierung wenigstens eines erfindungsgemäßen 10H-Benzo[g]pteridin-2,4-dion-Derivates der Formel (1) oder ein pharmakologisch verträgliches Salz und/oder Ester und/oder Komplex davon auf der Oberfläche von Medizinprodukten und nachfolgender Bestrahlung mit elektromagnetische Strahlung geeigneter Wellenlänge und Energiedichte die Besiedelung von Mikroorganismen auf der behandelten Oberflächen reduziert, vorzugsweise verhindert.

Die Bestrahlung kann dabei direkt nach der Behandlung der Oberfläche mit wenigstens einem erfindungsgemäßen 10H-Benzo[g]pteridin-2,4-dion-Derivat der Formel (1) oder ein pharmakologisch verträgliches Salz und/oder Ester und/oder Komplex davon, vorzugsweise nach dem Aufbringen des wenigstens einen erfindungsgemäßen 10H-Benzo[g]pteridin-2,4-dion-Derivates der Formel (1) oder ein pharmakologisch verträgliches Salz und/oder Ester und/oder Komplex davon auf die Oberfläche und/oder Einbringen des wenigstens einen erfindungsgemäßen 10H-Benzo[g]pterid!n-2,4-dion-Derivates der Formel (1) oder ein pharmakologisch verträgliches Salz und/oder Ester und/oder Komplex davon in die Oberfläche, erfolgen und/oder zu einem späteren Zeitpunkt, vor oder während der Verwendung des behandelten Medizinproduktes.

Bei einer weiter bevorzugten Verwendung des wenigstens einen erfindungsgemäßen 10H-Benzo[gjpteridin-2,4-dion-Derivates der Formel (1) oder eines pharmakologisch verträgliches Salzes und/oder Esters und/oder Komplexes davon in Wundauflagen und/oder Verbänden, beispielsweise Baumwollgaze, kann während oder nach dem Aufbringen einer Wundauflage und/oder Verbandes, die bzw. der wenigstens ein erfindungsgemäßes 10H-Benzo[g]pteridin-2,4-dion-Derivat der Formel (1) oder ein pharmakologisch verträgliches Salz und/oder Ester und/oder Komplex davon enthält, eine Bestrahlung mit elektromagnetische Strahlung geeigneter Wellenlänge und Energiedichte erfolgen wodurch es nachfolgend zu einer Reduzierung, vorzugsweise Inaktivierung, von Mikroorganismen im Wundbereich oder behandelten Hautpartien kommen.

Bei einer weiteren bevorzugten Ausführungsform umfasst die Wundauflage und/oder Verband neben wenigstens einem erfindungsgemäßen 10H-Benzo[g]pteridin-2,4-dion-Derivat der Formel (1) oder eines pharmakologisch verträgliches Salzes und/oder Esters und/oder Komplexes davon weitere Bestandteile, vorzugsweise Absorbtionsmittel, beispielsweise Calciumalginat oder Polyurethanschaum, oder weitere pharmazeutisch wirksame Substanzen.

Bei einer weiteren bevorzugten Ausführungsform wird wenigstens ein erfindungsgemäßes 10H-Benzo[g]pteridin-2,4-dion-Derivat der Formel (1) oder ein pharmakologisch verträgliches Salz und/oder Ester und/oder Komplex davon zur Inaktivierung von Mikroorganismen auf Oberflächen von Lebensmittelverpackungen verwendet.

Bei einer weiteren bevorzugten Ausführungsform wird wenigstens ein erfindungsgemäßes 10H-Benzo[g]pteridin-2,4-dion-Derivat der Formel (1) oder ein pharmakologisch verträgliches Salz und/oder Ester und/oder Komplex davon zur Inaktivierung von Mikroorganismen in einer Flüssigkeit oder flüssigen, vorzugsweise wässrigen, Zubereitung, beispielsweise Dispersionsfarbe, verwendet.

Vorzugsweise handelt es sich bei der Flüssigkeit um Wasser.

Dabei kann wenigstens ein erfindungsgemäßes 10H-Benzo[g]pteridin-2,4-dion-Derivat der Formel (1) oder ein pharmakologisch verträgliches Salz und/oder Ester und/oder Komplex davon zur Aufbereitung von Wasser für die Getränke- und Lebensmittelindustrie, die Pharma-, Chemie- und Kosmetikindustrie, die Elektroindustrie verwendet werden. Ferner kann wenigstens ein erfindungsgemäßes 10H-Benzo[g]pteridin-2,4-dion-Derivat der Formel (1) oder ein pharmakologisch verträgliches Salz und/oder Ester und/oder Komplex davon bei der Trinkwasser- und Regenwasseraufbereitung, der Behandlung von Abwasser oder bei der Aufbereitung von Wasser für den Einsatz in der Klimatechnik eingesetzt werden.

Bei dieser bevorzugten Verwendung wenigstens eines erfindungsgemäßen 10H-Benzo[g]pteridin-2,4-dion-Derivates der Formel (1) oder ein pharmakologisch verträgliches Salz und/oder Ester und/oder Komplex davon kann die Flüssigkeit oder flüssige Zubereitung nachfolgend mit einer geeigneten Strahlenquelle, die elektromagnetische Strahlung geeigneter Wellenlänge und Energiedichte erzeugt, bestrahlt werden. Vorzugsweise bewirkt das erfindungsgemäße 10H-Benzo[g]pteridin-2,4-dion-Derivat der Formel (1) oder ein pharmakologisch verträgliches Salz und/oder Ester und/oder Komplex davon während der Bestrahlung eine "Selbst-desinfektion" der Flüssigkeit oder der flüssigen Zubereitung.

Bei einer weiteren bevorzugten Verwendung des wenigstens einen erfindungsgemäßen 10H-Benzo[g]pteridin-2,4-dion-Derivates der Formel (1) oder ein pharmakologisch verträgliches Salz und/oder Ester und/oder Komplex davon kann das 10H-Benzo[g]pteridin-2,4-dion-Derivat der Formel (1) oder ein pharmakologisch verträgliches Salz und/oder Ester und/oder Komplex davon an einen festen Träger gebunden vorliegen und so als Teil einer festen Matrix verwendet werden. Besonders bevorzugt wird wenigstens ein an einen festen Träger gebundenes erfindungsgemäßes 10H-Benzo[g]pteridin-2,4-dion-Derivat der Formel (1) oder ein pharmakologisch verträgliches Salz und/oder Ester und/oder Komplex davon in die zu behandelnde Flüssigkeit, vorzugsweise Wasser oder Blut, eingebracht.

Besonders bevorzugt ist als Träger ein Polymer, welches wenigstens ein erfindungsgemäßes 10H-Benzo[g]pteridin-2,4-dion-Derivat der Formel (1) oder ein pharmakologisch verträgliches Salz und/oder Ester und/oder Komplex davon daran in kovalent gebundener Weise trägt. Diese Zusammensetzung, umfassend den Träger und wenigstens ein erfindungsgemäßes 10H-Benzo[g]pteridin-2,4-dion-Derivat der Formel (1) oder ein pharmakologisch verträgliches Salz und/oder Ester und/oder Komplex davon, entwickelt eine antimikrobielle Aktivität sobald sie elektromagnetischer Strahlung geeigneter Wellenlänge und Energiedichte ausgesetzt wird.

Des Weiteren betrifft die vorliegende Erfindung einen beschichteten Gegenstand, der wenigstens ein erfindungsgemäßes 10H-Benzo[g]pteridin-2,4-dion-Derivat der Formel (1) oder ein pharmakologisch verträgliches Salz und/oder Ester und/oder Komplex davon enthält und/oder damit beschichtet ist.

Vorzugsweise weist die Oberfläche des beschichteten Gegenstands wenigstens ein erfindungsgemäßes 10H-Benzo[g]pteridin-2,4-dion-Derivat der Formel (1) oder ein pharmakologisch verträgliches Salz und/oder Ester und/oder Komplex davon auf.

Der beschichtete Gegenstand kann nachfolgend mit einer geeigneten Strahlenquelle, die elektromagnetische Strahlung geeigneter Wellenlänge und Energiedichte erzeugt, bestrahlt werden. Vorzugsweise bewirkt das erfindungsgemäße 10H-Benzo[g]pteridin-2,4-dion-Derivat der Formel (1) oder ein pharmakologisch verträgliches Salz und/oder Ester und/oder Komplex davon während der Bestrahlung eine "Selbst-desinfektion" der Oberfläche des beschichteten Gegenstandes.

Die Bestrahlung kann dabei direkt nach der Behandlung des beschichteten Gegenstandes mit wenigstens einem erfindungsgemäßen 10H-Benzo[g]pteridin-2,4-dion-Derivat der Formel (1) oder ein pharmakologisch verträgliches Salz und/oder Ester und/oder Komplex davon, vorzugsweise nach dem Aufbringen des wenigstens einen erfindungsgemäßen 10H-Benzo[g]pteridin-2,4-dion-Derivates der Formel (1) oder ein pharmakologisch verträgliches Salz und/oder Ester und/oder Komplex davon auf die Oberfläche des beschichteten Gegenstandes und/oder Einbringen des wenigstens einen erfindungsgemäßen 1 0H-Benzo[g]pteridin-2,4-dion-Derivates der Formel (1) oder ein pharmakologisch verträgliches Salz und/oder Ester und/oder Komplex davon in die Oberfläche des beschichteten Gegenstandes, erfolgen und/oder zu einem späteren Zeitpunkt, vorzugsweise vor oder während der Verwendung des beschichteten Gegenstandes.

Geeignete Gegenstände werden vorzugsweise aus der Gruppe, die aus Medizinprodukten, Lebensmittelverpackungen oder Hygieneartikeln, besteht, ausgewählt.

Bei einer weiteren bevorzugten Ausführungsform des beschichteten Gegenstandes handelt es sich um mit wenigstens einem erfindungsgemäßen 10H-Benzo[g]pteridin-2,4-dion-Derivat der Formel (1) oder ein pharmakologisch verträgliches Salz und/oder Ester und/oder Komplex davon beschichtete Partikel, beispielsweise anorganische oder organische Partikel.

Weiter bevorzugt umfassen die Partikel wenigstens ein erfindungsgemäßes 10H-Benzo[g]pteridin-2,4-dion-Derivat der Formel (1) oder ein pharmakologisch verträgliches Salz und/oder Ester und/oder Komplex davon, das an die Partikel kovalent gebunden vorliegt.

Die Erfindung wird nachfolgend durch Figuren und Beispiele erläutert, ohne hierauf beschränkt zu sein.

### Beispiel 1) Herstellung verschiedener 10H-Benzo[g]pteridin-2,4-dion-Derivate.

### Überblick über die Synthesen

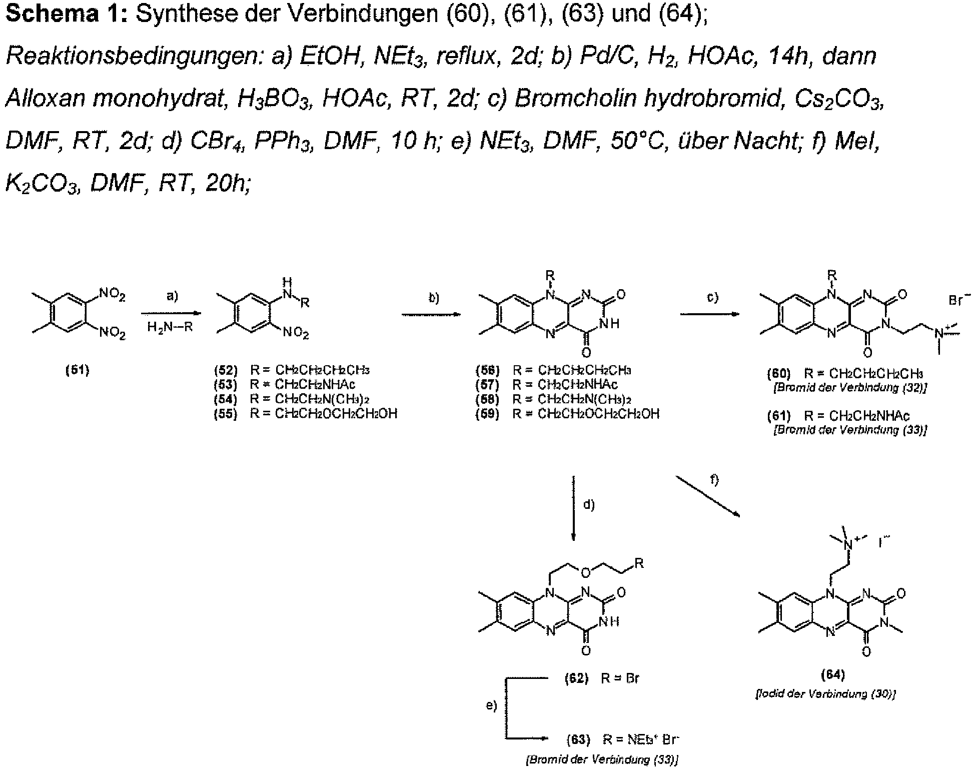

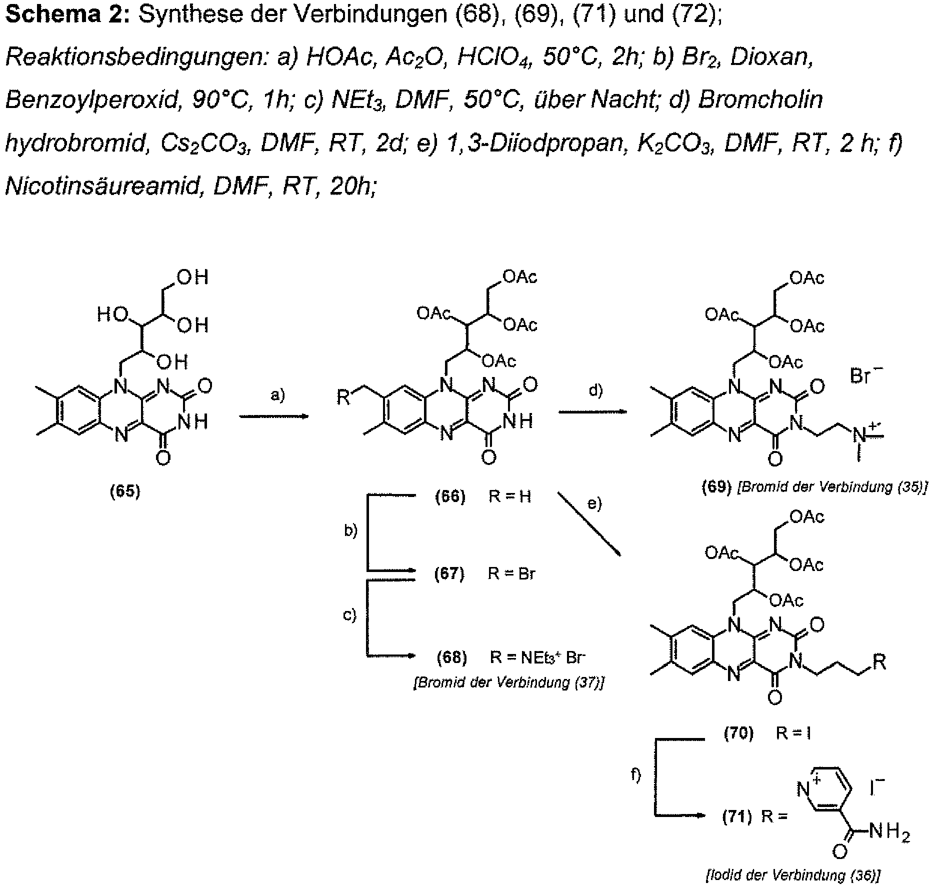

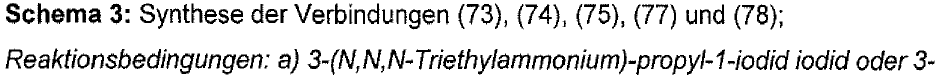

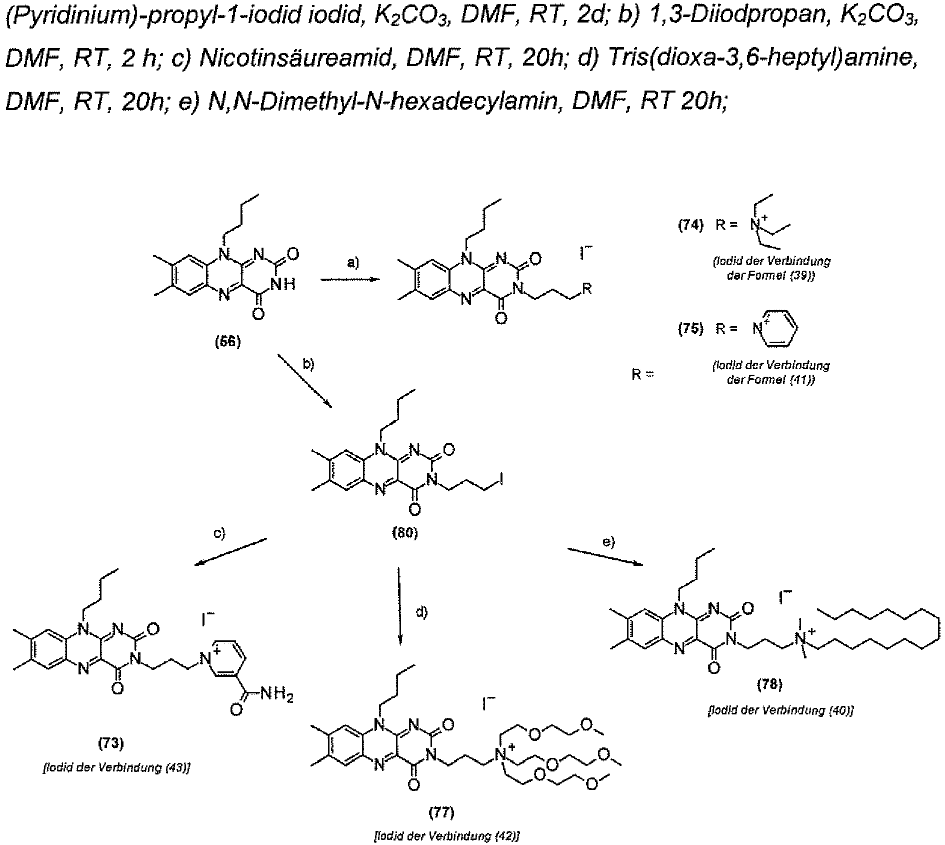

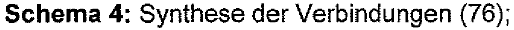

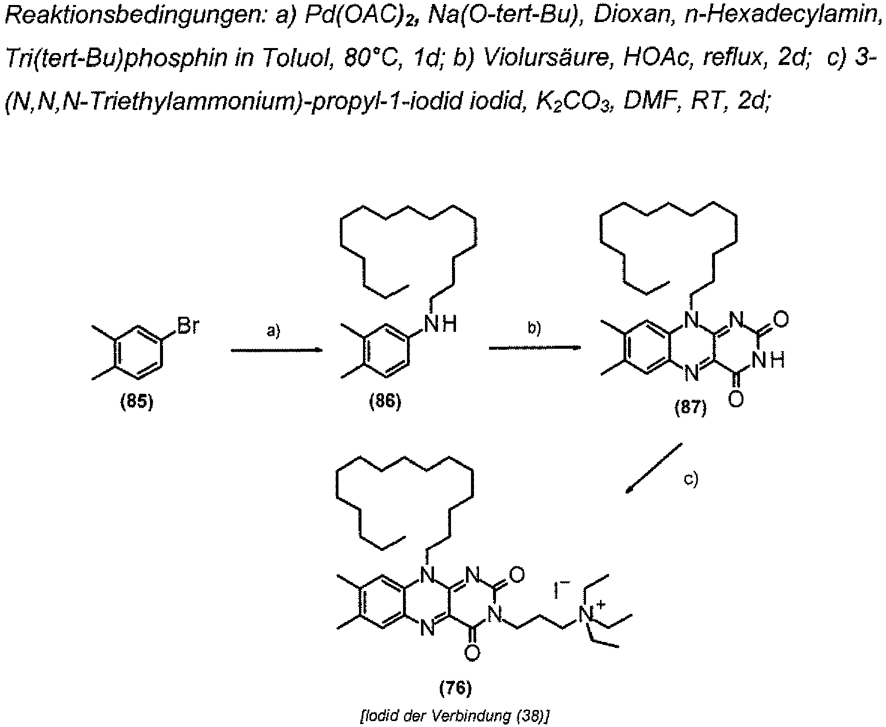

Alle verwendeten Chemikalien wurden von gängigen Anbietern käuflich erworben (TCI, ABCR, Acros, Merck und Fluka) und ohne weitere Reinigung eingesetzt. Lösemittel wurden vor Gebrauch destilliert und bei Bedarf auf übliche Art und Weise getrocknet. Trockenes DMF wurde bei Fluka (Taufkirchen, DE) käuflich erworben.

Dünnschichtchromatographien wurden auf Dünnschicht-Aluminiumfolien, beschichtet mit Kieselgel 60 F254, der Firma Merck (Darmstadt, DE) durchgeführt. Präparative Dünnschichtchromatographien wurden auf kommerziell erhältlichen, mit Kieselgel 60 beschichteten Glasplatten (20cm x 20 cm, Carl Roth GmbH & Co. KG, Karlsruhe, DE)) durchgeführt. Die Verbindungen wurden durch UV-Licht (λ = 254 nm, 333 nm) und teilweise mit bloßem Auge detektiert oder mit Ninhydrin angefärbt. Chromatographien wurden mit Kieselgel (0.060 - 0.200) der Firma Acros (Waltham, US) durchgeführt.

NMR-Spektren wurden auf einem Bruker-Spektrometer Avance 300 (300 MHz [¹H-NMR], 75 MHz [¹³C-NMR]) (Bruker Corporation, Billerica, US) gemessen.

Alle chemischen Verschiebungen sind in δ [ppm] relativ zum externen Standard (Tetramethylsilan, TMS) angegeben. Die Kopplungskonstanten sind jeweils in Hz angegeben; Charakterisierung der Signale: s = Singulett, d = Dublett, t = Triplett, m = Multiplett, dd = Dublett vom Dublett, br = breit. Die Integration bestimmt die relative Anzahl an Atomen. Die eindeutige Bestimmung der Signale in den Kohlenstoffspektren erfolgte mittels der DEPT-Methode (Pulswinkel: 135°). Fehlergrenzen: 0.01 ppm für ¹H-NMR, 0.1 ppm für ¹³C-NMR und 0.1 Hz für Kopplungskonstanten. Das verwendete Lösemittel ist jeweils für jedes Spektrum aufgeführt.

Die IR-Spektren wurden an einem Biorad Spektrometer Excalibur FTS 3000 (Bio-Rad Laboratories GmbH, München, DE) aufgenommen.

ES-MS wurden mit einem ThermoQuest Finnigan TSQ 7000 Spektrometer gemessen, sämtliche HR-MS auf einem ThermoQuest Finnigan MAT 95 (jeweils Thermo Fisher Scientific Inc, Waltham, US) Spektrometer ermittelt, Argon diente als lonisierungsgas für FAB.

Schmelzpunkte wurden mit Hilfe des Schmelzpunktmessgerätes Büchi SMP-20 (Büchi Labortechnik GmbH, Essen, DE) unter Verwendung einer Glaskapillare bestimmt.

Sämtliche UV/Vis-Spektren wurden mit einem Varian Cary 50 Bio UV/VIS Spektrometer, Fluoreszenzspektren mit einem Varian Cary Eclipse Spektrometer aufgenommen.

Die Lösungsmittel für Absorbtions- und Emissionsmessungen wurden in spezieller spektroskopischer Reinheit von Acros oder Baker bzw. Uvasol von Merck gekauft. Milliporewasser (18 MΩ, Milli Q_{Plus}) wurde für alle Messungen verwendet.

1-N-Acetyl-aminoethylamin^{[i]}, 1,2-Dinitro-4,5-dimethyl-benzen (**51**)^{[ii]}, Butyl-(4,5-dimethyl-2-nitro-phenyl)-amin (52) und 10-Butyt-7,8-dimethyl-10H-benzo[g]pteridin-2,4-dion (**56**)^{[iii]}, N-[2-({[(tert-Butyl)oxy]carbonyl}amino)ethyl]-4,5-dimethyl-2-nitroanilin^{[iv]}, N-(3'-Oxabut-1'-yl)-4,5-dimethyl-2-nitroanilin und 10-(2'-Methoxyethyl)-7,8-dimethyl-10H-benzo[g]pteridine-2,4-dion (**65**)^{[v]}, Essigsäure-2,3,4-triacetoxy-1-[3-(3-iodo-propyl)-7,8-dimethyl-2,4-dioxo-3,4-dihydro-2H-benzo[g]pteridin-10-ylmethyl]-butyl ester (**70**)^{[vi]}, Riboflavin tetraacetat (**66**)^{[vii]} und 8α-Bromo-tetraacetylriboflavin (**17**)^{[viii]}, wurden nach literaturbekannten Vorschriften hergestellt:
[i] M.Jasinski, G. Mloston, P. Mucha, A.Linden, H.Heimgartner, Helv. Chim. Acta 2007, 90, 1765 -1779
[ii] a) A. Monge, J. A. Palop, A. López de Ceráin, V. Senador, F. J. Martinez-Crespo, Y. Sainz, S. Narro, E. Garcia, C. de Miguel, M. González, E. Hamilton, A. J. Barker, E. D. Clarke, D. T. Greenhow, J. Med. Chem. 1995, 38, 1786-1792; b) T. Sugaya, K. Nobuyuki, A. Sakaguchi, S. Tomioka, Synthesis 1995, 1257-1262; c) R. R. Holmes, R. P. Bayer, J. Am. Chem. Soc. 1960, 82, 3454-3456.
[iii] O.Wiest, Ch.B. Harrison, N.J. Saettel, R. Cibulka, M. Sax, B. König, J. Org. Chem. 2004, 69, 8183-8185
[iv] J. Butenandt, R. Epple, E.-U. Wallenborn, A.P.M. Eker, V. Gramlich, T. Carell, Chem. Eur. J. 2000, 6, No. 1, 62-72
[v] R. Epple, E.-U. Wallenborn, T. Carell, J. Am. Chem. Soc. 1997, 119, 7440-7451.
[vi] A. Barthel, L. Trieschmann, D. Ströhl, R. Kluge, G. Böhm, Rene Csuk, Arch. Pharm. Chem. Life Sci. 2009, 342, 445-452.
[vii] McCormick, D. B. J. Heterocycl. Chem. 1970, 7, 447-450.
[viii] M.C. Falk, P.G. Johnson, D.B. McCormick, Biochemistry, 1976, 15(3), 639-645

Bromocholin hydrobromid ist kommerziell mit einem Reinheitsgrad >98% erhältlich (TCI Deutschland GmbH, Eschborn, Deutschland) und wurde ohne weitere Reinigung verwendet.

### Allgemeine Vorschrift I): Umsetzung von 1,2-Dinitro-4.5-dimethyl-benzen zu substituierten 4,5-Dimethyl-2-Nitroanilinen

1,2-Dinitro-4,5-dimethyl-benzen (**51**) (3,92g, 20 mmol) und das entsprechende primäre Amin (100 mmol) weurden in trockenem Ethanol (100 mL) und frisch destilliertem Triethylamin (50 mL) 2 Tage bei 90°C Ölbadtemperatur unter Stickstoff refluxiert. Nach Abkühlen auf Raumtemperatur wurde das Lösungsmittelgemisch bei vermindertem Druck abgezogen und der Rückstand getrocknet.

**Tabelle 1: verwendete Amine**

| Beispiel | Zielverbindung | primäres Amin |
|---|---|---|
| | (52) | H₂NCH₂CH₂CH₂CH₃ |
| Ia) | (53) | H₂NCH₂CH₂NHAc |
| Ib) | (54) | H₂NCH₂CH₂N(CH₃)₂ |
| Ic) | (55) | H₂NCH₂CH₂OCH₂CH₂OH |

### Ia) N'-(4,5-Dimethyl-2-nitro-phenyl)-N,N-acetyl-ethane-1,2-diamine (53):

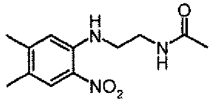

Das Rohprodukt wurde aus Ethanol umkristallisiert und es wurden 3,54 g rotorange Kristallnadeln erhalten (71 % der Theorie, 141 mmol).
**¹H-NMR** (300 MHz, CDCl₃): δ [ppm] = 2,03 (s, 3 H), 2,15 (s, 3 H), 2,25 (s, 3 H), 3.18 (t, 2 H, J = 6.0 Hz), 3.39 (t, 2 H, J = 5.8 Hz), 6.94 (s, 1 H), 7,02 (m, 1 H), 7.84 (s, 1 H), 8.08 (bs, 1 H); - **MS** (ESI-MS, H₂O/MeOH + 0.1 % TFA): m/z (%) = 252.1 (100, (MH⁺)); - **MG** = 251,29 g/mol - **MF** = C₁₂H₁₇N₃O₃

### Ib) N'-(4.5-Dimethyl-2-nitro-phenyl)-N,N-dimethyl-ethane-1.2-diamine (54):

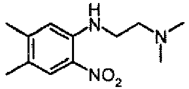

Das Rohprodukt wurde aus Ethanol umkristallisiert. Man erhält 2,99 g orange Kristallnadeln (63 % der Theorie, 126 mmol).
**¹H-NMR** (300 MHz, CDCl₃): δ [ppm] = 2,17 (s, 3 H), 2,26 (s, 3 H), 2,32 (s, 6 H), 2,71 (t, 2 H, J = 544 Hz), 3,32 (m, 2 H), 6,61 (s, 1 H), 7,91 (s, 1 H), 8,19 (bs, 1 H); - **MS** (CI-MS, NH₃): m/z (%) = 238.1 (100, (MH⁺)); - **MG** = 237,30 g/mol - **MF** = C₁₂H₁₉N₃O₂

### Ic) 2-[2-(4,5-Dimethyl-2-nitro-phenylamino)-ethoxy]-ethanol (55):

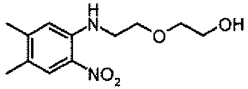

Das Rohprodukt wurde durch Säulenchromatographie an Kieselgel mit Ethylacetat / Petrolether 1:1 gereinigt unter Erhalt eines roten Öls, welches nach längerem Stehen zu einem orangen Feststoff erstarrt (4,31 g, 86 % der Theorie, 16,9 mmol).
**¹H-NMR** (300 MHz, CDCl₃): δ [ppm] = 2,02 (s, 3 H), 2,11 (s, 3 H), 2,98 (bs, 1 H), 3,37 (t, 2 H, J = 5,4 Hz), 3,54 (t, 2 H, J = 5,4 Hz), 3,69 (m, 4 H), 6,49 (s, 1 H), 7,70 (s, 1 H), 8,01 (m, 1 H); - **MS** (ESI-MS, H₂O/MeOH + 0.1 % TFA): m/z (%) = 255.1 (58, (MH⁺)), 509.3 (100, (2MH⁺)); - **MG** = 254,29 g/mol - **MF** = C₁₂H₁₈N₂O₄

### Allgemeine Vorschrift II): Umsetzung von substituierten 2-Nitroanilinen zu den entsprechenden monosubstituierten 10H-benzo[g]pteridin-2,4-dion-Derivaten

Das jeweils oben unter Ia) bis Ic) erhaltene 2-Nitroanilin (**53**) bis (**55**) (10 mmol) wurde in Essigsäure (100 mL) gelöst. Palladium auf Aktivkohle (100 mg, 10% Pd) wurde zugegeben und der Ansatz wurde 14 h bei Raumtemperatur im Autoklaven in Wasserstoffatmosphäre bei 20 bar gerührt. Die farblose Lösung wurde in einen Schlenckkolben mit Stickstoffatmosphäre gefiltert, Alloxan monohydrat (4,00 g, 25 mmol) und Borsäure (15,50 g, 250 mmol) wurden zugegeben. Der Kolben wurde mit Alufolie umwickelt und der Ansatz 2 Tage bei Raumtemperatur unter Stickstoff im Dunklen gerührt. Die orangegelbe Suspension wurde mit Wasser (200 mL) verdünnt und viermal mit Dichlormethan (je 150 mL) extrahiert. Die vereinigten organischen Phasen wurden mit Wasser (100 mL) gewaschen, über Magnesiumsulfat getrocknet und einrotiert.

### IIa) 10-(2-N-Acetyl-amino-ethyl)-7,8-dimethyl-10H-benzo[g]pteridine-2,4-dion (57)

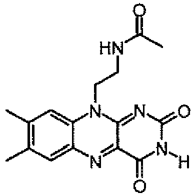

Der Rückstand wurde aus Essigsäure und Wasser (1:1) umkristallisiert. Man erhält das Produkt als orangen Feststoff (2,56 g, 7,82 mmol, 78 % der Theorie).
**¹H-NMR** (300 MHz, DMSO-d6): δ [ppm] = 2,03 (s, 3 H), 2,41 (s, 3 H), 2.54 (s, 3 H), 3,32 (t, 2 H, J = 5,4 Hz), 3,46 (t, 2 H, J = 5,4 Hz), 7.79 - 7,83 (s, 1 H), 11.32 (s, 1 H); - **MS** (ESI-MS, H₂O/MeOH + 0.1 % TFA): m/z (%) = 328.0 (100, (MH⁺)); - **MG** = 327,35 g/mol - **MF** = C₁₆H₁₇N₅O₃

### IIb) 10-(2-Dimethylamino-ethyl)-7,8-dimethyl-10H-benzo[g]lpteridine-2,4-dion (58)

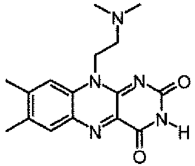

Der Rückstand wurde durch Säulenchromatographie an Kieselgel mit Ethylacetat / Methanol 20:1 → 5:2 gereinigt unter Erhalt eines orangen Feststoffs (2,22 g, 7,07 mmol, 71 % der Theorie).
**¹H-NMR** (300 MHz, DMSO-d6): δ [ppm] = 2.34 (s, 3 H), 2.43 (s, 3 H), 2,91 (s, 6 H), 3,39 (m, 2 H), 4,61 (m, 2 H), 7.52 (s, 1 H), 7.78 (s, 1 H), 11.38 (s, 1 H); - **MS** (ESI-MS, H₂O/MeOH + 0.1 % TFA): m/z (%) = 314.0 (100, (MH⁺)); - **MG** = 313,36 g/mol - **MF** = C₁₆H₁₉N₅O₂

### IIc) 10-[2-(2-Hydroxy-ethoxy)-ethyl]-7,8-dimethyl-10H-benzo[g]pteridine-2,4-dion (59)

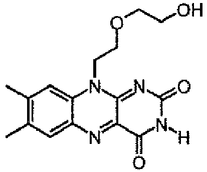

Der Rückstand wurde durch Säulenchromatographie an Kieselgel mit Ethylacetat / Methanol 20:1 → 5:2 gereinigt. Man erhält 1,98 g orangen Feststoff (6,0 mmol, 60 % der Theorie).
**¹H-NMR** (300 MHz, DMSO-d6): δ [ppm] = 2,42 (s, 3 H), 2,53 (s, 3 H), 3,28 (t, 2 H, J = 5,4 Hz), 3,49 (t, 2 H, J = 5,4 Hz), 3,66 (m, 4 H), 7.76 (s, 1 H), 7.88 (s, 1 H), 11.33 (s, 1 H); - **MS** (ESI-MS, H₂O/MeOH + 0.1 % TFA): m/z (%) = 331.1 (100, (MH⁺)), 661.4 (61, (2MH⁺)); - **MG** = 330,35 g/mol - **MF** = C₁₆H₁₈N₄O₄

### III) 10-[2-(2-Bromo-ethoxy)-ethyl]-7,8-dimethyl-10H-benzo[g]pteridine-2,4-dion (62)

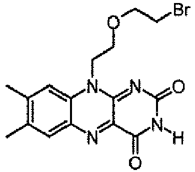

Das unter IIc) erhaltene 10-[2-(2-Hydroxy-ethoxy)-ethyl]-7,8-dimethyl-10H-benzo[g]pteridine-2,4-dion (**59**) (80 mg, 0.25 mmol) wurde zusammen mit Tetrabrommethan (100 mg, 0.3 mmol) in trockenem DMF (5 mL) gelöst. Triphenylphosphin (168 mg, 0.5 mmol) wurde bei 0°C in mehreren kleinen Portionen zugegeben. Der Ansatz wurde über Nacht bei Raumtemperatur gerührt und anschließend das Lösungsmittel bei vermindertem Druck abgezogen. Der Rückstand wurde durch präparative Dünnschichtchromatographie mit Essigsäureethylester / Methanol 25:1 gereinigt. Es wurden 72 mg eines gelben Feststoffs erhalten (0.186 mmol, 73% der Theorie).
**¹H-NMR** (300 MHz, DMSO-d6): δ [ppm] = 2,40 (s, 3 H), 2,51 (s, 3 H), 3,23 (t, 2 H, J = 5,4 Hz), 3,56 (t, 2 H, J = 5,4 Hz), 3,61 (m, 2 H), 3,87 (m, 2 H), 7.78 (s, 1 H), 7.93 (s, 1 H), 11.30 (s, 1 H); - **MS** (ESI-MS, H₂O/MeOH + 0.1 % TFA): m/z (%) = 393.1 & 395.1 (100, (MH⁺)); - **MF:** C₁₆H₁₇BrN₄O₃ - **FW:** 393.24 g/mol;

### IV) Trimethyl-[2-(3,7,8-trimethyl-2,4-dioxo-3,4-dihydro-2H-benzo[g]pteridin-10-yl)-ethyl]-ammonium iodid (64)

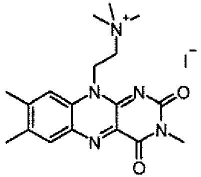

Das unter IIb) erhaltene Flavin (**58**) (331 mg, 1.0 mmol) wurde in trockenem DMF (20 mL) gelöst, Caesium carbonat (488 mg, 1.5 mmol) und Methyliodid (1.42 g, 10.0 mmol) wurden zugegeben und der Ansatz für 20 h bei Raumtemperatur im Dunklen gerührt. Die Suspension wurde mit Chloroform (100 mL) verdünnt und mit Wasser (30 mL) gewaschen. Die organische Phase wurde über Magnesiumsulfat getrocknet und die Lösungsmittel wurden bei vermindertem Druck abgezogen. Das Rohprodukt wurde durch präparative Dünnschichtchromatographie an Kieselgel (CHCl₃/MeOH - 4:1) gereinigt.

Ausbeute: 263 mg eines orangen Feststoffes (0,56 mmol, 56 % der Theorie) R_{f} = 0.1 (CHCl₃:MeOH -4:1)
**¹H-NMR** (300 MHz, DMSO-d6): δ [ppm] = 2.36 (s, 3 H), 2.42 (s, 3 H), 3,17 (bs, 9 H), 3,41 (m, 2 H), 3,48 (s, 3 H), 4,36 (m, 2 H), 7.56 (s, 1 H), 7.79 (s, 1 H); - **MS** (ESI-MS, H₂O/MeOH + 0.1 % TFA): m/z (%) = 342.1 (100, (M⁺)); - **MG** = 342.42 + 126.90 g/mol - **MF** = C₁₈H₂₄N₅O₂I

### V) {2-[2-(7,8-Dimethyl-2,4-dioxo-3,4-dihydro-2H-benzo[g]pteridin-10-yl)ethoxy]-ethyl}-triethyl-ammonium bromid (63)

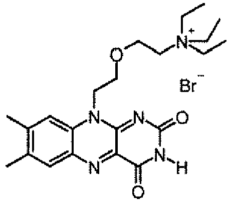

Das unter III) erhaltene Flavin (**62**) (393 mg, 1.0 mmol) wurde in trockenem DMF (20 mL) gelöst, Triethylamin (1.01 g, 10.0 mmol) wurde zugegeben und der Ansatz über Nacht bei 50°C im Dunklen gerührt. Die Suspension wurde mit Chloroform (100 mL) verdünnt und mit Wasser (30 mL) gewaschen. Die organische Phase wurde über Magnesiumsulfat getrocknet und die Lösungsmittel wurden bei vermindertem Druck abgezogen. Das Rohprodukt wurde durch präparative Dünnschichtchromatographie an Kieselgel (CHCl₃/MeOH -4:1) gereinigt.

Ausbeute: 141 mg mikrokristalliner oranger Feststoff (0,284 mmol, 28 % der Theorie) R_{f} = 0.1 (CHCl₃:MeOH - 4: 1)
**¹H-NMR** (300 MHz, DMSO-d6): δ [ppm] = 1,26 (t, 9 H, J = 7,4 Hz), 2,42 (s, 3 H), 2,53 (s, 3 H), 3,22 - 3,37 (m, 8 H), 3,46 (t, 2 H, J = 5,4 Hz), 3,68 (m, 4 H), 7.78 (s, 1 H), 7.91 (s, 1 H), 11.31 (s, 1 H); - **MS** (ESI-MS, H₂O/MeOH + 0.1 % TFA): m/z (%) = 414.1 (100, (M⁺)); - **MG** = 414.53 + 79.90 g/mol - **MF** = C₂₂H₃₂N₅O₃Br

### Allgemeine Vorschrift VI): Funktionalisierung von Flavinen in Position 3 durch Bromocholin hydrobromid

Das jeweils angegebene Flavin (1,0 mmol) wurde in trockenem DMF (20 mL) gelöst, Caesium carbonat (1,63 g, 5 mmol) und Bromocholin hydrobromid (0.5 g, 2.0 mmol) wurden zugegeben und der Ansatz für 1d bei Raumtemperatur im Dunklen gerührt. Es wurde erneut Bromocholin hydrobromid (0.5 g, 2.0 mmol) zugegeben und der Ansatz einen weiteren Tag bei Raumtemperatur im Dunklen gerührt. Das DMF wurde abgezogen und der Rückstand in Chloroform/Methanol 6:1 (50 mL) suspendiert. Die Suspension wurde gefiltert und das Filtrat wurde bis zur Trockene eingeengt. Das Rohprodukt wurde durch präparative Dünnschichtchromatographie an Kieselgel (CHCl₃/MeOH - 4:1) und durch Umkristallisation aus Wasser gereinigt.

### VIa) [2-(10-Butyl-7,8-dimethyl-2,4-dioxo-4,10-dihydro-2H-benzo[g]pteridin-3-yl)-ethyl]-trimethyl-ammonium bromid (60)

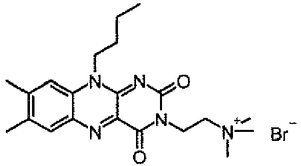

Aus 10-Butyl-7,8-dimethyl-10H-benzo[g]pteridin-2,4-dion (**56**) (310 mg, 1.0 mmol) durch Umsetzung gemäß der allgemeinen Vorschrift VI) als oranger Feststoff in einer Ausbeute von 161 mg (0,347 mmol, 35 % der Theorie) erhalten.

R_{f} = 0.1 (CHCl₃:MeOH - 4:1)
**¹H-NMR** (300 MHz, DMSO-d6): δ [ppm] = 0,97 (t, 3 H, J = 7.3 Hz), 1,48 (m, 2 H), 1,70 (m, 2 H), 2,41 (s, 3 H), 2,51 (s, 3 H), 3,14 - 3,35 (m, 8 H), 3,95 (t, 2 H, J = 6 Hz), 4,56 (t, 2 H, J = 7,8 Hz), 7,77 (s, 1 H), 7,89 (s, 1 H); - **MS** (ESI-MS, H₂O/MeOH + 0.1 % TFA): m/z (%) = 384.1 (100, (M⁺)); - **MG** = 384.51 + 79.90 g/mol - **MF** = C₂₁H₃₀N₅O₂Br

### VIb) {2-[10-(2-Acetylamino-ethyl)-7,8-dimethyl-2,4-dioxo-4,10-dihydro-2H-benzo[g]pteridin-3:yl]-ethyl}-trimethyl-ammonium bromid (61)

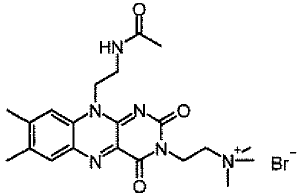

Das unter IIa) erhaltene Flavin (57) (330 mg, 1.0 mmol) ergab bei Umsetzung nach der allgemeinen Vorschrift IV) 178 mg eines orangen Feststoffes (0,361 mmol, 36 % der Theorie)

R_{f} = 0.1 (CHCl₃:MeOH -4:1)
**¹H-NMR** (300 MHz, DMSO-d6): δ [ppm] = 2,03 (s, 3 H), 2,41 (s, 3 H), 2,51 (s, 3 H), 3,16 - 3,32 (m, 8 H), 3.46 (t, 2 H, J = 5.6 Hz), 4,66 (t, 2 H, J = 5.6 Hz), 3,96 (t, 2 H, J = 6 Hz), 6,81 (m, 1 H), 7,75 (s, 1 H), 7,88 (s, 1 H); - **MS** (ESI-MS, H₂O/MeOH + 0.1 % TFA): m/z (%) = 413.1 (100, (M⁺)); - **MG** = 413.50 + 79.90 g/mol - **MF** = C₂₁H₂₉N₆O₃Br

### VII) {2-[7.8-Dimethyl-2,4-dioxo-10-(2,3,4,5-tetraacetoxy-pentyl)-4.10-dihydro-2H-benzo[g]pteridin-3-yl]-ethyl}-trimethyl-ammonium bromid (69)

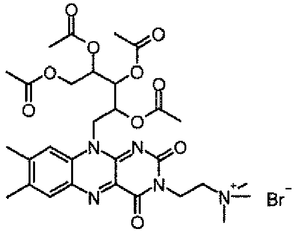

Riboflavin tetraacetat (**66**) (540 mg, 1.0 mmol) ergab bei Umsetzung nach der allgemeinen Vorschrift IV) 191 mg eines hellbraunen Feststoffes (0,268 mmol, 27 % der Theorie)

R_{f} = 0.1 (CHCl₃:MeOH -4:1)
**¹H-NMR** (300 MHz, MeOD): δ [ppm] = 1,71 (s, 3 H), 2,02 (s, 3 H), 2,21 (s, 3 H), 2,29 (s, 3 H,), 2,42 (s, 3 H), 2,52 (s, 3 H), 3,12 - 3,27 (m, 8 H), 3,94 (t, 2 H, J = 5,8 Hz), 4,20 - 4,28 (m, 1 H), 4,52 (m, 1 H), 5,18 (m, 2 H), 5,44 (m, 1 H), 5,54 (m, 1 H), 5,68 (m, 1 H), 7,89 (s, 1 H), 7,93 (s, 1 H); - **MS** (ESI-MS, H₂O/MeOH + 0.1 % TFA): m/z (%) = 630.1 (100, (M⁺)); - **MG** = 630.68 + 79.90 g/mol - **MF** = C₃₀H₄₀N₅O₁₀Br

### VIII) Triethyl-[7-methyl-2,4-dioxo-10-(2,3,4,5-tetraacetoxy-pentyl)-2,3,4,10-tetrahydro-benzo[g]pteridin-8-ylmethyl]-ammonium bromid (68)

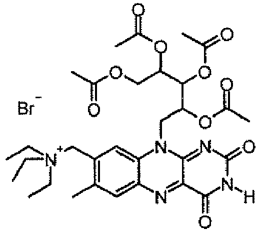

8α-Bromo-tetraacetylriboflavin (**67**) (0,78 g, 1,25 mmol) wurde in DMF (10 mL) gelöst, die Lösung wurde entgast und Triethylamin (0,60 g, 0,76 mL, 6.0 mmol) wurde über 5 min. zugetropft. Der Ansatz wurde bei 50°C über Nacht unter N₂ im Dunklen gerührt. Nach Abkühlen auf Raumtemperatur wurde die Reaktionsmischung in 200 mL eiskalten Diethylether eingetropft, das Produkt wurde abgefiltert, mit Diethylether gewaschen und bei vermindertem Druck getrocknet. Der Rückstand wurde in Chloroform gelöst, mit Diethylether gefällt, abzentrifugiert und getrocknet. Zur weiteren Reinigung wurde aus Wasser umkristallisiert. Hellbrauner Feststoff (0.31 g, 0.43 mmol, 34 % der Theorie).
**¹H-NMR** (300 MHz, MeOD): δ [ppm] = 1,25 (t, 9 H, J = 7,3 Hz), 1,71 (s, 3 H), 2,02 (s, 3 H), 2,21 (s, 3 H), 2,29 (s, 3 H,), 2,42 (s, 3 H), 2,59 (m, 2 H), 3,15 - 3,30 (m, 6 H), 4,20 - 4,28 (m, 1 H), 4,52 (m, 1 H), 5,18 (m, 2 H), 5,44 (m, 1 H), 5,54 (m, 1 H), 5,68 (m, 1 H), 7,89 (s, 1 H), 7,93 (s, 1 H), 11,32 (s, 1 H); - **MS** (ESI-MS, H₂O/MeOH + 0.1 % TFA): m/z (%) = 644.1 (100, (M⁺)); - **MG** = 644.71 + 79.90 - **MF** = C₃₁H₄₂N₅O₁₀Br

### IX) 2-Carbamoyl-1-{3-[7,8-dimethyl-2,4-dioxo-10-(2,3,4,5-tetraacetoxy-pentyl)-4.10-dihvdro-2H-benzo[g]pteridin-3-yl]-propyl}-pyridinium iodid (72)

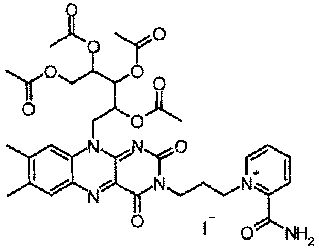

Essigsäure-2,3,4-triacetoxy-1-[3-(3-iodo-propyl)-7,8-dimethyl-2,4-dioxo-3,4-dihydro-2H-benzo[g]pteridin-10-ylmethyl]-butyl ester (**70**) (0.75 g, 1.05 mmol) wurde mit Nicotinsäureamid (146 mg, 1,2 mmol) und NaHCO₃ (0.1 g, 1.2 mmol) in DMF (10 mL) in Stickstoffatmosphäre im Dunklen bei 50°C für 24 h gerührt. Das Lösungsmittel wurde bei vermindertem Druck abgezogen und der Rückstand durch präparative Dünnschichtchromatographie gereinigt (CHCl₃/MeOH 6:1).
Es wurde 277 mg hellbrauner Feststoff (0,33 mmol, 32 % der Theorie) erhalten.
**¹H-NMR** (300 MHz, MeOD): δ [ppm] = 1,70 (s, 3 H), 2,03 (s, 3 H), 2,20 (s, 3 H), 2,28 (s, 3 H,), 2,42 (s, 3 H), 2,48 (m, 2 H), 2,52 (s, 3 H), 4,18 - 4,31 (m, 3 H), 4,51 (m, 1 H), 4,88 (m, 2 H), 5,17 (m, 2 H), 5,45 (m, 1 H), 5,52 (m, 1 H), 5,67 (m, 1 H), 7,86 (s, 1 H), 7,92 (s, 1 H), 8,70 - 8,78 (m, 2 H), 9,23 (d, J = 5,6 Hz, 1 H), 9,38 (d, J = 5.6 Hz, 1 H); - **MS** (ESI-MS, H₂O/MeOH + 0.1 % TFA): m/z (%) = 707.1 (100, (M⁺)); - **MG** = 707.72 + 126.90 - **MF** = C₃₄H₃₉N₆O₁₁I

### X) 3-(10-Butyl-7,8-dimethyl-2,4-dioxo-4,10-dihydro-2H-benzo[g]pteridin-3-yl)-propyliodid (80)

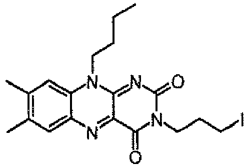

Verbindung (**56**) (0.6 g, 2.0 mmol) und Cs₂CO₃ (1.8 g, 6 mmol) wurden in trockenem DMF (40 mL) vorgelegt. 1,3-Diiodopropan (1.5 g, 5.0 mmol) wurde zugegeben und der Ansatz 2 h im Dunklen unter N₂ bei Raumtemperatur gerührt. Das Lösungsmittel wurde unter vermindertem Druck abgezogen, der Rückstand in Ethylacetat (200 mL) gelöst und mit H₂O (2x 100 mL) gewaschen. Nach Trocknen der organischen Phase über MgSO₄ wurde das Lösungsmittel bei vermindertem Druck abgezogen. Nach Reinigung durch Säulenchromatographie (Kieselgel, CHCl₃ /MeOH, 98:2), wurde die Zielverbindung (0.82 g, 1.76 mmol, 88%) als gelber Feststoff erhalten.
**¹H-NMR** (300 MHz, CDCl₃): δ [ppm] = 0,97 (t, *J* = 7,4 Hz, 3 H), 1,48 (m, 2 H), 1,72 (m, 2 H), 2,23-2,36 (m, 2 H), 2,41 (s, 3 H), 2,50 (s, 3 H), 3,24 (t, *J* = 7,4 Hz, 2 H), 4,15 - 4,26 (m, 2 H), 4,56 (t, *J=* 7,8 Hz, 2 H), 7,77 (s, 1 H), 7,89 (s, 1 H); - **MS** (ESI-MS, H₂O/MeOH + 0.1 % TFA): m/z (%) = 467.1 (100, (MH⁺)); - **MG** = 466.33 g/mol - **MF** = C₁₉H₂₃N₄O₂I

### XI) 2-Carbamoyl-1-{3-[7,8-dimethyl-2,4-dioxo-10-butyl-4,10-dihydro-2H-benzo[g]pteridin-3-yl]-propyl}-pyridinium iodid (73)

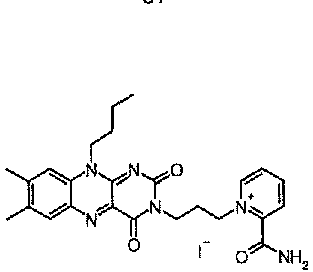

3-(10-Butyl-7,8-dimethyl-2,4-dioxo-4,10-dihydro-2H-benzo[g]pteridin-3-yl)-propyliodid (**80**) (0.51 g, 1.05 mmol) wurde mit Nicotinsäureamid (146 mg, 1,2 mmol) und NaHCO₃ (0.1 g, 1.2 mmol) in DMF (10 mL) in Stickstoffatmosphäre im Dunklen bei 50°C für 24 h gerührt. Das Lösungsmittel wurde bei vermindertem Druck abgezogen und der Rückstand durch präparative Dünnschichtchromatographie gereinigt (CHCl₃/MeOH 6:1).

Es wurde 276 mg oranger Feststoff (0,47 mmol, 46 % der Theorie) erhalten.
**¹H-NMR** (300 MHz, DMSO): δ [ppm] = 0,91 (t, 3 H), 1,43 (m, 2 H), 1,72 (m, 2H), 2,31 (m, 2 H), 2,41 (s, 3 H), 2,58 (s, 3 H,), 4,02 (m, 2 H), 4,63 (m, 2 H), 4,71 (m, 2 H), 5,31 (s, 2 H), 7,86 (s, 1 H), 7,95 (m, 1 H), 8,18 (s, 1 H), 8,51 (s, 1 H), 8,94 (d, J = 5,6 Hz, 1 H), 9,23 (d, J = 5.6 Hz, 1 H), 9.52 (m, 1 H); - **MS** (ESI-MS, H₂O/MeOH + 0.1 % TFA): m/z (%) = 461.1 (100, (M⁺)); - **MG** = 461.55 + 126.90 - **MF** = C₂₅H₂₉N₆O₃I

### XII) 10-Hexadecyl-7,8-dimethyl-10H-benzo[g]pteridin-2,4-dion (87)

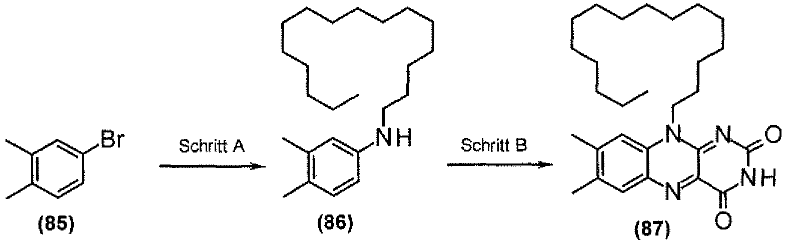

### Schritt A, Synthese des Amins (86)

3,4-Dimethyl-1-brombenzen (85) (0.37 g, 2 mmol) wurde zu einer Suspension von Palladium(II)acetat (22 mg, 0.1 mmol) und Natrium-tert-butylat (224 mg, 2.3 mmol) in trockenem Dioxan (5 mL) unter Argon in einem trockenem Schlenckrohr zugegeben. Nach Zugabe von Hexadecylamin (0.72 g, 3 mmol) und Tri(tert-butyl)phosphin in Toluol (0.3 mL) wurde über Nacht bei 80°C gerührt. Die Reaktionsmischung wurde nach Abkühlen auf Raumtemperatur mit Dichlormethan (30 mL) verdünnt und mit H₂O (2x 10 mL) gewaschen. Nach Trocknen der organischen Phase über MgSO₄ wurde das Lösungsmittel bei vermindertem Druck abgezogen. Nach Reinigung durch Säulenchromatographie (Kieselgel, Ethylacetat/EtOH, 9:1), wurde die Zielverbindung (86) (0.62 g, 1.79 mmol, 90%) als farbloser Feststoff erhalten.

### Schritt B, Synthese des Flavins (87)

Das Amin (86) aus dem vorherigen Schritt (0.62 g, 1.79 mmol) wurde mit Violursäure (320 mg, 2 mmol) in Eisessig (10 mL) in Stickstoffatmosphäre im Dunklen für 24 h refluxiert. Violursäure (320 mg, 2 mmol) wurde zugegeben und der Ansatz weitere 24h bei denselben Bedingungen gehalten. Das Lösungsmittel wurde bei vermindertem Druck abgezogen und der Rückstand durch präparative Dünnschichtchromatographie gereinigt (CH₂Cl₂/EtOH 20:1). Es wurden 254 mg oranger Feststoff (0,54 mmol, 30 % der Theorie) erhalten.
**¹H-NMR** (300 MHz, DMSO-d6): δ [ppm] = 1,02 (t, 3 H), 1,20 - 1,37 (m, 26 H), 1,73 (m, 4H), 2,43 (s, 3 H), 2,56 (s, 3 H,), 7,48 (s, 1 H), 8,04 (s, 1 H), 11,30 (bs, 1 H); - **MS** (ESI-MS, H₂O/MeOH + 0.1 % TFA): m/z (%) = 467.1 (100, (M⁺)); - **MG** = 466.67 - **MF** = C₂₈H₄₂N₄O₂

### Allgemeine Vorschrift XIII): Funktionalisierung von Flavinen in Position 3 durch 3-(N,N,N-Triethylammonium)-propyl-1-iodid iodid oder 3-(Pyridinium)-propyl-1-iodid iodid

**Tabelle 2: verwendete Flavine und**

| Beispiel | Zielverbindung | Flavin |
|---|---|---|
| XIa) | (74) | (56) |
| XIb) | (75) | (56) |
| XIc) | (76) | (87) |

Das jeweils in Tabelle 2 angegebene Flavin (1,0 mmol) wurde in trockenem DMF (20 mL) gelöst, Kaliumcarbonat (0,69 g, 5 mmol) und 3-(N,N,N-Triethylammonium)-propyl-1-iodid iodid oder 3-(Pyridinium)-propyl-1-iodid iodid (0,8 g bzw. 0,76 g, 2.0 mmol) wurden zugegeben und der Ansatz für 1d bei Raumtemperatur im Dunklen gerührt. Es wurde erneut 3-(N,N,N-Thethylammonium)-propyl-1-iodid iodid oder 3-(Pyridinium)-propyl-1-iodid iodid (0,8 g bzw. 0,76 g, 2.0 mmol) zugegeben und der Ansatz einen weiteren Tag bei Raumtemperatur im Dunklen gerührt. Das DMF wurde abgezogen. Das Rohprodukt wurde durch Säulenchromatographie an Kieselgel (CHCl₃/MeOH - 20:1 → 6:1) gereinigt.

### XIIIa) [3-(10-Butyl-7,8-dimethyl-2,4-dioxo-4,10-dihydro-2H-benzo[g]pteridin-3-yl)-propyl]-triethyl-ammonium iodid (74)

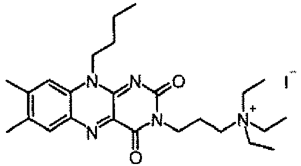

Aus 10-Butyl-7,8-dimethyl-10H-benzo[g]pteridin-2,4-dion (**56**) (310 mg, 1.0 mmol) und 3-(N,N,N-Triethytammonium)-propyl-1-iodid iodid durch Umsetzung gemäß der allgemeinen Vorschrift XII) als oranger Feststoff in einer Ausbeute von 188 mg (0,331 mmol, 33 % der Theorie) erhalten.

R_{f} = 0,05 (CHCl₃:MeOH - 6:1)
**¹H-NMR** (300 MHz, DMSO): δ [ppm] = 0,92 (t, 3 H), 1,18 (m, 9 H), 1,45 (m, 2 H), 1,68 (m, 2H), 1,97 (m, 2 H), 2,39 (s, 3 H), 2,52 (s, 3 H,), 3,21 (m, 8 H), 3,31 (s, 6 H), 3,97 (m, 2 H), 4,01 (m, 2 H), 7,86 (s, 1 H), 7,98 (s, 1 H); - **MS** (ESI-MS, H₂O/MeOH + 0.1 % TFA): m/z (%) = 440.2 (100, (M⁺)); **-MG** = 440.61 + 126.90 g/mol - **MF** = C₂₅H₃₈N₅O₂I

### XIIIb) [3-(10-Butyl-7,8-dimethyl-2,4-dioxo-4,10-dihydro-2H-benzo[g]pteridin-3-yl)-propyl]-pyridinium iodid (75)

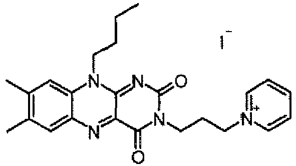

Aus 10-Butyl-7,8-dimethyl-10H-benzo[g]pteridin-2,4-dion (**56**) (310 mg, 1.0 mmol) und 3-(Pyridinium)-propyl-1-iodid iodid durch Umsetzung gemäß der allgemeinen Vorschrift XIII) als oranger Feststoff in einer Ausbeute von 196 mg (0,36 mmol, 36 % der Theorie) erhalten.

R_{f} = 0.05 (CHCl₃:MeOH - 6:1)
**¹H-NMR** (300 MHz, DMSO): δ [ppm] = 0,93 (t, 3 H), 1,48 (m, 2 H), 1,71 (m, 2H), 2,30 (m, 2 H), 2,46 (s, 3 H), 2,54 (s, 3 H,), 3,98 (m, 2 H), 4,61 (m, 2 H), 4,72 (m, 2 H), 5,31 (s, 2 H), 7,82 (s, 1 H), 7,92 (s, 1 H), 8,19 (m, 2 H), 8,61 (m, 1 H), 9,22 (d, J = 5.6 Hz, 1 H); - **MS** (ESI-MS, H₂O/MeOH + 0.1 % TFA): m/z (%) = 418.1 (100, (M⁺)); - **MS** (ESI-MS, H₂O/MeOH + 0.1 % TFA): m/z (%) = 418.2 (100, (M⁺)); - **MG** = 418.51 + 126.90 g/mol - **MF** = C₂₄H₂₈N₅O₂I

### XIIIc) [3-(10-Hexadecyl-7,8-dimethyl-2,4-dioxo-4,10-dihydro-2H-benzo[g]pteridin-3-yl)-propyl]-triethyl-ammonium iodid (76)

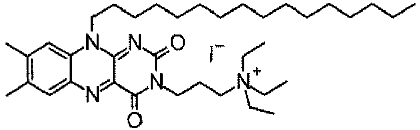

Das unter XII) erhaltene Flavin (**87**) (466 mg, 1.0 mmol) ergab bei Umsetzung nach der allgemeinen Vorschrift XIII) mit 3-(N,N,N-Triethylammonium)-propyl-1-iodid iodid 131 mg eines orangen Feststoffes (0,178 mmol, 18 % der Theorie)

R_{f} = 0.1 (CHCl₃:MeOH - 6:1)
**¹H-NMR** (300 MHz, DMSO): δ [ppm] = 0,85 (t, 3 H), 1,17 (m, 9 H), 1,03 (t, 3 H), 1,20 - 1,39 (m, 26 H), 1,52 (m, 2 H), 1,73 (m, 4H), 1,81 (m, 2 H), 2,26 (m, 2 H), 2,42 (s, 3 H), 2,53 (s, 3 H,), 3,28 (s, 6 H), 3,74 (m, 2 H), 3,46 (m, 2 H), 4,30 (m, 2 H), 4,72 (m, 2 H), 7,48 (s, 1 H), 8,05 (s, 1 H); - **MS** (ESI-MS, H₂O/MeOH + 0.1 % TFA): m/z (%) = 608.2 (100, (M⁺)); - **MG** = 608.90 + 126.90 g/mol - **MF** = C₃₇H₆₂N₅O₂I

### XIV) [3-(10-Bulyl-7,8-dimethyl-2,4-dioxo-4,10-dihydro-2H-benzo[g]pteridin-3-yl)-propyl]-tri(dioxa-3,6-heptyl)-ammonium iodid (77)

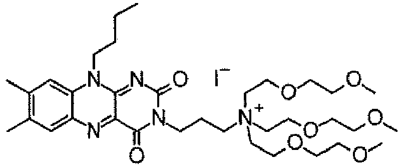

3-(10-Butyl-7,8-dimethy)-2,4-dioxo-4,10-dihydro-2H-benzo[g]pteridin-3-y))-propyliodid (**80**) (0.51 g, 1.05 mmol) wurde mit Tris(dioxa-3,6-heptyl)amine (646 mg, 2 mmol) und NaHCO₃ (0.1 g, 1.2 mmol) in DMF (4 mL) in Stickstoffatmosphäre im Dunklen bei 50°C für 24 h gerührt. Das Lösungsmittel wurde bei vermindertem Druck abgezogen und der Rückstand durch präparative Dünnschichtchromatographie gereinigt (CHCl₃/MeOH 6:1).

Es wurde 103 mg braunrotes, zähes Öl (0,13 mmol, 12 % der Theorie) erhalten.

**MS** (ESI-MS, H₂O/MeOH + 0.1 % TFA): m/z (%) = 662.1 (100, (M⁺)); - **MG** = 662.55 + 126.90 - **MF** = C₃₄H₅₆N₅O₈I

### XV) [3-(10-Butyl-7,8-dimethyl-2,4-dioxo-4,10-dihydro-2H-benzo[g]pteridin-3-yl)-propyl]-dimethyl-hexadecyl-ammonium iodid (78)

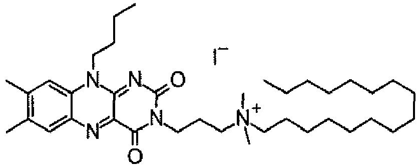

3-(10-Butyl-7,8-dimethyl-2,4-dioxo-4,10-dihydro-2H-benzo[g]pteridin-3-yl)-propyliodid (**80**) (0.51 g, 1.05 mmol) wurde mit N,N-Dimethyl-hexadecylamin (540 mg, 2 mmol) und NaHCO₃ (0.1 g, 1.2 mmol) in DMF (4 mL) in Stickstoffatmosphäre im Dunklen bei 50°C für 24 h gerührt. Das Lösungsmittel wurde bei vermindertem Druck abgezogen und der Rückstand durch präparative Dünnschichtchromatographie gereinigt (CHCl₃/MeOH 6:1).

Es wurde 147 mg hellbrauner Feststoff (0,20 mmol, 19 % der Theorie) erhalten.
**¹H-NMR** (300 MHz, CDCl₃): δ [ppm] = 0,83 (t, 3 H), 1,04 (t, 3 H), 1,21 - 1,38 (m, 26 H), 1,52 (m, 2 H), 1,71 (m, 4H), 1,82 (m, 2 H), 2,28 (m, 2 H), 2,41 (s, 3 H), 2,54 (s, 3 H,), 3,29 (s, 6 H), 3,75 (m, 2 H), 3,43 (m, 2 H), 4,29 (m, 2 H), 4,70 (m, 2 H), 7,41 (s, 1 H), 8,06 (s, 1 H); - **MS** (ESI-MS, H₂O/MeOH + 0.1 % TFA): m/z (%) = 608.1 (100, (M⁺)); - **MG** = 608.90 + 35.45 - **MF** = C₃₇H₆₂N₅O₂Cl

### Beispiel 2) Phototoxizitätsexperimente

### a) Herstellung der Testplatten und Bakterienstämme

Eine Probe des Bakterienstammes *Staphylococcus. aureus* (ATCC-Nummer: 25923) *oder Escherichia. coli* (ATCC-Nummer: 25922) wurde aus einer Cryo-Gefrierkultur entnommen, auf Müller-Hinton-Agar-Platten vereinzelt und unter aeroben Bedingungen bei 37°C in einer Übernachtkultur kultiviert. Daran anschließend wurden 5 ml Müller-Hinton-Flüssigmedium mit einem Abstrich der Bakterienkultur (Einzelkolonie) beimpft und über Nacht bei 37°C bebrütet. Die so gewonnene Bakteriensuspension wurde 10 min bei 2500 Upm zentrifugiert und das erhaltene Bakterienpellet in 5 ml sterilem PBS resuspendiert. Die optische Dichte der Bakteriensupensionen für die Phototoxizitätstests betrug OD₆₀₀ₙₘ= 0.6, was einer Bakterienzahl von ∼1-8x10⁸⁻¹² Bakterien pro ml entspricht. Die biochemische Analyse und Resistenzbestimmung der Bakterien wurde mit dem VITEK2-System gemäß den Richtlinien M100-S14 des NCCLS (2004) durchgeführt.

Zur Empfindlichkeitsprüfung medizinisch bedeutender Krankheitserreger gegenüber Antibiotika und Sulfonamiden wurden gemäß der NCCLS Richtlinien Müller-Hinton-Medien verwendet (Deutsche Gesellschaft für Hygiene und Mikrobiologie (DGHM), Institut für Hygiene und Mikrobiologie, Universität Bonn, Deutschland):
a) Müller-Hinton-Bouillon (Oxoid, Wesel, Deutschland)
   2,0 g/l Rindfleisch, getrocknete Infusion aus 300 g, 17,5 g/l Caseinhydrolysat, 1,5 g/l Stärke, pH: 7,4 + 0,2
b) Müller Hinton-Agar (Oxoid, Wesel, Deutschland)
   2,0 g/l Rindfleisch, getrocknete Infusion aus 300 g, 17,5 g/l Caseinhydrolysat,
   1,5 g/l Stärke, pH: 7,4 + 0,2
   13 g/l Agaragar

### b) Durchführung des Phototoxizitätstests:

200 µl einer Bakteriensuspension (Bakteriendichte: 10⁸-10¹²/ml) wurden mit je 200 µl verschiedener Konzentrationen der zu testenden Photosensibilisatoren bei Raumtemperatur für 10 min oder 30 min inkubiert. Danach wurden die Bakterien zweimal mit Aqua dest. gewaschen, in 200 µl Aqua dest. resuspendiert, das gesamte Volumen auf eine 96-well Mikrotiterplatte übertragen und anschließend bestrahlt. Die verwendeten Photosensibilisatoren wurden in Aqua dest. gelöst und verschiedene Verdünnungsreihen hergestellt (0µM, 1µM, 10µM, 100µM),

Zur Sensibilisierung wurde die Lampe Omnicure Series 2000 (Photonics Solutions Inc., Edinburgh, UK) benutzt, die Licht aus einem Bereich von 390 nm bis 500nm emittiert und Emissionsmaxima Eₘₐₓ bei 405 nm und 436 nm aufweist. Die applizierte Leistung betrug jeweils 50mW/cm².

Als Kontrollen wurden bestrahlte und nicht bestrahlte Proben verwendet. Ebenso wurden nur mit Photosensibilisator inkubierte Bakteriensuspensionen mitgeführt (Dunkelkontrolle).

Die Bestimmung der koloniebildenden Einheiten (KBE bzw. CFU) pro ml wurde gemäß der von Miles und Misra publizierten Methode durchgeführt (Miles, AA; Misra, SS, Irwin, JO (1938 Nov). "The estimation of the bactericidal power of the blood.". The Journal of hygiene 38 (6): 732-49). Dazu wurden serielle Verdünnungen von 10⁻² bis 10⁻⁹ der entsprechenden Bakteriensuspension hergestellt. Je 3x 20 µl der entsprechenden Bakterienverdünnungen wurden dann auf Müller-Hinton Platten aufgetropft und bei 37° C für 24 h inkubiert. Danach wurde die Anzahl der überlebenden koloniebildenden Einheiten (KBE bzw. CFU) bestimmt. Alle Versuche wurden jeweils dreimal wiederholt.

### c) Ergebnis der Phototoxizitätsexperimente:

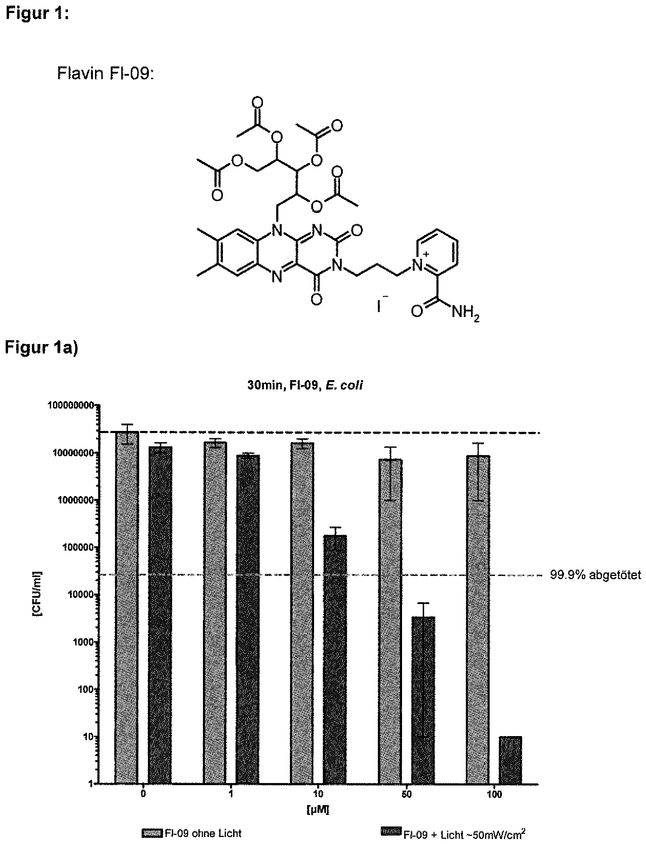

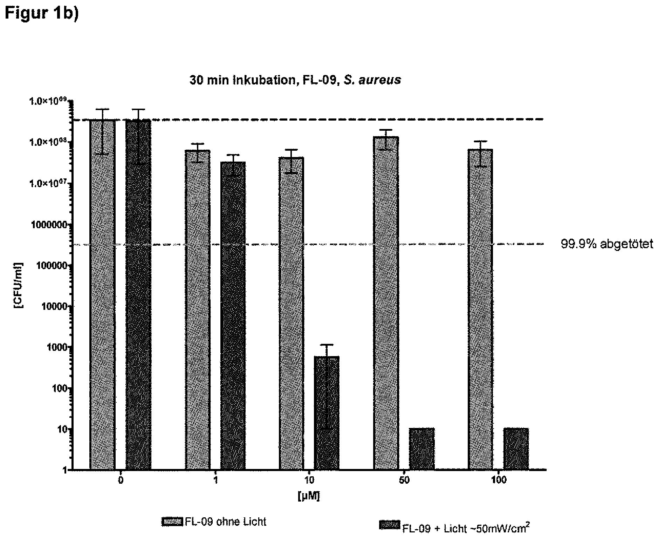

Die Ergebnisse der Phototoxizitätsexperimente sind in den Figuren 1-7 dargestellt.

Die Figuren 1-8 zeigen die logarithmische Abnahmen der CFU/ml 24 Std nach Bestrahlung sowie die dazugehörigen Kontrollen (nur bestrahlte Bakterien; mit Photosensibilisator inkubierte Bakterien, aber nicht bestrahlt; unbehandelte Bakterien) für den jeweils angegeben Photosensibilisator.

Die angegebenen koloniebildenden Einheiten (KBE bzw. CFU) pro ml sind jeweils der Median aus drei Versuchen.
Figur 1 zeigt die Wirkung von Flavin FL-09 (Iodid der Verbindung mit den Formeln (36)) auf E. coli und S. aureus.
   Figur 1a: E. coli Proben wurden mit verschiedenen Konzentrationen (0 µM, 1 µM, 10 µM, 50 µM, 100 µM) von Flavin FL-09 für 30 min inkubiert. Anschließend wurden die Proben entweder mit 50 mW/cm² (210 sec; 10.5 J/cm²) bestrahlt (dunkelgraue Balken) oder nicht bestrahlt (hellgraue Balken). Nach 24 h wurden die überlebenden Kolonien ausgezählt (CFU/ml). Die schwarze Linie kennzeichnet die Referenz der Dunkelkontrolle (0 µM Flavin FL-09, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.
   Figur 1b: S. aureus Proben wurden mit verschiedenen Konzentrationen (0 µM, 1 µM, 10 µM, 50 µM, 100 µM) von Flavin FL-09 für 30 min inkubiert. Anschließend wurden die Proben entweder mit 50 mW/cm² (210 sec; 10.5 J/cm²) bestrahlt (dunkelgraue Balken) oder nicht bestrahlt (hellgraue Balken). Nach 24 h wurden die überlebenden Kolonien ausgezählt (CFU/ml). Die schwarze Linie kennzeichnet die Referenz der Dunkelkontrolle (0 µM Flavin FL-09, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

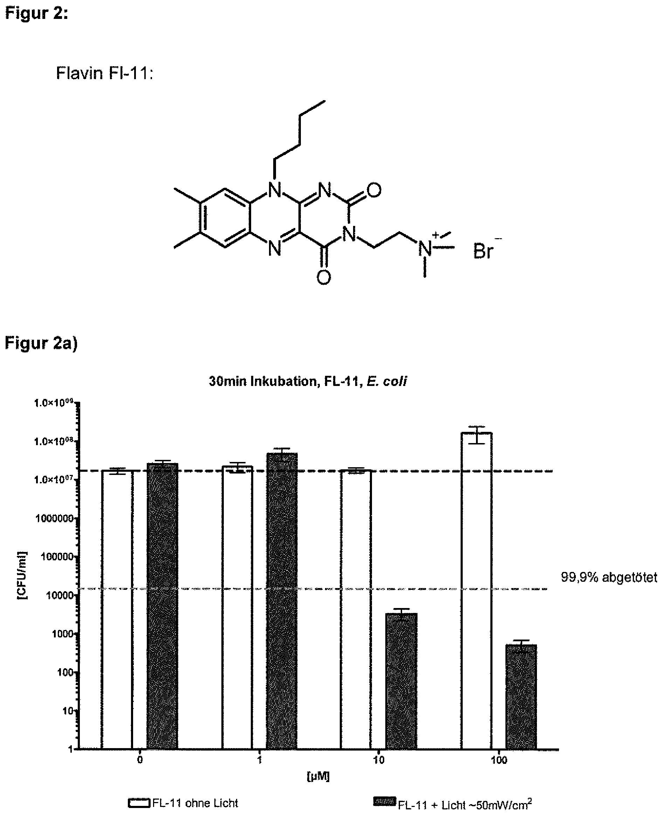

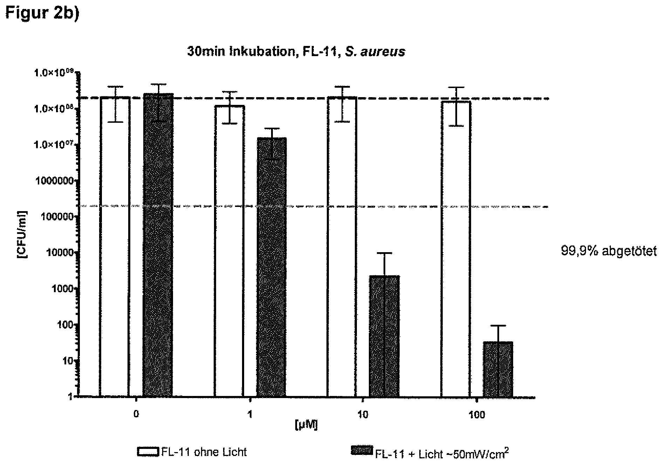

Figur 2 zeigt die Wirkung von Flavin FL-11 (Bromid der Verbindung mit den Formeln (32)) auf E. coli und S. aureus.
   Figur 2a: E. coli Proben wurden mit verschiedenen Konzentrationen (0 µM, 1 µM, 10 µM, 100 µM) von Flavin FL-11 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-11, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.
   Figur 2b: S. aureus Proben wurden mit verschiedenen Konzentrationen (0 µM, 1 µM, 10 µM, 50 µM, 100 µM) von Flavin FL-11 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-11, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

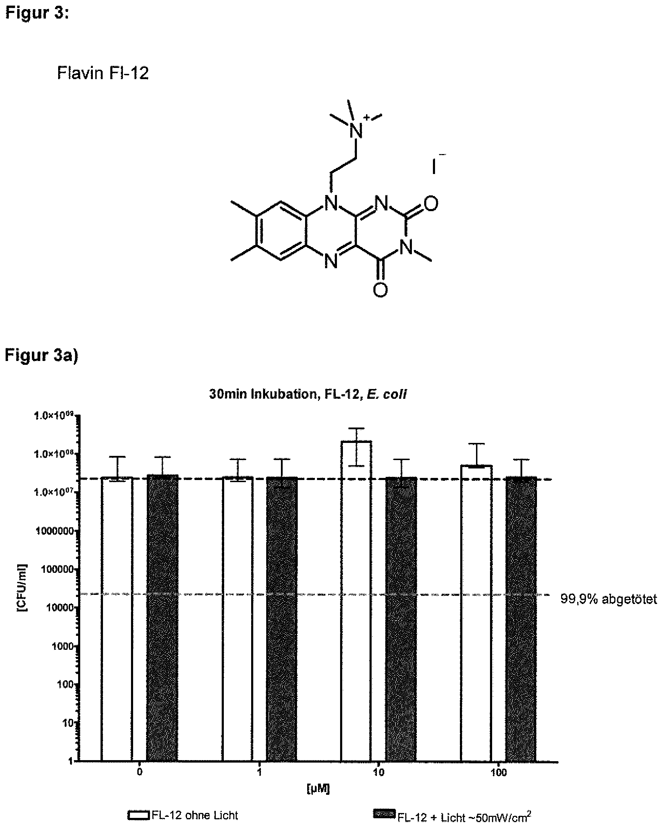

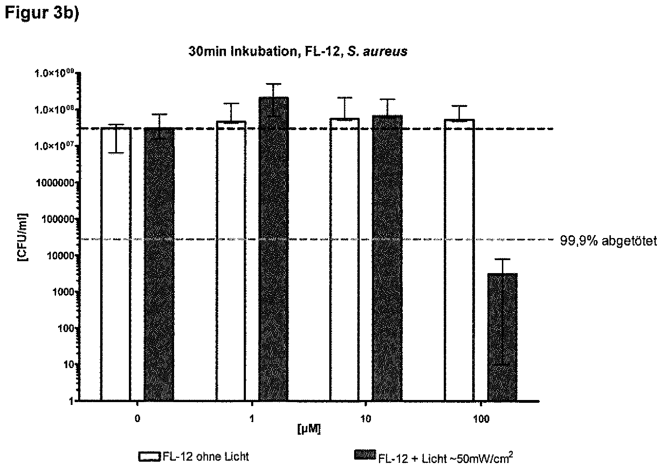

Figur 3 zeigt die Wirkung von Flavin FL-12 (lodid der Verbindung mit den Formeln (30)) auf E. coli und S. aureus.
   Figur 3a: E. coli Proben wurden mit verschiedenen Konzentrationen (0 µM, 1 µM, 10 µM, 100 µM) von Flavin FL-12 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-12, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.
   Figur 3b: S. aureus Proben wurden mit verschiedenen Konzentrationen (0 µM, 1 µM, 10 µM, 50 µM, 100 µM) von Flavin FL-12 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-12, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

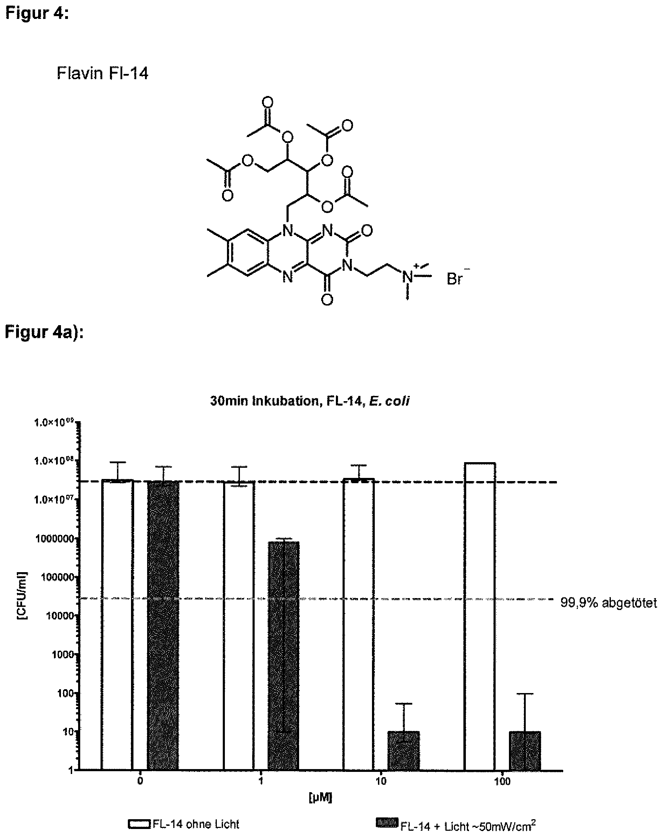

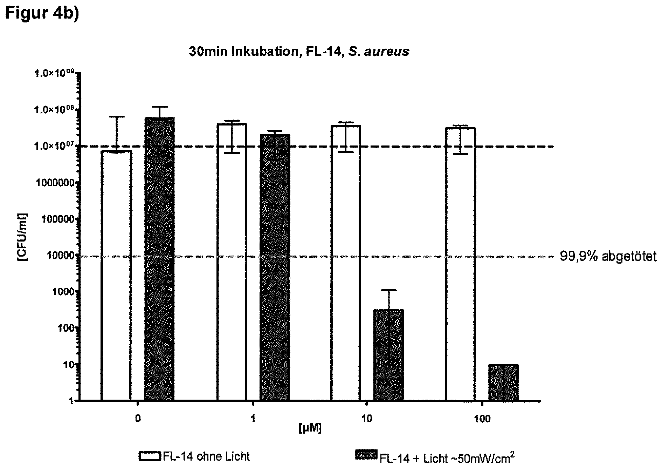

Figur 4 zeigt die Wirkung von Flavin FL-14 (Bromid der Verbindung mit den Formeln (35)) auf E. coli und S. aureus.
   Figur 4a: E. coli Proben wurden mit verschiedenen Konzentrationen (0 µM, 1 µM, 10 µM, 100 µM) von Flavin FL-14 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-14, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.
   Figur 4b: S. aureus Proben wurden mit verschiedenen Konzentrationen (0 µM, 1 µM, 10 µM, 50 µM, 100 µM) von Flavin FL-14 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-14, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

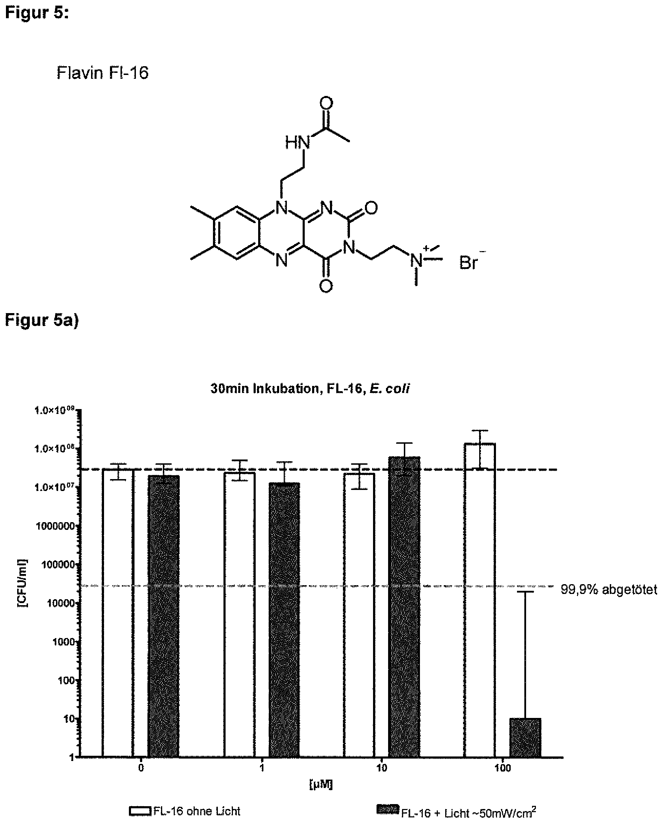

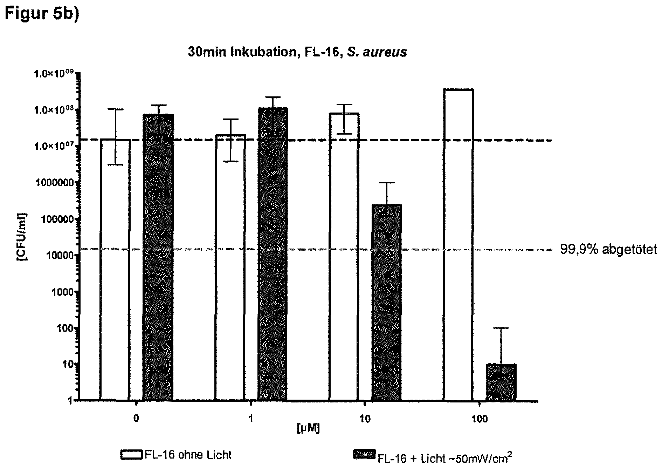

Figur 5 zeigt die Wirkung von Flavin FL-16 (Bromid der Verbindung mit den Formeln (33)) auf E. coli und S. aureus.
   Figur 5a: E. coli Proben wurden mit verschiedenen Konzentrationen (0 µM, 1 µM, 10 µM, 100 µM) von Flavin FL-16 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-16, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.
   Figur 5b: S. aureus Proben wurden mit verschiedenen Konzentrationen (0 µM, 1 µM, 10 µM, 50 µM, 100 µM) von Flavin FL-16 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-16, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

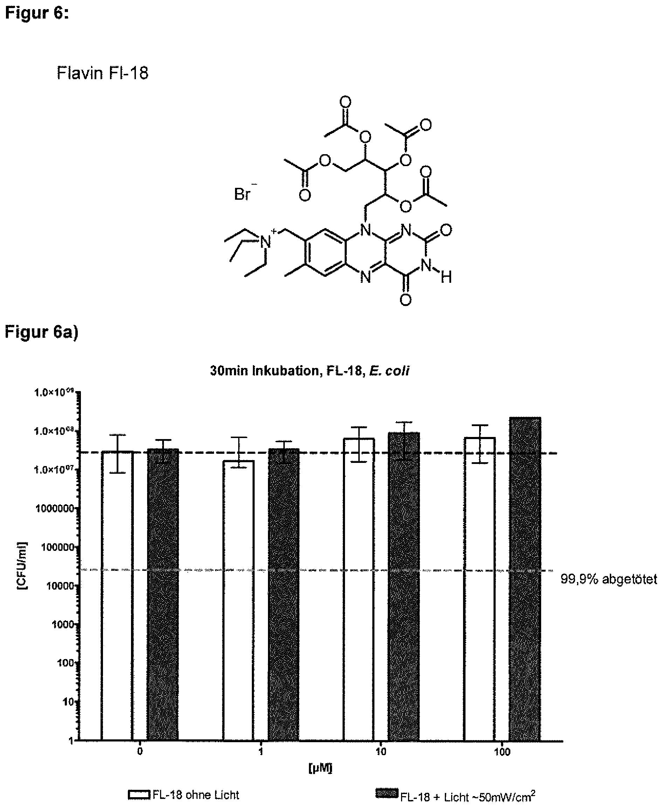

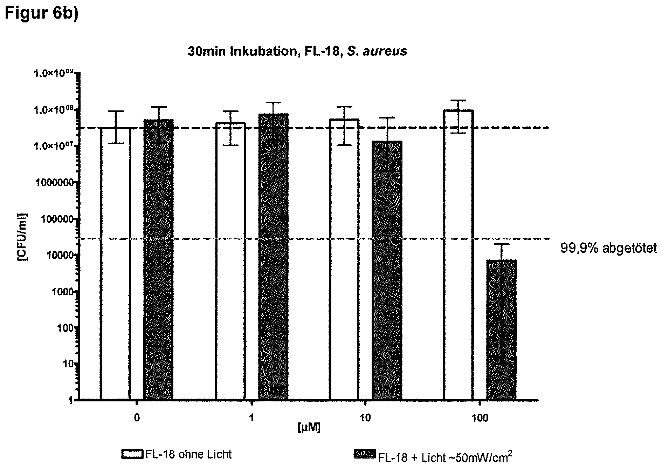

Figur 6 zeigt die Wirkung von Flavin FL-18 (Bromid der Verbindung mit den Formeln (37)) auf E. coli und S. aureus.
   Figur 6a: E. coli Proben wurden mit verschiedenen Konzentrationen (0 µM, 1 µM, 10 µM, 100 µM) von Flavin FL-18 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-18, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.
   Figur 6b: S. aureus Proben wurden mit verschiedenen Konzentrationen (0 µM, 1 µM, 10 µM, 50 µM, 100 µM) von Flavin FL-18 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-18, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

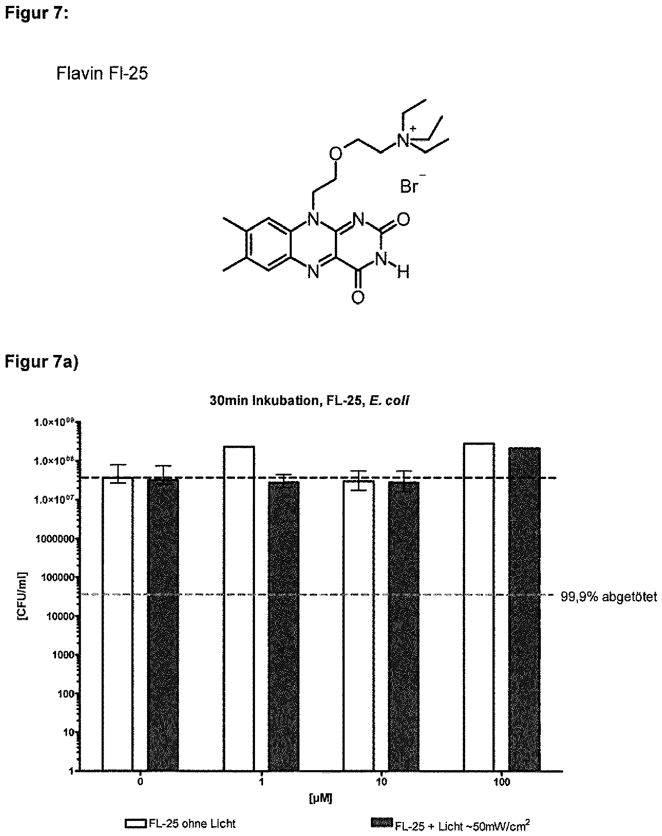

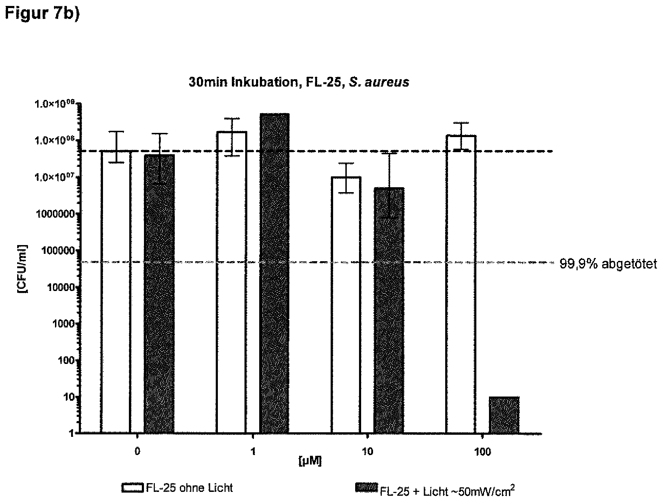

Figur 7 zeigt die Wirkung von Flavin FL-25 (Bromid der Verbindung mit den Formeln (31)) auf E. coli und S. aureus.
   Figur 7a: E. coli Proben wurden mit verschiedenen Konzentrationen (0 µM, 1 µM, 10 µM, 100 µM) von Flavin FL-25 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-25, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.
   Figur 7b: S. aureus Proben wurden mit verschiedenen Konzentrationen (0 µM, 1 µM, 10 µM, 50 µM, 100 µM) von Flavin FL-25 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-25, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

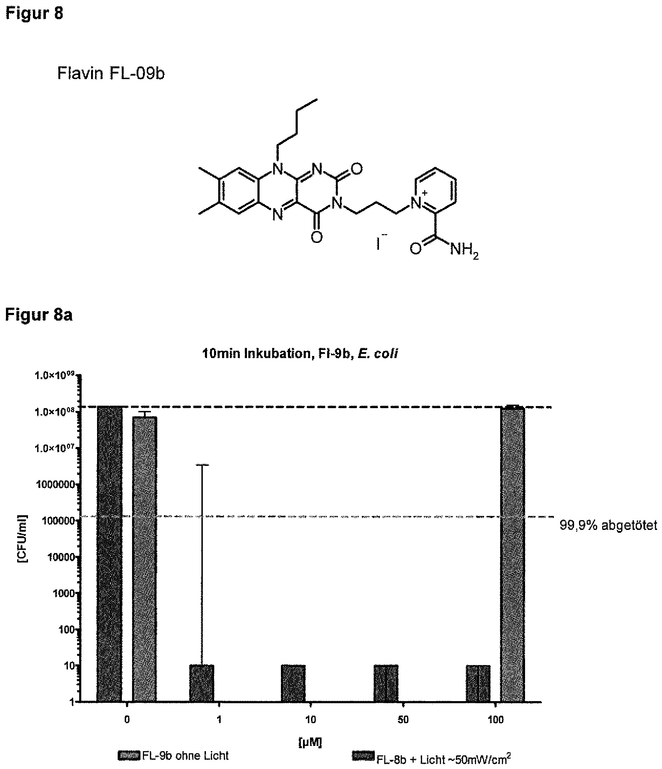

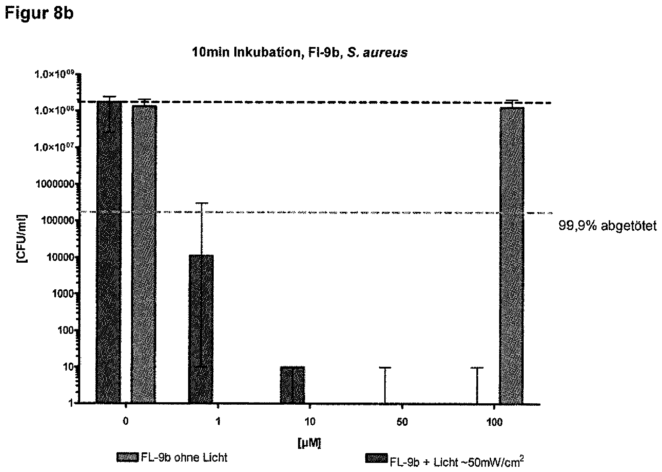

Figur 8 zeigt die Wirkung von Flavin FL-9a (Jodid der Verbindung mit den Formeln (43)) auf E. coli und S. aureus.
   Figur 8a: E. coli wurde mit verschiedenen Konzentrationen von FL-09b [µM] für 10 min inkubiert, anschließend wurden die Proben mit 50mW/cm2 (210sec; 10.5J/cm2) bestrahlt. 24h später wurde die überlebenden Kolonien ausgezählt (CFU/ml). Graue Balken: Dunkelkontrolle ohne Licht. Rote Balken: mit Photosensibilisator inkubiert und bestrahlt. Die schwarze Linie kennzeichnet die Referenz der Dunkelkontrolle (kein Photosensibilisatorinkubation, kein Licht). Die grüne Linie kennzeichnet eine Abnahme der CFU/ml um 3-log10-Stufen; dies entspricht eine Abnahme um 99.9% ("antibakterielle Wirkung"). N= 3; Median CFU/ml ± SEM.
   Figur 8a: S. aureus wurde mit verschiedenen Konzentrationen von FL-09b [µM] für 10 min inkubiert, anschließend wurden die Proben mit 50mW/cm2 (210sec; 10.5J/cm2) bestrahlt. 24h später wurde die überlebenden Kolonien ausgezählt (CFU/ml). Graue Balken: Dunkelkontrolle ohne Licht. Rote Balken: mit Photosensibilisator inkubiert und bestrahlt. Die schwarze Linie kennzeichnet die Referenz der Dunkelkontrolle (kein Photosensibilisatorinkubation, kein Licht). Die grüne Linie kennzeichnet eine Abnahme der CFU/ml um 3-log10-Stufen; dies entspricht eine Abnahme um 99.9% ("antibakterielle Wirkung"). N= 3; Median CFU/ml ± SEM.

Wie aus den Figuren 1-8 ersichtlich, hat eine Bestrahlung der verwendeten Mikroorganismen *Staphylococcus aureus* (*S. aureus*) und *Escherichia coli* (*E. coli*) mit einer Lichtdosis von 10.5J/cm² mit blauem Licht (390nm - 500nm) in Abwesenheit eines Photosensibilisators (µM des jeweiligen Flavins) keinen Einfluss auf die Anzahl der überlebenden Mikroorganismen im Vergleich zur unbelichteten Kontrolle.

Darüber hinaus zeigen die in den Figuren 1-8 dargestellten Ergebnisse, dass die Inkubation (10min bzw. 30min) des jeweiligen Photosensibilisators mit den Mikroorganismen ohne nachfolgende Belichtung ebenfalls keinen Einfluss auf die Anzahl der überlebenden Mikroorganismen hat.

Wie aus den Figuren 1-8 ersichtlich erfolgt nach Inkubation (10min bzw. 30min) der Mikroorganismen in Abhängigkeit der verwendeten Konzentration der jeweiligen Photosensibilisatoren und nachfolgender Bestrahlung mit einer Lichtdosis von 10.5J/cm² eine Abnahme der KBE/ml und somit eine Inaktivierung von *E*. *coli* und *S*. *aureus.*

Die Effektivität der Phototoxizität gegenüber Bakterien nach Bestrahlung wurde nach folgenden Richtlinen für Handhygiene im Gesundheitswesen festgelegt (Boyce, J. M., and D. Pittet. 2002. Guideline for Hand Hygiene in Health-Care Settings: recommendations of the Healthcare Infection Control Practices Advisory Committee and the HICPAC/SHEA/APIC/IDSA Hand Hygiene Task Force, infect Control Hosp Epidemiol 23:S3-40.):

| | |
|---|---|
| - Reduktion der KBE/ml um 1 log₁₀-Stufe | ≙ 90% Effektivität |
| - Reduktion der KBE/ml um 3 log₁₀-Stufen | ≙ 99,9% Effektivität |
| - Reduktion der KBE/ml um 5 log₁₀-Stufen | ≙ 99,999% Effektivität |

Für eine effektive Inaktivierung kann daher die Abnahme von 2≥3log₁₀ Stufen angenommen werden, wobei *S*. *aureus* und *E. coli* als Beispiele für Vertreter aus der Gruppe der Gram-positiven und Gram-negativen Bakterien ausgewählt wurden (siehe Boyce J.M and D. Pittet 2009).

Die benötigte Konzentration, um jeweils eine Reduktion um ≥3log₁₀ Stufen zu erzielen, ist in Tabelle 2 dargestellt.

**Tabelle 2: Zusammenfassung photodynamische Inaktivierung**

| **Photosensibilisator** | **Benötigte Konzentration [µM], um eine Reduktion um ≥ 3log₁₀ Stufen zu erzielen (Abnahme um 99,9%), Bestrahlung mit 10.5 J/cm²** | |
|---|---|---|
| | ***E .coli*** | ***S. aureus*** |
| **FL-09** | 50 | 10 |
| **FL-09b** | 1 | 1 |
| **FL-11** | 10 | 10 |
| **FL-12** | ---^{(*)} | 100 |
| **FL-14** | 10 | 10 |
| **FL-16** | 100 | 100 |
| **FL-18** | ---^{(*)} | 100 |
| **FL-25** | ---^{(*)} | 100 |

| | | |
|---|---|---|
| (*) Reduktion weniger als 1log₁₀ unter den bisher untersuchten Bedingungen^{^} | | |

## Patentansprüche

1. Verwendung einer Verbindung mit der Formel (1) 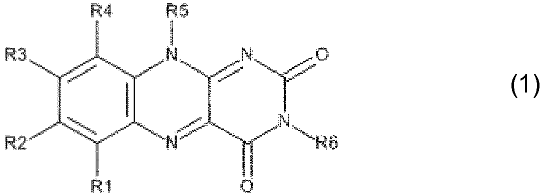 bei der Oberflächenreinigung, insbesondere von Medizinprodukten, Lebensmittelverpackungen oder Hygieneartikeln, wobei
A) nur 1 Rest R1, R2, R3 oder R4 ein organischer Rest der allgemeinen Formel - (C(D)(E))ₕ-X oder -(C(D)(E))ₖ-Aryl-(C(D)(E))ₗ-X, wobei h eine ganze Zahl von 1 bis 20 ist und k, l jeweils unabhängig voneinander eine ganze Zahl von 0 bis 6 bedeuten, wobei D und E jeweils unabhängig voneinander Wasserstoff, Halogen, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet, ist und
wobei die Reste R1, R2, R3 oder R4, die nicht -(C(D)(E))ₕ-X oder - (C(D)(E))ₖ-Aryl-(C(D)(E))ₗ-X sind, jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten und wobei jeder der Reste R5 oder R6 jeweils unabhängig voneinander gleich oder voneinander verschieden ist und Wasserstoff, Alkyl mit 1 bis 20 C-Atomen, Alkenyl mit 1 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und wobei R5 ein nichtcyclischer Polyolrest der allgemeinen Formel -CH₂(CH(OH))_{g}CH₂OH oder ein Ether, Ester oder Acetal davon, wobei g eine ganze Zahl von 1 bis 10, vorzugsweise 1 bis 4, bedeutet, sein kann,
oder
wobei B) nur 1 Rest R5 oder R6 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20 ist und k, l jeweils unabhängig voneinander eine ganze Zahl von 0 bis 6 bedeuten, wobei D und E jeweils unabhängig voneinander Wasserstoff, Halogen, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet, ist,
und
wobei der Rest R5 oder R6, der nicht -(C(D)(E))ₕ-X oder -(C(D)(E))ₖ-Aryl-(C(D)(E))ₗ-X ist, Wasserstoff, Alkyl mit 1 bis 20 C-Atomen, Alkenyl mit 2 bis 20 C-Atomen, Cycloalkyl mit 3 bis 20 C-Atomen, Ether mit 2 bis 20 C-Atomen, Thioether mit 2 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und wobei die Reste R1 bis R4 jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten, und wobei der Rest R5 ein nichtcyclischer Polyolrest der allgemeinen Formel -CH₂(CH(OH))_{g}CH₂OH oder ein Ether, Ester oder Acetal davon, wobei g eine ganze Zahl von 1 bis 10, vorzugsweise 1 bis 4, bedeutet, sein kann.

2. Verwendung einer Verbindung mit der Formel (1) 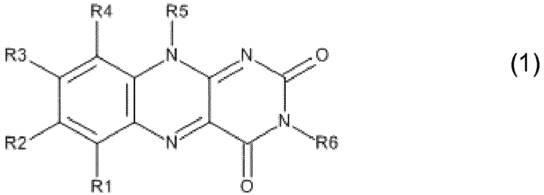 bei der Oberflächenbeschichtung von Medizinprodukten, Lebensmittelverpackungen oder Hygieneartikeln,
wobei A) nur 1 Rest R1, R2, R3 oder R4 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20 ist und k, l jeweils unabhängig voneinander eine ganze Zahl von 0 bis 6 bedeuten, wobei D und E jeweils unabhängig voneinander Wasserstoff, Halogen, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet, ist, und
wobei die Reste R1, R2, R3 oder R4, die nicht -(C(D)(E))ₕ-X oder - (C(D)(E))ₖ-Aryl-(C(D)(E))ₗ-X sind, jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten und wobei jeder der Reste R5 oder R6 jeweils unabhängig voneinander gleich oder voneinander verschieden ist und Wasserstoff, Alkyl mit 1 bis 20 C-Atomen, Alkenyl mit 1 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und wobei der Rest R5 ein nichtcyclischer Polyolrest der allgemeinen Formel -CH₂(CH(OH))_{g}CH₂OH oder ein Ether, Ester oder Acetal davon, wobei g eine ganze Zahl von 1 bis 10, vorzugsweise 1 bis 4, bedeutet, sein kann,
oder
wobei B) nur 1 Rest R5 oder R6 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20 ist und k, l jeweils unabhängig voneinander eine ganze Zahl von 0 bis 6 bedeuten, wobei D und E jeweils unabhängig voneinander Wasserstoff, Halogen, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet, ist,
und
wobei der Rest R5 oder R6, der nicht -(C(D)(E))ₕ-X oder -(C(D)(E))ₖ-Aryl-(C(D)(E))ₗ-X ist, Wasserstoff, Alkyl mit 1 bis 20 C-Atomen, Alkenyl mit 2 bis 20 C-Atomen, Cycloalkyl mit 3 bis 20 C-Atomen, Ether mit 2 bis 20 C-Atomen, Thioether mit 2 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und wobei die Reste R1 bis R4 jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten, und wobei der Rest R5 ein nichtcyclischer Polyolrest der allgemeinen Formel -CH₂(CH(OH))_{g}CH₂OH oder ein Ether, Ester oder Acetal davon, wobei g eine ganze Zahl von 1 bis 10, vorzugsweise 1 bis 4, bedeutet, sein kann.

3. Verwendung nach Anspruch 1 oder 2, wobei die Reste R1 und R4, die jeweils unabhängig voneinander gleich oder voneinander verschieden sein können, Wasserstoff oder Methyl sind und wobei nur 1 Rest R2, R3, R5 oder R6 ein organischer Rest der allgemeinen Formel -(C(D)(E))ₕ-X oder-(C(D)(E))ₖ-Aryl-(C(D)(E))ₗ-X ist.

4. Verwendung nach einem der Ansprüche 1 bis 3, wobei X einen Rest der allgemeinen Formel (2): 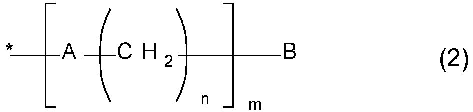 bedeutet und wobei A ein Sauerstoff- oder ein Schwefelatom ist und wobei n eine ganze Zahl von 1 bis 8 und m eine ganze Zahl von 0 bis 100 ist und wobei B ein Rest der Formel (3), (4a), (4b), (5a) oder (5b): 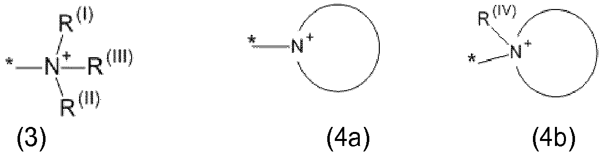 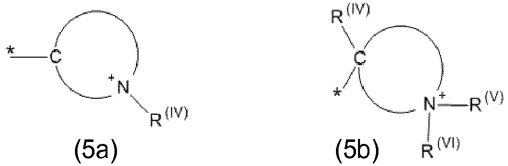 ist und wobei jeder der Reste R^{(I)}, R^{(II)}, R^{(III)}, R^{(IV)} und R^{(V)} unabhängig voneinander ein C1 - C20 Aryl, ein C1 - C20 Alkyl, das geradkettig oder verzweigt sein kann,
oder ein C1 - C20 Ether ist und wobei der Rest mit der Formel (4a) und der Rest mit der Formel (5a): 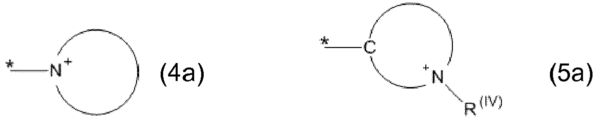 einen substituierten oder unsubstituierten heterocyclischen Rest mit 5 bis 7 Ringatomen, die mindestens ein 1 Kohlenstoffatom und 1 Stickstoffatom sowie optional 1 oder 2 Sauerstoff- oder Schwefelatom umfassen, wobei 1 Stickstoffatom eine Doppelbindung ausbildet, darstellt und wobei der Rest mit der Formel (4b) und der Rest mit der Formel (5b): 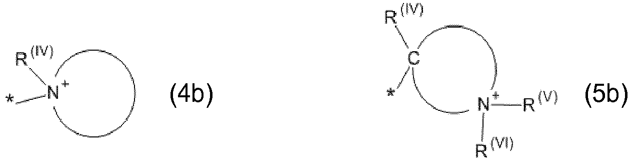 einen substituierten oder unsubstituierten heterocyclischen Rest mit 5 bis 7 Ringatomen, die mindestens ein 1 Kohlenstoffatom und 1 Stickstoffatom sowie optional 1 oder 2 Sauerstoff- oder Schwefelatom umfassen, wobei 1 Stickstoffatom eine Einfachbindung ausbildet, darstellt.

5. Verwendung nach einem der Ansprüche 1 bis 4, wobei die Verbindung mit der Formel (1) aus der Gruppe, die aus Verbindungen der Formeln (30) bis (43) besteht, ausgewählt wird:

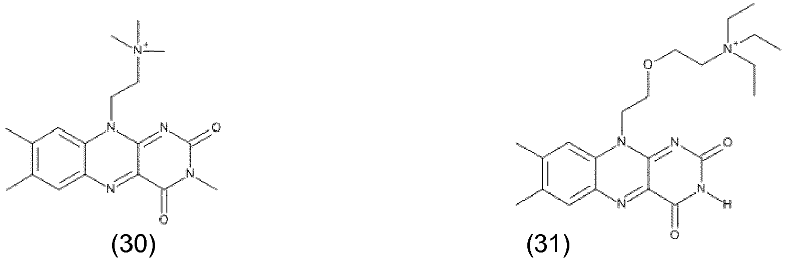

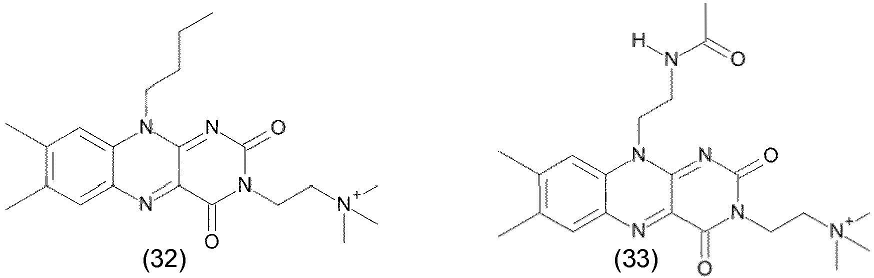

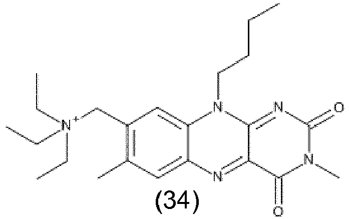

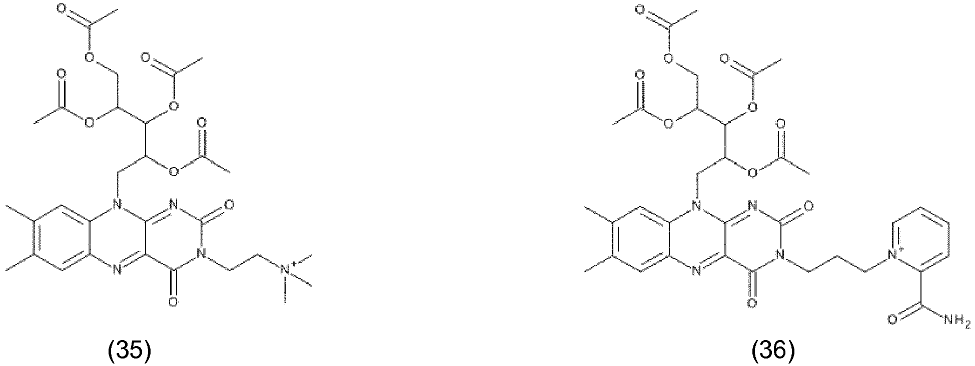

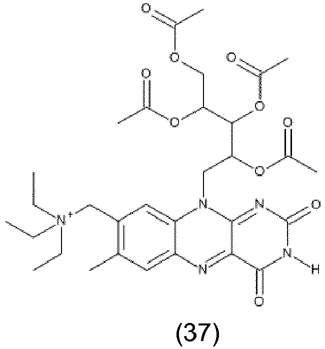

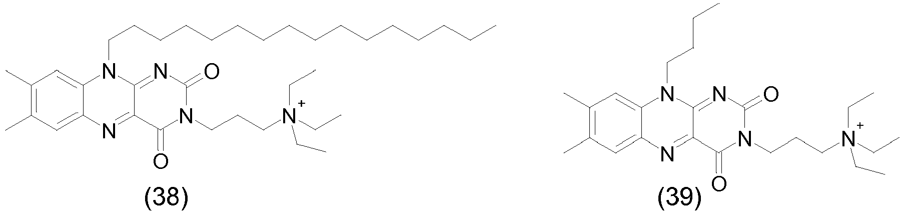

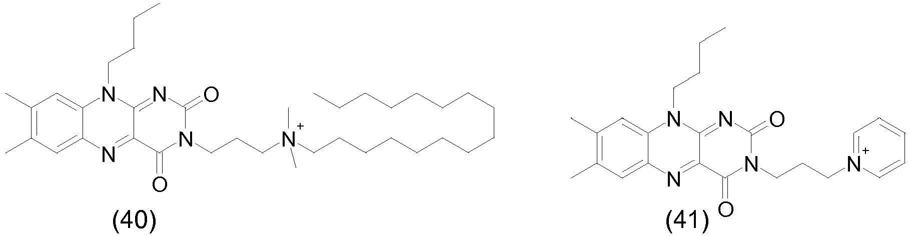

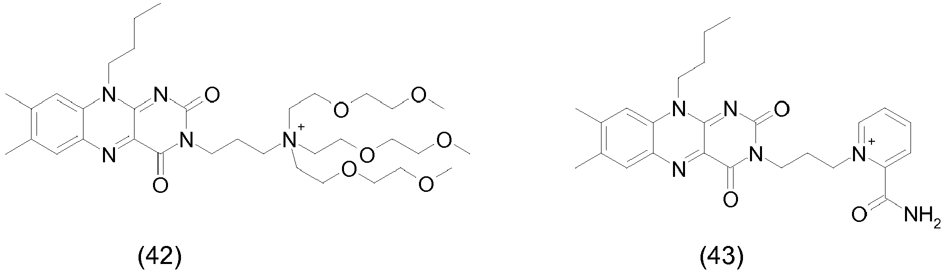

6. Beschichteter Gegenstand, wobei der Gegenstand aus der Gruppe, die aus Medizinprodukten, Lebensmittelverpackungen und Hygieneartikeln besteht, ausgewählt ist und der Gegenstand mit einer Verbindung gemäß Formel (1) 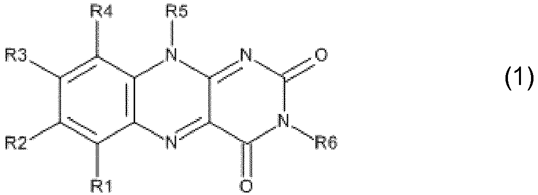 beschichtet ist,
wobei A) nur 1 Rest R1, R2, R3 oder R4 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20 ist und k, l jeweils unabhängig voneinander eine ganze Zahl von 0 bis 6 bedeuten, wobei D und E jeweils unabhängig voneinander Wasserstoff, Halogen, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet, ist und
wobei die Reste R1, R2, R3 oder R4, die nicht -(C(D)(E))ₕ-X oder - (C(D)(E))ₖ-Aryl-(C(D)(E))ₗ-X sind, jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten und wobei jeder der Reste R5 oder R6 jeweils unabhängig voneinander gleich oder voneinander verschieden ist und Wasserstoff, Alkyl mit 1 bis 20 C-Atomen, Alkenyl mit 1 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und wobei der Rest R5 ein nichtcyclischer Polyolrest der allgemeinen Formel -CH₂(CH(OH))_{g}CH₂OH oder ein Ether, Ester oder Acetal davon, wobei g eine ganze Zahl von 1 bis 10, vorzugsweise 1 bis 4, bedeutet, sein kann,
oder
wobei B) nur 1 Rest R5 oder R6 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20 ist und k, l jeweils unabhängig voneinander eine ganze Zahl von 0 bis 6 bedeuten, wobei D und E jeweils unabhängig voneinander Wasserstoff, Halogen, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet, ist, und
wobei der Rest R5 oder R6, der nicht -(C(D)(E))ₕ-X oder -(C(D)(E))ₖ-Aryl-(C(D)(E))ₗ-X ist, Wasserstoff, Alkyl mit 1 bis 20 C-Atomen, Alkenyl mit 2 bis 20 C-Atomen, Cycloalkyl mit 3 bis 20 C-Atomen, Ether mit 2 bis 20 C-Atomen, Thioether mit 2 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und wobei der Rest R5 ein nichtcyclischer Polyolrest der allgemeinen Formel - CH₂(CH(OH))_{g}CH₂OH oder ein Ether, Ester oder Acetal davon, wobei g eine ganze Zahl von 1 bis 10, vorzugsweise 1 bis 4, bedeutet, sein kann, und wobei die Reste R1 bis R4 jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten, und wobei der Rest R5 ein nichtcyclischer Polyolrest der allgemeinen Formel - CH₂(CH(OH))_{g}CH₂OH ist oder ein Ether, Ester oder Acetal davon, wobei g eine ganze Zahl von 1 bis 10, vorzugsweise 1 bis 4, bedeutet, sein kann.

7. Beschichteter Gegenstand nach Anspruch 6, wobei die Reste R1 und R4, die jeweils unabhängig voneinander gleich oder voneinander verschieden sein können, Wasserstoff oder Methyl sind und wobei nur 1 Rest R2, R3, R5 oder R6 ein organischer Rest der allgemeinen Formel -(C(D)(E))ₕ-X oder-(C(D)(E))ₖ-Aryl-(C(D)(E))ₗ-X ist.

8. Beschichteter Gegenstand nach einem der Ansprüche 6 oder 7, wobei X einen Rest der allgemeinen Formel (2): 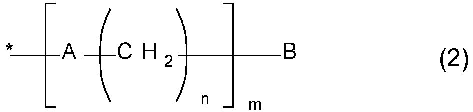 bedeutet und wobei A ein Sauerstoff- oder ein Schwefelatom ist und wobei n eine ganze Zahl von 1 bis 8 und m eine ganze Zahl von 0 bis 100 ist und wobei B ein Rest der Formel (3), (4a), (4b), (5a) oder (5b): 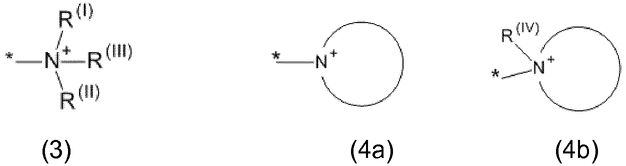 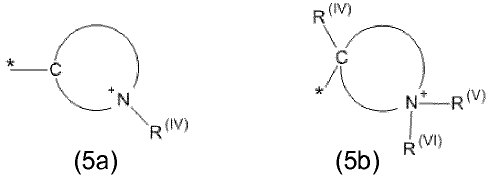 ist und wobei jeder der Reste R^{(I)}, R^{(II)}, R^{(III)}, R^{(IV)} und R^{(V)} unabhängig voneinander ein C1 - C20 Aryl, ein C1 - C20 Alkyl, das geradkettig oder verzweigt sein kann, oder ein C1 - C20 Ether ist und wobei der Rest mit der Formel (4a) und der Rest mit der Formel (5a): 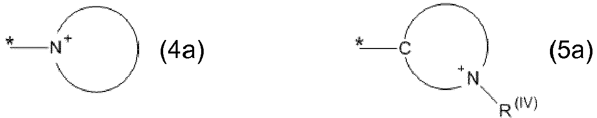 einen substituierten oder unsubstituierten heterocyclischen Rest mit 5 bis 7 Ringatomen, die mindestens ein 1 Kohlenstoffatom und 1 Stickstoffatom sowie optional 1 oder 2 Sauerstoff- oder Schwefelatom umfassen, wobei 1 Stickstoffatom eine Doppelbindung ausbildet, darstellt und wobei der Rest mit der Formel (4b) und der Rest mit der Formel (5b): 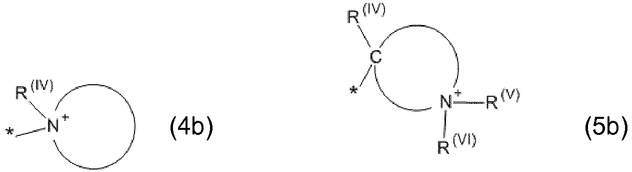 einen substituierten oder unsubstituierten heterocyclischen Rest mit 5 bis 7 Ringatomen, die mindestens ein 1 Kohlenstoffatom und 1 Stickstoffatom sowie optional 1 oder 2 Sauerstoff- oder Schwefelatom umfassen, wobei 1 Stickstoffatom eine Einfachbindung ausbildet, darstellt.

9. Beschichteter Gegenstand nach einem der Ansprüche 6 bis 8, wobei die Verbindung mit der Formel (1) aus der Gruppe, die aus Verbindungen der Formeln (30) bis (43) besteht, ausgewählt wird

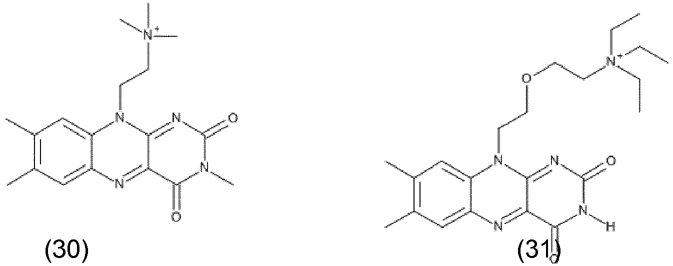

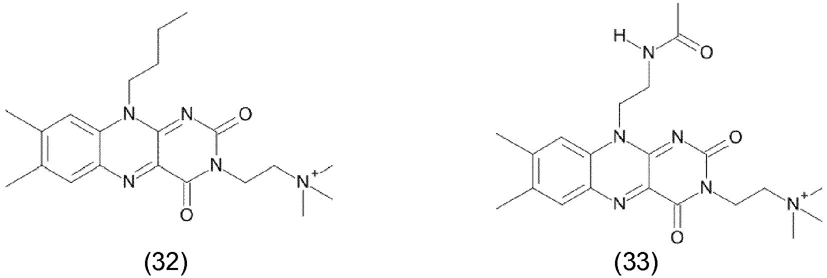

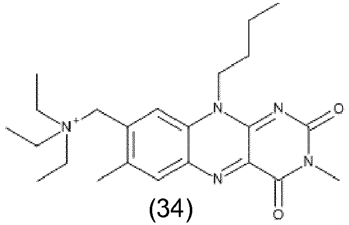

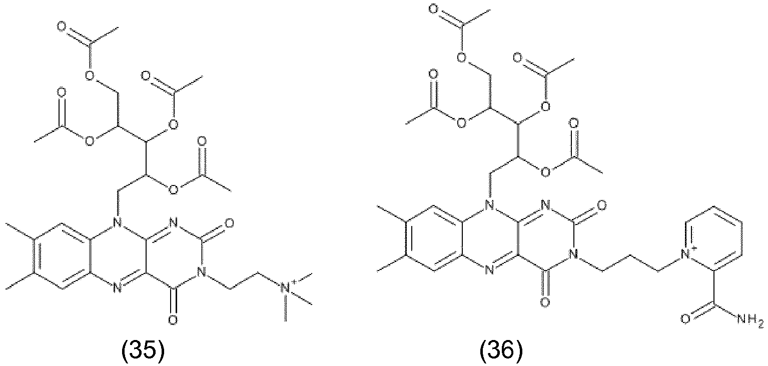

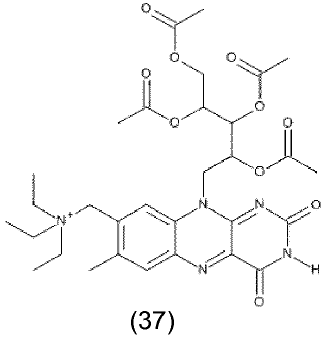

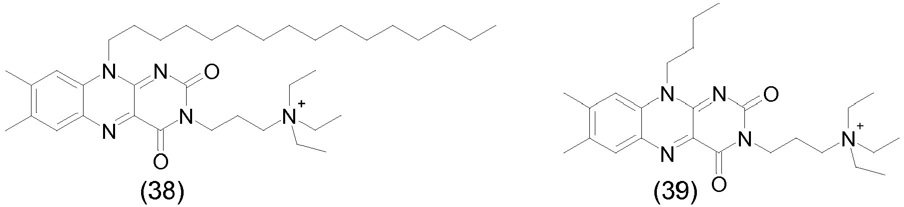

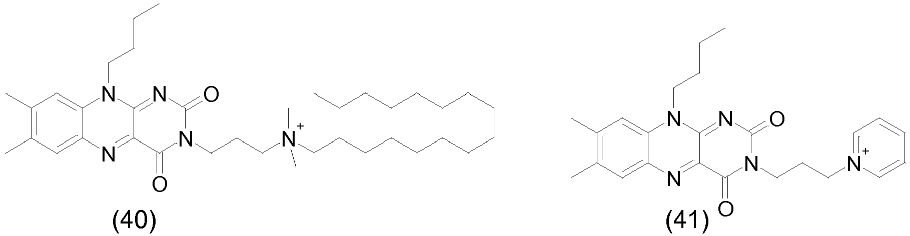

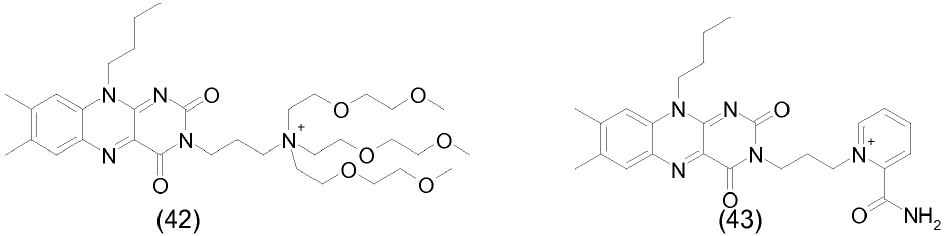

10. Beschichteter Gegenstand nach einem der Ansprüche 6 bis 9, wobei das Medizinprodukt aus der Gruppe, die aus invasiven medizinischen Hilfsmitteln, Wundauflagen, Verbänden, zahnärztlichen Abdrücken, Zahnersatz, wie Prothesen, Kronen oder Implantaten, besteht, ausgewählt werden.

11. Beschichteter Gegenstand nach Anspruch 10, wobei die invasiven medizinischen Hilfsmittel aus der Gruppe, die aus Kathetern, Hohlsonden, Schläuchen und Nadeln besteht, ausgewählt werden.

12. Verwendung nach einem der Ansprüche 1 bis 5, wobei das Medizinprodukt aus der Gruppe, die aus invasiven medizinischen Hilfsmitteln, Wundauflagen, Verbänden, zahnärztlichen Abdrücken, Zahnersatz, wie Prothesen, Kronen oder Implantaten, besteht, ausgewählt werden.

13. Verwendung nach Anspruch 12, wobei die invasiven medizinischen Hilfsmittel aus der Gruppe, die aus Kathetern, Hohlsonden, Schläuchen und Nadeln besteht, ausgewählt werden.

## Claims

1. Use of a compound having formula (1) 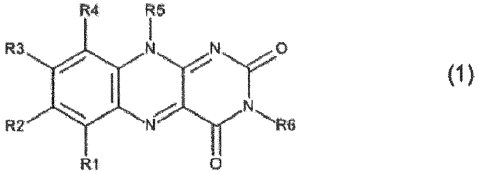 for surface cleaning, in particular of medical devices, food packaging or hygiene items, wherein
A) only 1 radical R1, R2, R3 or R4 is an organic radical having the general formula - (C(D)(E))ₕ-X or -(C(D)(E))ₖ-aryl-(C(D)(E))_{I}-X, wherein h is an integer from 1 to 20 and k, I are each independently an integer from 0 to 6, wherein D and E are - independent of one another - hydrogen, halogen, hydroxyl, O-R^{(VIII)}, wherein R^{(VIII)} is methyl, ethyl, n-propyl, n-butyl, phenyl or benzyl, O-C(=O)-R^{(IX)}, wherein R^{(IX)} is hydrogen, methyl, ethyl, n-propyl, n-butyl, phenyl or benzyl, or thiol, and wherein X is an organic radical having only one quaternary nitrogen atom, and aryl is a substituted or unsubstituted aromatic, or a substituted or unsubstituted heteroaromatic, containing no nitrogen atom, and
wherein the radicals R1, R2, R3 or R4 which are not -(C(D)(E))ₕ-X or - (C(D)(E))ₖ-aryl-(C(D)(E))_{I}-X, may, independently of one another, be identical to or different from one another, and are hydrogen, halogen, hydroxyl, thiol, nitro, carboxylate, aldehyde having 1 to 20 C atoms, ketone having 2 to 20 C atoms, O-alkyl having 1 to 20 C atoms, S-alkyl having 1 to 20 C atoms, O-alkenyl having 2 to 20 C atoms, S-alkenyl having 2 to 20 C atoms, O-aryl having 5 to 20 C atoms, S-aryl having 5 to 20 C atoms, ethers having 2 to 20 C atoms, thioethers having 2 to 20 C atoms, carboxylic acid esters having 1 to 20 C atoms, carboxylic acid amide having 1 to 20 C atoms, thioesters having 1 to 20 C atoms, alkyl having 1 to 20 C atoms, alkenyl having 2 to 20 C atoms, cycloalkyl having 3 to 20 C atoms, cycloalkenyl having 3 to 20 C atoms, aryl having 5 to 20 C atoms or heteroaryl having 4 to 20 C atoms, not containing any N atoms, and wherein each of the radicals R5 or R6 is, independently of one another, identical to or different from one another, and is hydrogen, alkyl having 1 to 20 C atoms, alkenyl having 1 to 20 C atoms, ethers having 2 to 20 C atoms, thioethers having 2 to 20 C atoms, cycloalkyl having 3 to 20 C atoms, cycloalkenyl having 3 to 20 C atoms, aryl having 5 to 20 C atoms or heteroaryl having 4 to 20 C atoms, containing no N atoms, and wherein R5 can be a non-cyclic polyol radical of the general formula -CH₂(CH(OH))_{g}CH₂OH or an ether, ester or acetal thereof, wherein g is an integer from 1 to 10, preferably 1 to 4,
or
wherein B) only 1 radical R5 or R6 is an organic radical of the general formula - (C(D)(E))ₕ-X or - (C(D)(E))ₖ-aryl-(C(D)(E))_{I}-X, wherein h is an integer from 1 to 20 and k, I are independently in each case an integer from 0 to 6, wherein D and E are each independently hydrogen, halogen, hydroxyl, O-R^{(VIII)}, wherein R^{(VIII)} is methyl, ethyl, n-propyl, n-butyl, phenyl or benzyl, O-C(=O)-R^{(IX)}, wherein R^{(IX)} is hydrogen, methyl, ethyl, n-propyl, n-butyl, phenyl or benzyl, or thiol, and wherein X is an organic radical having only one quaternary nitrogen atom and aryl is a substituted or unsubstituted aromatic or a substituted or unsubstituted heteroaromatic, containing no nitrogen atom, and
wherein the radical R5 or R6, which is not -(C(D)(E))ₕ-X or -(C(D)(E))ₖ-aryl-(C(D)(E))_{I}-X, is hydrogen, alkyl having 1 to 20 C atoms, alkenyl having 2 to 20 C atoms, cycloalkyl having 3 to 20 C atoms, ethers having 2 to 20 C atoms, thioethers having 2 to 20 C atoms, cycloalkenyl having 3 to 20 C atoms, aryl having 5 to 20 C atoms or heteroaryl having 4 to 20 C atoms, containing no N atoms, and wherein the radicals R1 to R4 may, independently of one another, be identical to or different from one another, and are hydrogen, halogen, hydroxyl, thiol, nitro, carboxylate, aldehyde having 1 to 20 C atoms, ketone having 2 to 20 C atoms, O-alkyl having 1 to 20 C atoms, S-alkyl having 1 to 20 C atoms, O-alkenyl having 2 to 20 C atoms, S-alkenyl having 2 to 20 C atoms, O-aryl having 5 to 20 C atoms, S-aryl having 5 to 20 C atoms, ethers having 2 to 20 C atoms, thioethers having 2 to 20 C atoms, carboxylic acid esters having 1 to 20 C atoms, carboxylic acid amide having 1 to 20 C atoms, thioester having 1 to 20 C atoms, alkyl having 1 to 20 C atoms, alkenyl having 2 to 20 C atoms, cycloalkyl having 3 to 20 C atoms, cycloalkenyl having 3 to 20 C atoms, aryl having 5 to 20 C atoms or heteroaryl having 4 to 20 C atoms, containing no N atoms, and wherein the radical R5 can be a non-cyclic polyol radical of the general formula -CH₂(CH(OH))_{g}CH₂OH or an ether, ester or acetal thereof, wherein g is an integer from 1 to 10, preferably 1 to 4.

2. Use of a compound of formula (1) 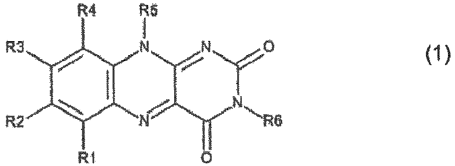 in the surface coating of medical devices, food packaging or hygiene items,
wherein A) only 1 radical R1, R2, R3 or R4 is an organic radical of the general formula - (C(D)(E))ₕ-X or -(C(D)(E))ₖ-aryl-(C(D)(E))_{I}-X, wherein h is an integer from 1 to 20 and k, I are each independently an integer from 0 to 6, wherein D and E are each independently hydrogen, halogen, hydroxyl, O-R^{(VIII)}, wherein R^{(VIII)} is methyl, ethyl, n-propyl, n-butyl, phenyl or benzyl, O-C(=O)-R^{(IX)}, wherein R^{(IX)} is hydrogen, methyl, ethyl, n-propyl, n- butyl, phenyl or benzyl, or thiol, and wherein X is an organic radical having only one quaternary nitrogen atom and aryl is a substituted or unsubstituted aromatic or a substituted or unsubstituted heteroaromatic, containing no nitrogen atom, and
wherein the radicals R1, R2, R3 or R4 which are not -(C(D)(E))ₕ-X or -(C(D)(E))ₖ-aryl-(C(D)(E))_{I}-X, may in each case be identical to or different from one another and are hydrogen, halogen, hydroxyl, thiol, nitro, carboxylate, aldehyde having 1 to 20 C atoms, ketone having 2 to 20 C atoms, O-alkyl having 1 to 20 C atoms, S-alkyl having 1 to 20 C atoms, O-alkenyl having 2 to 20 C atoms, S-alkenyl having 2 to 20 C atoms, O-aryl having 5 to 20 C atoms, S-aryl having 5 to 20 C atoms, ethers having 2 to 20 C atoms, thioethers having 2 to 20 C atoms, carboxylic acid esters having 1 to 20 C atoms, carboxylic acid amide having 1 to 20 C atoms, thioesters having 1 to 20 C atoms, alkyl having 1 to 20 C atoms, alkenyl having 2 to 20 C atoms, cycloalkyl having 3 to 20 C atoms, cycloalkenyl having 3 to 20 C atoms, aryl having 5 to 20 C atoms or heteroaryl having 4 to 20 C atoms, containing no N atoms, and wherein each of the radicals R5 or R6 is identical to or different from one another, and is hydrogen, alkyl having 1 to 20 C atoms, alkenyl having 1 to 20 C atoms, ethers having 2 to 20 C atoms, thioethers having 2 to 20 C atoms, cycloalkyl having 3 to 20 C atoms, cycloalkenyl having 3 to 20 C atoms, aryl having 5 to 20 C atoms or heteroaryl having 4 to 20 C atoms, containing no N atoms and wherein the radical R5 may be a non-cyclic polyol radical of the general formula -CH₂(CH(OH))_{g}CH₂OH or an ether, ester or acetal thereof, wherein g is an integer from 1 to 10, preferably 1 to 4,
or
wherein B) only 1 radical R5 or R6 is an organic radical of the general formula - (C(D)(E))ₕ-X or -(C(D)(E))ₖ-aryl-(C(D)(E))_{I}-X, wherein h is an integer from 1 to 20 and k, I are each - independently of one another - an integer from 0 to 6, wherein D and E are each independently hydrogen, halogen, hydroxyl, O-R^{(VIII)}, wherein R^{(VIII)} is methyl, ethyl, n-propyl, n-butyl, phenyl or benzyl, O-C(=O)-R^{(IX)}, wherein R^{(IX)} is hydrogen, methyl, ethyl, n-propyl, n-butyl, phenyl or benzyl, or thiol, and wherein X is an organic radical having only one quaternary nitrogen atom, and aryl is a substituted or unsubstituted aromatic, or unsubstituted heteroaromatic, containing no nitrogen atom, and
wherein the radical R5 or R6, which is not -(C(D)(E))ₕ-X or -(C(D)(E))ₖ-aryl-(C(D)(E))_{I}-X, is hydrogen, alkyl having 1 to 20 C atoms, alkenyl having 2 to 20 C atoms, cycloalkyl having 3 to 20 C atoms, ethers having 2 to 20 C atoms, thioethers having 2 to 20 C atoms, cycloalkenyl having 3 to 20 C atoms, aryl having 5 to 20 C atoms or heteroaryls having 4 to 20 C atoms containing no N atoms, and wherein the radicals R1 to R4 may each be identical to or different from one another, and are hydrogen, halogen, hydroxyl, thiol, nitro, carboxylate, aldehyde having 1 to 20 C atoms, ketone having 2 to 20 C atoms, O-alkyl having 1 to 20 C atoms, S-alkyl having 1 to 20 C Atoms, O-alkenyl having 2 to 20 C atoms, S-alkenyl having 2 to 20 C atoms, O-aryl having 5 to 20 C atoms, S-aryl having 5 to 20 C atoms, ethers having 2 to 20 C atoms, thioethers having 2 to 20 C atoms, carboxylic acid esters having 1 to 20 C atoms, carboxylic acid amide having 1 to 20 C atoms, thioesters having 1 to 20 C atoms, alkyl having 1 to 20 C atoms, alkenyl having 2 to 20 C atoms, cycloalkyl having 3 to 20 C atoms, cycloalkenyl having 3 to 20 C atoms, aryl having 5 to 20 C atoms or heteroaryl having 4 to 20 C atoms, which does not have N atoms, and R5 can be a non-cyclic polyol radical of the general formula -CH₂(CH(OH))_{g}CH₂OH or an ether, ester or acetal thereof, wherein g is an integer from 1 to 10, preferably 1 to 4.

3. Use in accordance with claim 1 or 2, wherein the radicals R1 and R4, each of which may be identical or different from one another, are hydrogen or methyl, and wherein only 1 radical R2, R3, R5 or R6 is an organic radical of the general formula -(C(D)(E))ₕ-X or -(C(D)(E))ₖ-aryl-(C(D)(E))_{I}-X.

4. Use in accordance with one of claims 1 to 3, wherein X is a radical of the general formula (2): 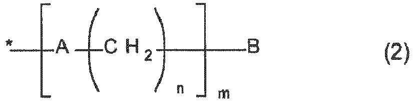 and wherein A is an oxygen or a sulphur atom and wherein n is an integer from 1 to 8, and m is an integer from 0 to 100 and wherein B is a radical of the formula (3), (4a), (4b), (5a) or (5b): 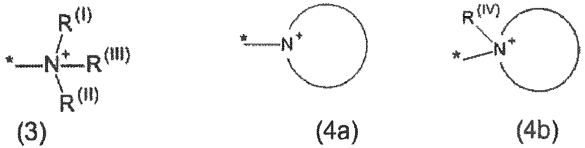 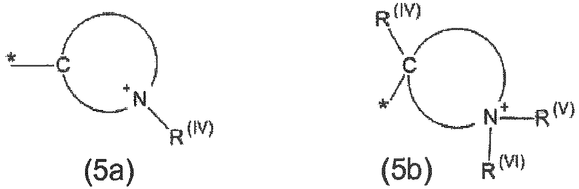 and wherein each of the radicals R^{(I)}, R^{(II)}, R^{(III)}, R^{(IV)} and R^{(V)} is, independently of one another, a C1-C20 aryl, a C1-C20 alkyl which may be straight-chain or branched, or a C1-C20 ether, and wherein the radical of the formula (4a) and the radical of the formula (5a): 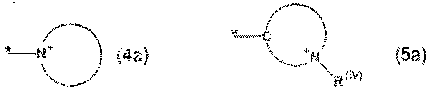 represents a substituted or unsubstituted heterocyclic radical having from 5 to 7 ring atoms, comprising at least one 1 carbon atom and 1 nitrogen atom, as well as optionally 1 or 2 oxygen or sulphur atoms, wherein 1 nitrogen atom forms a double bond, and wherein the radical represented by the formula (4b) and the radical of the formula (5b): 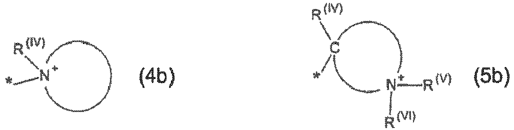 represents a substituted or unsubstituted heterocyclic radical, having from 5 to 7 ring atoms comprising at least one 1 carbon atom and 1 nitrogen atom, and optionally 1 or 2 oxygen or sulphur atoms, wherein 1 nitrogen atom forms a single bond.

5. Use in accordance with any one of claims 1 to 4, wherein the compound of formula (1) is selected from the group consisting of compounds of formulas (30) to (43):

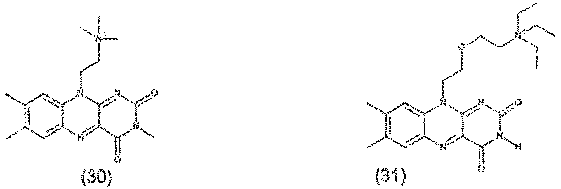

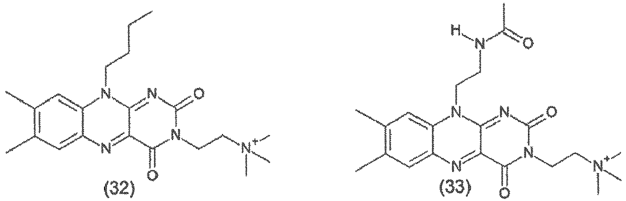

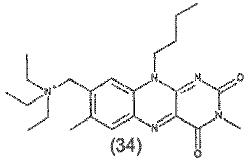

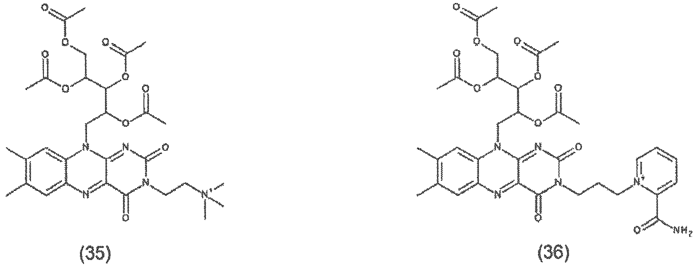

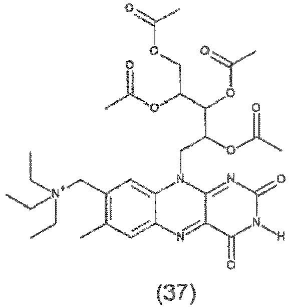

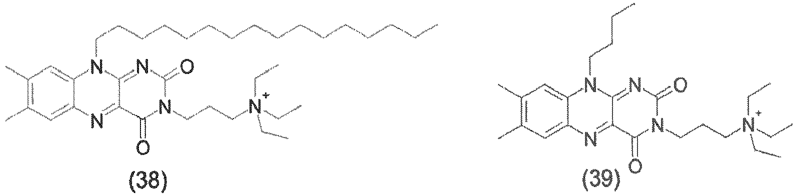

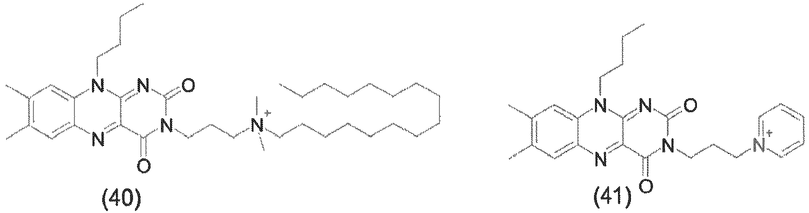

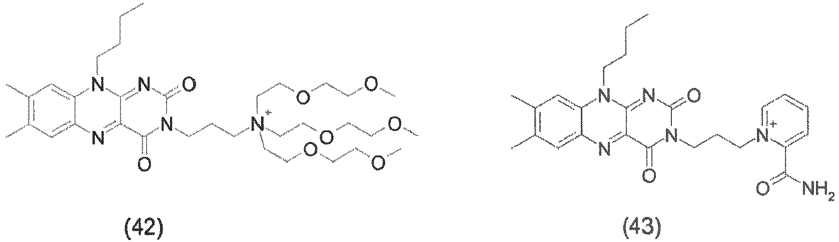

6. Coated object, wherein the object is selected from the group consisting of medical devices, food packaging and hygiene items, and the object is coated with a compound in accordance with formula (1) 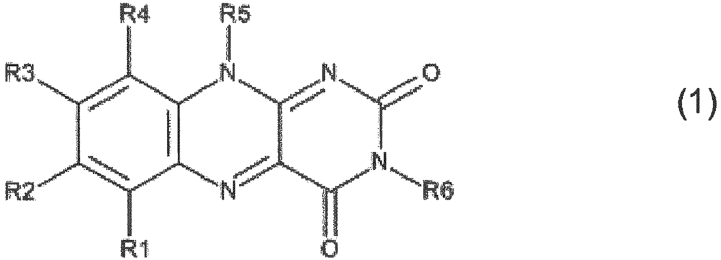
wherein A) only 1 radical R1, R2, R3 or R4 represents an organic radical of the general formula -(C(D)(E))ₕ-X or -(C(D)(E))ₖ-aryl-(C(D)(E))_{I}-X, wherein h is an integer from 1 to 20 and k, I are each independently from one another an integer from 0 to 6, wherein D and E are each independently hydrogen, halogen, hydroxyl, O-R^{(VIII)} wherein R^{(VIII)} is methyl, ethyl, n-propyl, n-butyl, phenyl or benzyl, O-C(=O)-R^{(IX)}, wherein R^{(IX)} is hydrogen, methyl, ethyl, n-propyl, n-butyl, phenyl or benzyl, or thiol, and wherein X is an organic radical having only one quaternary nitrogen atom and aryl is a substituted or unsubstituted aromatic or a substituted or unsubstituted heteroaromatic containing no nitrogen atom, and
wherein the radicals R1, R2, R3 or R4 which are not -(C(D)(E))ₕ-X or -(C(D)(E))ₖ-aryl-(C(D)(E))_{I}-X may each be independently identical to or different from one another and are hydrogen, halogen, hydroxyl, thiol, nitro, carboxylate, aldehyde having 1 to 20 C atoms, ketone having 2 to 20 C atoms, O-alkyl having 1 to 20 C atoms, S-alkyl having 1 to 20 C atoms, O-alkenyl having 2 to 20 C atoms, S-alkenyl having 2 to 20 C atoms, O-aryl having 5 to 20 C atoms, S-aryl having 5 to 20 C atoms, ethers having 2 to 20 C atoms, thioethers having 2 to 20 C atoms, carboxylic acid esters having 1 to 20 C atoms, carboxylic acid amide having 1 to 20 C atoms, thioester having 1 to 20 C atoms, alkyl having 1 to 20 C atoms, alkenyl having 2 to 20 C atoms, cycloalkyl having 3 to 20 C atoms, cycloalkenyl having 3 to 20 C atoms, aryl having 5 to 20 C atoms or heteroaryl having 4 to 20 C atoms, which contains no N atoms, and wherein each of the radicals R5 or R6 is each independently the same as or different from one another and is hydrogen, alkyl having 1 to 20 C atoms, alkenyl having 1 to 20 C atoms, ethers having 2 to 20 C atoms, thioethers having 2 to 20 C atoms, cycloalkyl having 3 to 20 C atoms, cycloalkenyl having 3 to 20 C atoms, aryl having 5 to 20 C atoms or heteroaryl having 4 to 20 C atoms, which contains no N atoms, and wherein the radical R5 may be a non-cyclic polyol radical of the general formula -CH₂(CH(OH))_{g}CH₂OH or an ether, ester or acetal thereof, wherein g is an integer from 1 to 10, preferably 1 to 4,
or
wherein B) only 1 radical R5 or R6 is an organic radical of the general formula -(C(D)(E))ₕ-X or -(C(D)(E))ₖ-aryl-(C(D)(E))_{I}-X, wherein h is an integer from 1 to 20 and k, I each independently are an integer from 0 to 6, wherein D and E are each independently from one another hydrogen, halogen, hydroxyl, O-R^{(VIII)} wherein R^{(VIII)} is methyl, ethyl, n-propyl, n-butyl, phenyl or benzyl, O-C(=O)-R^{(IX)}, wherein R^{(IX} is hydrogen, methyl, ethyl, n-propyl, n-butyl, phenyl or benzyl, or thiol, and wherein X is an organic radical having only one quaternary nitrogen atom and aryl is a substituted or unsubstituted aromatic or a substituted or unsubstituted heteroaromatic containing no nitrogen atom, and
wherein the radical R5 or R6, which is not -(C(D)(E))ₕ-X or -(C(D)(E))ₖ-aryl-(C(D)(E))_{I}-X, is hydrogen, alkyl having 1 to 20 C atoms, alkenyl having 2 to 20 C atoms, cycloalkyl having 3 to 20 C atoms, ethers having 2 to 20 C atoms, thioethers having 2 to 20 C atoms, cycloalkenyl having 3 to 20 C atoms, aryl having 5 to 20 C atoms or heteroaryl having 4 to 20 C atoms, containing no N atoms, and wherein the radical R5 may be a non-cyclic polyol radical of the general formula -CH₂CH(OH))_{g}CH₂OH or an ether, ester or acetal thereof, wherein g is an integer from 1 to 10, preferably 1 to 4, and wherein R1 to R4 may each be the same as or different from each other independently and are hydrogen, halogen, hydroxyl, thiol, nitro, carboxylate, aldehyde having 1 to 20 C atoms, ketone having 2 to 20 C atoms, O-alkyl having 1 to 20 C atoms, S-alkyl having 1 to 20 C atoms , O-alkenyl having 2 to 20 C atoms, S-alkenyl having 2 to 20 C atoms, O-Aryl having 5 to 20 C atoms, S-aryl having 5 to 20 C atoms, ethers having 2 to 20 C atoms, thioethers having 2 to 20 C atoms, carboxylic acid esters having 1 to 20 C atoms, carboxylic acid amide having 1 to 20 C atoms, thioesters having 1 to 20 C atoms, alkyl having 1 to 20 C atoms, alkenyl having 2 to 20 C atoms, cycloalkyl having 3 to 20 C atoms, cycloalkenyl having 3 to 20 C atoms, aryl having 5 to 20 C atoms or heteroaryl having 4 to 20 C atoms, which contains no N atoms, and wherein the radical R5 is a non-cyclic polyol radical of the general formula -CH₂(CH(OH))_{g}CH₂OH or may be an ether, ester or acetal thereof, wherein g is an integer from 1 to 10, preferably 1 to 4.

7. Coated object in accordance with claim 6, wherein the radicals R1 and R4, which may in each case independently from one another be identical or different, are hydrogen or methyl, and wherein only 1 radical R2, R3, R5 or R6 is an organic radical of the general formula - (C(D)(E))ₕ-X or -(C(D)(E))ₖ-aryl-(C(D)(E))_{I}-X.

8. Coated object in accordance with one of claims 6 or 7, wherein X is a radical of the general formula (2): 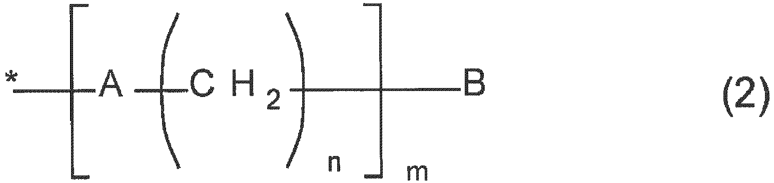 and wherein A is an oxygen or a sulphur atom and wherein n is an integer from 1 to 8, and m is an integer from 0 to 100, and wherein B is a radical of the formula (3), (4a), (4b), (5a) or (5b): 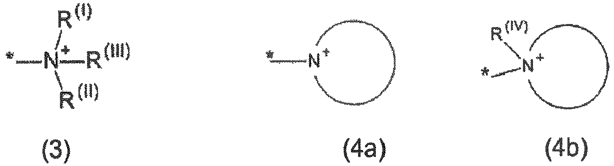 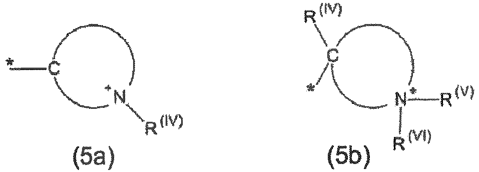 and wherein each of the radicals R^{(I)}, R^{(II)}, R^{(III)}, R^{(IV)} and R^{(V)} independently of one another is a C1-C20 aryl, a C1-C20 alkyl which may be straight-chain or branched, or a C1-C20 ether, and wherein the radical having the formal (4a) and the radical having the formula (5a): 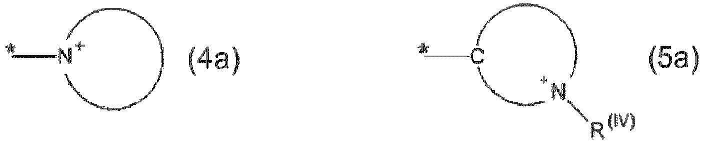 represents a substituted or unsubstituted heterocyclic radical having 5 to 7 ring atoms, which comprise at least one 1 carbon atom and 1 nitrogen atom as well as optionally 1 or 2 oxygen or sulphur atoms, wherein 1 nitrogen atom forms a double bond, and wherein the radical having the formula (4b) and the radical having the formula (5b): 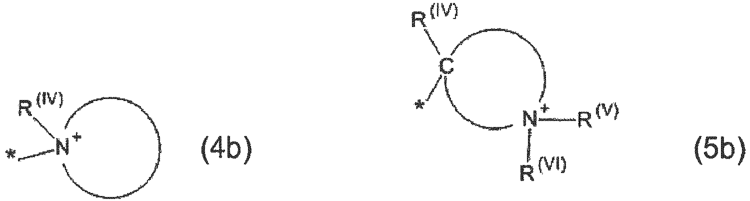 represents a substituted or unsubstituted heterocyclic radical having 5 to 7 ring atoms, which comprise at least 1 carbon atom and 1 nitrogen atom, as well as optionally 1 or 2 oxygen or sulphur atoms, wherein 1 nitrogen atom forms a single bond.

9. Coated object in accordance with any one of claims 6 to 8, wherein the compound having the formula (1) is selected from the group consisting of compounds of the formulas (30) to (43):

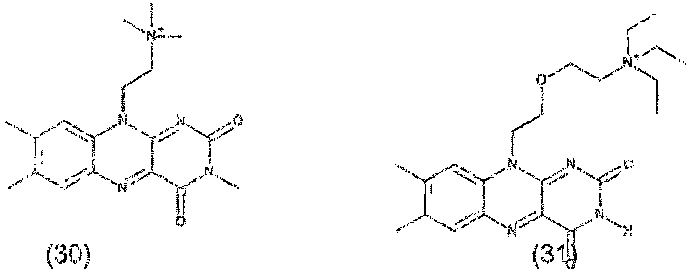

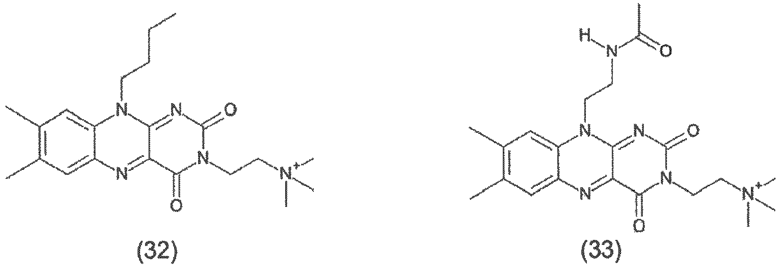

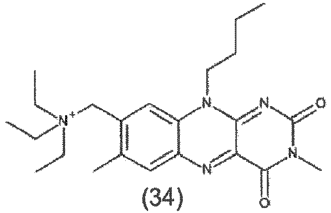

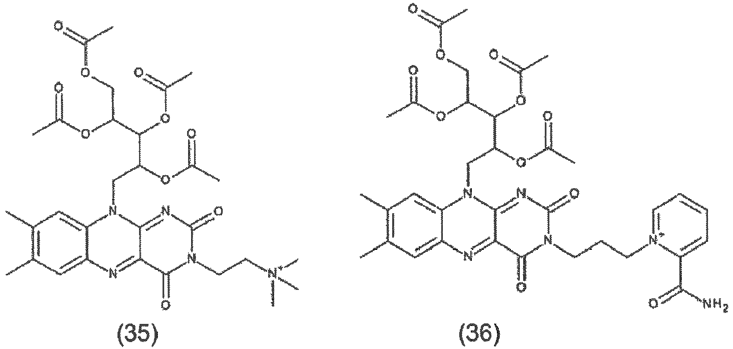

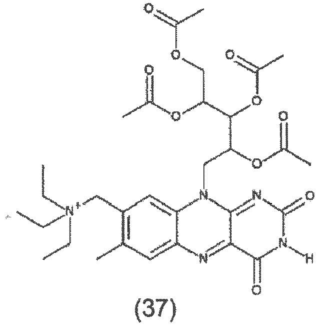

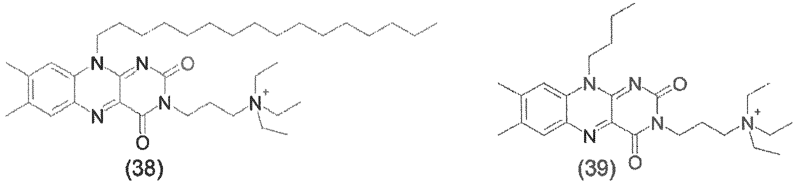

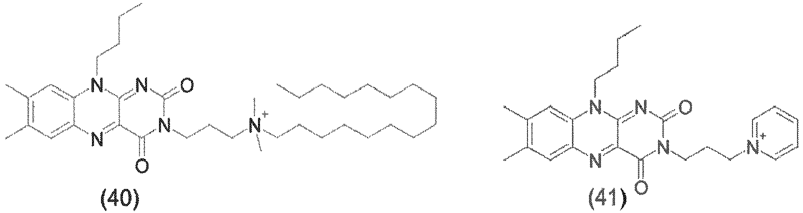

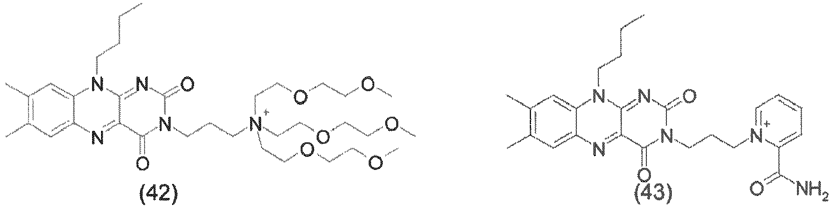

10. Coated object in accordance with any one of claims 6 to 9, wherein the medical device is selected from the group consisting of invasive medical products, wound dressings, bandages, dental impressions, dentures such as prostheses, crowns or implants.

11. Coated object in accordance with claim 10, wherein the invasive medical products are selected from the group consisting of catheters, hollow probes, tubes and needles.

12. Use of any one of claims 1 to 5, wherein the medical device is selected from the group consisting of invasive medical products, wound dressings, bandages, dental impressions, dentures such as prostheses, crowns or implants.

13. Use according to claim 12, wherein the invasive medical products are selected from the group consisting of catheters, hollow probes, tubes and needles.

## Revendications

1. Utilisation d'un composé de formule (1) 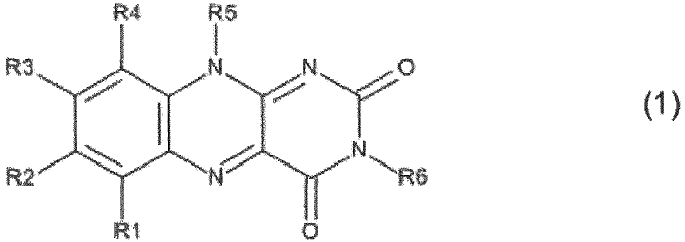 pour le nettoyage de surfaces, en particulier de dispositifs médicaux, d'emballages de produits alimentaires ou d'articles hygiéniques, dans laquelle
A) seul un radical R1, R2, R3 ou R4 est un radical organique de formule générale -(C(D)(E))ₕ-X ou (C(D)(E))ₖ-aryl-(C(D)(E))ₗ-X, dans laquelle h est un nombre entier de 1 à 20, et k, l représentent chacun indépendamment de l'autre un nombre entier de 0 à 6, dans laquelle D et E représentent chacun indépendamment de l'autre un atome d'hydrogène, un groupe halogéno, hydroxyle, O-R^{(VIII)}, dans laquelle R^{(VIII)} représente un groupe méthyle, éthyle, n-propyle, n-butyle, phényle ou benzyle, O-C(=O)-R^{(IX)}, dans laquelle R^{(IX)} représente un atome d'hydrogène, un groupe méthyle, éthyle, n-propyle, n-butyle, phényle ou benzyle, ou thiol, et dans laquelle X représente un radical organique n'ayant qu'un atome d'azote quaternaire et aryle un radical aromatique substitué ou non substitué ou hétéroaromatique substitué ou non substitué, qui ne contient pas d'atome d'azote, et
dans laquelle les radicaux R1, R2, R3 ou R4 qui ne sont pas -(C(D)(E))ₕ-X ou (C(D)(E))ₖ-aryl-(C(D)(E))ₗ-X peuvent chacun indépendamment des autres être identiques ou différents, et représentent chacun un atome d'hydrogène, un groupe halogéno, hydroxyle, thiol, nitro, carboxylate, aldéhyde ayant 1 à 20 atomes de carbone, cétone ayant 2 à 20 atomes de carbone, O-alkyle ayant 1 à 20 atomes de carbone, S-alkyle ayant 1 à 20 atomes de carbone, O-alcényle ayant 2 à 20 atomes de carbone, S-alcényle ayant 2 à 20 atomes de carbone, O-aryle ayant 5 à 20 atomes de carbone, S-aryle ayant 5 à 20 atomes de carbone, éther ayant 2 à 20 atomes de carbone, thioéther ayant 2 à 20 atomes de carbone, ester d'acide carboxylique ayant 1 à 20 atomes de carbone, carboxamide ayant 1 à 20 atomes de carbone, thioester ayant 1 à 20 atomes de carbone, alkyle ayant 1 à 20 atomes de carbone, alcényle ayant 2 à 20 atomes de carbone, cycloalkyle ayant 3 à 20 atomes de carbone, cycloalcényle ayant 3 à 20 atomes de carbone, aryle ayant 5 à 20 atomes de carbone ou hétéroaryle ayant 4 à 20 atomes de carbone, qui ne contient aucun atome d'azote, et chacun des radicaux R5 ou R6 est, indépendamment de l'autre, identique ou différent, et représente un atome d'hydrogène, un groupe alkyle ayant 1 à 20 atomes de carbone, alcényle ayant 1 à 20 atomes de carbone, éther ayant 2 à 20 atomes de carbone, thioéther ayant 2 à 20 atomes de carbone, cycloalkyle ayant 3 à 20 atomes de carbone, cycloalcényle ayant 3 à 20 atomes de carbone, aryle ayant 5 à 20 atomes de carbone ou hétéroaryle ayant 4 à 20 atomes de carbone, qui ne contient aucun atome d'azote, et R5 peut être un radical polyol acyclique de formule générale - CH₂(CH(OH))_{g}CH₂OH ou un éther, un ester ou un acétate de celui-ci, g pouvant être un nombre entier de 1 à 10, de préférence de 1 à 4,
ou dans laquelle
B) seul un radical R5 ou R6 représente un radical organique de formule générale -(C(D)(E))ₕ-X ou (C(D)(E))ₖ-aryl-(C(D)(E))ₗ-X, dans laquelle h est un nombre entier de 1 à 20 et k, l représentent chacun indépendamment de l'autre un nombre entier de 0 à 6, dans laquelle D et E représentent chacun indépendamment de l'autre un atome d'hydrogène, un groupe halogéno, hydroxyle, O-R^{(VIII)}, dans laquelle R^{(VIII)} représente un groupe méthyle, éthyle, n-propyle, n-butyle, phényle ou benzyle, O-C(=O)-R^{(IX)}, dans laquelle R^{(IX)} représente un atome d'hydrogène, un groupe méthyle, éthyle, n-propyle, n-butyle, phényle ou benzyle, ou thiol, et dans laquelle X représente un radical organique n'ayant qu'un atome d'azote quaternaire et aryle un radical aromatique substitué ou non substitué ou hétéroaromatique substitué ou non substitué, qui ne contient aucun atome d'azote,
et
le radical R5 ou R6 qui n'est pas -(C(D)(E))ₕ-X ou (C(D)(E))ₖ-aryl-(C(D)(E))ₗ-X représente un atome d'hydrogène, un groupe alkyle ayant 1 à 20 atomes de carbone, alcényle ayant 2 à 20 atomes de carbone, cycloalkyle ayant 3 à 20 atomes de carbone, éther ayant 2 à 20 atomes de carbone, thioéther ayant 2 à 20 atomes de carbone, cycloalcényle ayant 3 à 20 atomes de carbone, aryle ayant 5 à 20 atomes de carbone ou hétéroaryle ayant 4 à 20 atomes de carbone, qui ne contient aucun atome d'azote, et les radicaux R1 à R4 peuvent chacun indépendamment des autres être identiques ou différents, et représentent chacun un atome d'hydrogène, un groupe halogéno, hydroxyle, thiol, nitro, carboxylate, aldéhyde ayant 1 à 20 atomes de carbone, cétone ayant 2 à 20 atomes de carbone, O-alkyle ayant 1 à 20 atomes de carbone, S-alkyle ayant 1 à 20 atomes de carbone, O-alcényle ayant 2 à 20 atomes de carbone, S-alcényle ayant 2 à 20 atomes de carbone, O-aryle ayant 5 à 20 atomes de carbone, S-aryle ayant 5 à 20 atomes de carbone, éther ayant 2 à 20 atomes de carbone, thioéther ayant 2 à 20 atomes de carbone, ester d'acide carboxylique ayant 1 à 20 atomes de carbone, carboxamide ayant 1 à 20 atomes de carbone, thioester ayant 1 à 20 atomes de carbone, alkyle ayant 1 à 20 atomes de carbone, alcényle ayant 2 à 20 atomes de carbone, cycloalkyle ayant 3 à 20 atomes de carbone, cycloalcényle ayant 3 à 20 atomes de carbone, aryle ayant 5 à 20 atomes de carbone ou hétéroaryle ayant 4 à 20 atomes de carbone qui ne contient aucun atome d'azote, et le radical R5 peut être un radical polyol acyclique de formule générale -CH₂(CH(OH))_{g}CH₂OH ou un éther, un ester ou un acétal de celui-ci, g pouvant être un nombre entier de 1 à 10, de préférence de 1 à 4.

2. Utilisation d'un composé de formule (1) 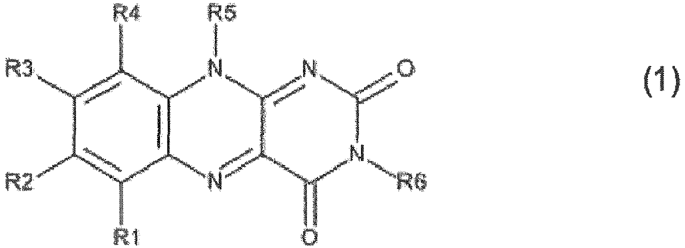 pour le revêtement de la surface de dispositifs médicaux, d'emballages de produits alimentaires ou d'articles hygiéniques,
dans laquelle A) seul un radical R1, R2, R3 ou R4 est un radical organique de formule générale -(C(D)(E))ₕ-X ou (C(D)(E))ₖ-aryl-(C(D)(E))ₗ-X, dans laquelle h est un nombre entier de 1 à 20, et k, l représentent chacun indépendamment de l'autre un nombre entier de 0 à 6, dans laquelle D et E représentent chacun indépendamment de l'autre un atome d'hydrogène, un groupe halogéno, hydroxyle, 0-R^{(VIII)}, dans laquelle R^{(VIII)} représente un groupe méthyle, éthyle, n-propyle, n-butyle, phényle ou benzyle, O-C(=O)-R^{(IX)}, dans laquelle R^{(IX)} représente un atome d'hydrogène, un groupe méthyle, éthyle, n-propyle, n-butyle, phényle ou benzyle, ou thiol, et dans laquelle X représente un radical organique n'ayant qu'un atome d'azote quaternaire et aryle un radical aromatique substitué ou non substitué ou hétéroaromatique substitué ou non substitué, qui ne contient pas d'atome d'azote, et
dans laquelle les radicaux R1, R2, R3 ou R4 qui ne sont pas -(C(D)(E))ₕ-X ou (C(D)(E))ₖ-aryl-(C(D)(E))ₗ-X peuvent chacun indépendamment des autres être identiques ou différents, et représentent chacun un atome d'hydrogène, un groupe halogéno, hydroxyle, thiol, nitro, carboxylate, aldéhyde ayant 1 à 20 atomes de carbone, cétone ayant 2 à 20 atomes de carbone, O-alkyle ayant 1 à 20 atomes de carbone, S-alkyle ayant 1 à 20 atomes de carbone, O-alcényle ayant 2 à 20 atomes de carbone, S-alcényle ayant 2 à 20 atomes de carbone, O-aryle ayant 5 à 20 atomes de carbone, S-aryle ayant 5 à 20 atomes de carbone, éther ayant 2 à 20 atomes de carbone, thioéther ayant 2 à 20 atomes de carbone, ester d'acide carboxylique ayant 1 à 20 atomes de carbone, carboxamide ayant 1 à 20 atomes de carbone, thioester ayant 1 à 20 atomes de carbone, alkyle ayant 1 à 20 atomes de carbone, alcényle ayant 2 à 20 atomes de carbone, cycloalkyle ayant 3 à 20 atomes de carbone, cycloalcényle ayant 3 à 20 atomes de carbone, aryle ayant 5 à 20 atomes de carbone ou hétéroaryle ayant 4 à 20 atomes de carbone, qui ne contient aucun atome d'azote, et chacun des radicaux R5 ou R6 est, indépendamment de l'autre, identique ou différent, et représente un atome d'hydrogène, un groupe alkyle ayant 1 à 20 atomes de carbone, alcényle ayant 1 à 20 atomes de carbone, éther ayant 2 à 20 atomes de carbone, thioéther ayant 2 à 20 atomes de carbone, cycloalkyle ayant 3 à 20 atomes de carbone, cycloalcényle ayant 3 à 20 atomes de carbone, aryle ayant 5 à 20 atomes de carbone ou hétéroaryle ayant 4 à 20 atomes de carbone, qui ne contient aucun atome d'azote, et R5 peut être un radical polyol acyclique de formule générale - CH₂(CH(OH))_{g}CH₂OH ou un éther, un ester ou un acétate de celui-ci, g pouvant être un nombre entier de 1 à 10, de préférence de 1 à 4,
ou dans laquelle
B) seul un radical R5 ou R6 représente un radical organique de formule générale -(C(D)(E))ₕ-X ou (C(D)(E))ₖ-aryl-(C(D)(E))ₗ-X, dans laquelle h est un nombre entier de 1 à 20 et k, l représentent chacun indépendamment de l'autre un nombre entier de 0 à 6, dans laquelle D et E représentent chacun indépendamment de l'autre un atome d'hydrogène, un groupe halogéno, hydroxyle, O-R^{(VIII)}, dans laquelle R^{(VIII)} représente un groupe méthyle, éthyle, n-propyle, n-butyle, phényle ou benzyle, O-C(=O)-R^{(IX)}, dans laquelle R^{(IX)} représente un atome d'hydrogène, un groupe méthyle, éthyle, n-propyle, n-butyle, phényle ou benzyle, ou thiol, et dans laquelle X représente un radical organique n'ayant qu'un atome d'azote quaternaire et aryle un radical aromatique substitué ou non substitué ou hétéroaromatique substitué ou non substitué, qui ne contient aucun atome d'azote,
et
dans laquelle le radical R5 ou R6 qui n'est pas -(C(D)(E))ₕ-X ou (C(D)(E))ₖ-aryl-(C(D)(E))ₗ-X représente un atome d'hydrogène, un groupe alkyle ayant 1 à 20 atomes de carbone, alcényle ayant 2 à 20 atomes de carbone, cycloalkyle ayant 3 à 20 atomes de carbone, éther ayant 2 à 20 atomes de carbone, thioéther ayant 2 à 20 atomes de carbone, cycloalcényle ayant 3 à 20 atomes de carbone, aryle ayant 5 à 20 atomes de carbone ou hétéroaryle ayant 4 à 20 atomes de carbone, qui ne contient aucun atome d'azote, et les radicaux R1 à R4 peuvent chacun indépendamment des autres être identiques ou différents, et représentent chacun un atome d'hydrogène, un groupe halogéno, hydroxyle, thiol, nitro, carboxylate, aldéhyde ayant 1 à 20 atomes de carbone, cétone ayant 2 à 20 atomes de carbone, O-alkyle ayant 1 à 20 atomes de carbone, S-alkyle ayant 1 à 20 atomes de carbone, O-alcényle ayant 2 à 20 atomes de carbone, S-alcényle ayant 2 à 20 atomes de carbone, O-aryle ayant 5 à 20 atomes de carbone, S-aryle ayant 5 à 20 atomes de carbone, éther ayant 2 à 20 atomes de carbone, thioéther ayant 2 à 20 atomes de carbone, ester d'acide carboxylique ayant 1 à 20 atomes de carbone, carboxamide ayant 1 à 20 atomes de carbone, thioester ayant 1 à 20 atomes de carbone, alkyle ayant 1 à 20 atomes de carbone, alcényle ayant 2 à 20 atomes de carbone, cycloalkyle ayant 3 à 20 atomes de carbone, cycloalcényle ayant 3 à 20 atomes de carbone, aryle ayant 5 à 20 atomes de carbone ou hétéroaryle ayant 4 à 20 atomes de carbone qui ne contient aucun atome d'azote, et le radical R5 peut être un radical polyol acyclique de formule générale - CH₂(CH(OH))_{g}CH₂OH ou un éther, un ester ou un acétal de celui-ci, g pouvant être un nombre entier de 1 à 10, de préférence de 1 à 4.

3. Utilisation selon la revendication 1 ou 2, dans laquelle les radicaux R1 à R4, qui peuvent chacun indépendamment des autres être identiques ou différents, représentent chacun un atome d'hydrogène ou un groupe méthyle, et seul un radical R2, R3, R5 ou R6 est un radical organique de formule générale - (C(D)(E))ₕ-X ou (C(D)(E))ₖ-aryl-(C(D)(E))ₗ-X.

4. Utilisation selon l'une des revendications 1 à 3, dans laquelle X est un radical de formule générale (2) 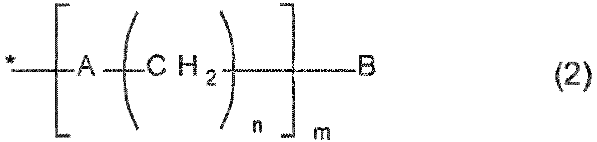 et dans laquelle A représente un atome d'oxygène ou un atome de soufre, et dans laquelle n est un nombre entier de 1 à 8 et m un nombre entier de 0 à 100, et B est un radical de formule (3), (4a), (4b), (5a) ou (5b) : 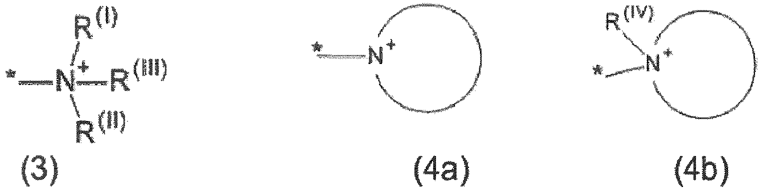 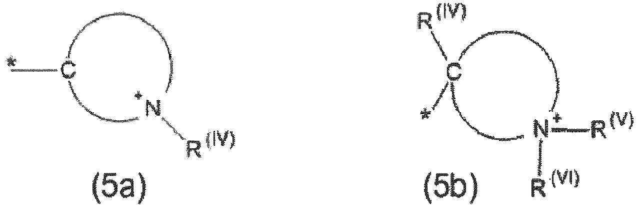 et dans laquelle chacun des radicaux R^{(I)}, R^{(II)}, R^{(III)}, R^{(IV)} et R^{(V)} représente indépendamment des autres un groupe aryle en C1 à C20, alkyle en C1 à C20, qui peut avoir une chaîne droite ou ramifiée, ou un éther en C1 à C20, et le radical de formule (4a) et le radical de formule (5a) 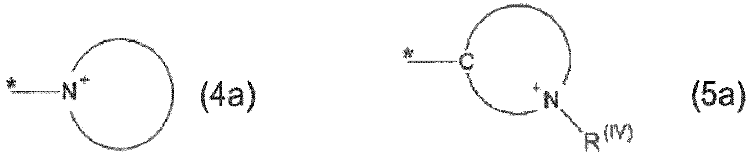 représentent un radical hétérocyclique substitué ou non substitué ayant 5 à 7 atomes nucléaires, qui comprennent au moins un atome de carbone et un atome d'azote, ainsi qu'en option 1 ou 2 atomes d'oxygène ou de soufre, un atome d'azote formant une double liaison, et le radical de formule (4b) et le radical de formule (5b) 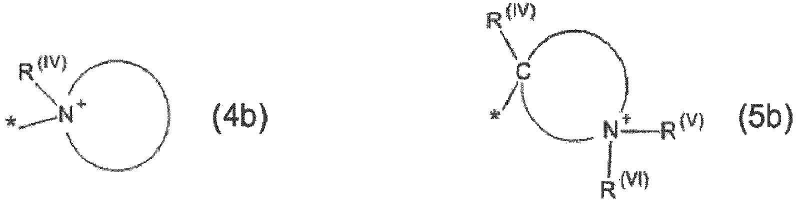 représentent chacun un radical hétérocyclique substitué ou non substitué ayant 5 à 7 atomes nucléaires, qui comprennent au moins un atome de carbone et un atome d'azote, ainsi qu'en option 1 ou 2 atomes d'oxygène ou de soufre, 1 atome d'azote formant une liaison simple.

5. Utilisation selon l'une des revendications 1 à 4, dans laquelle le composé de formule (1) est choisi dans le groupe consistant en les composés ayant les formules (30) à (43) :

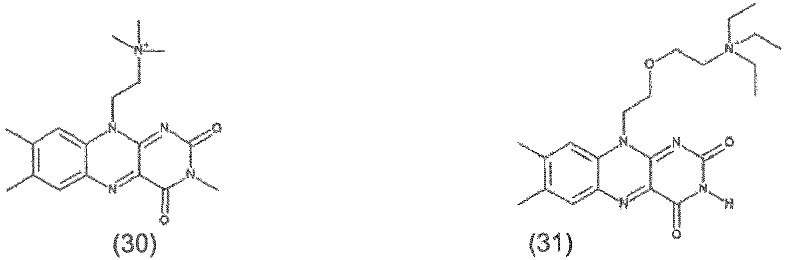

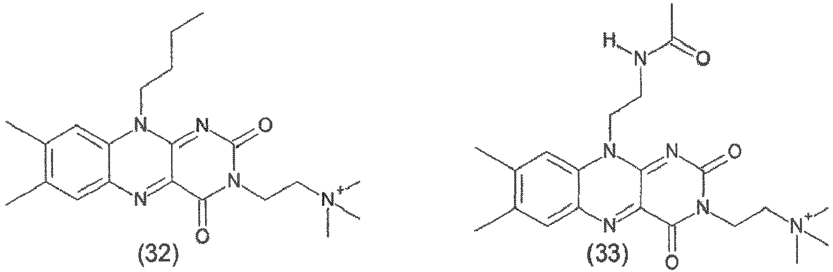

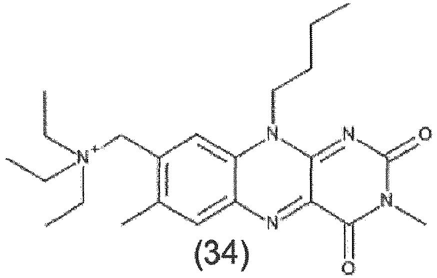

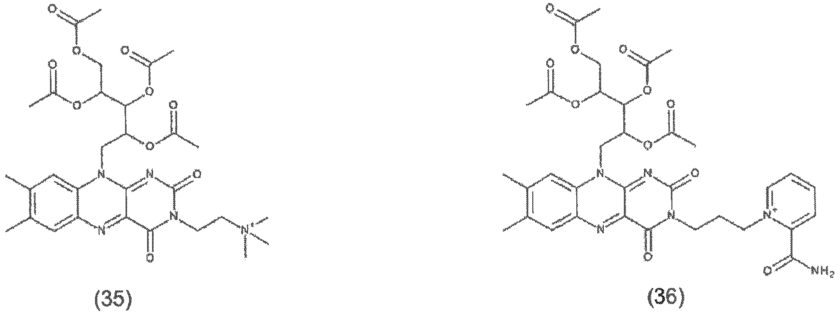

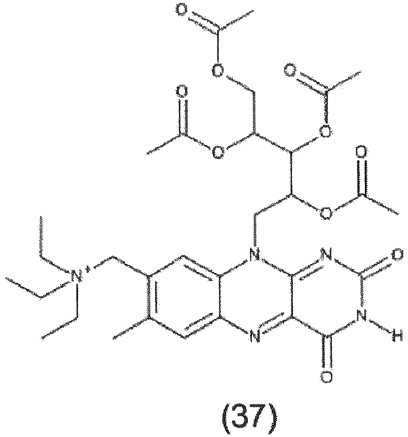

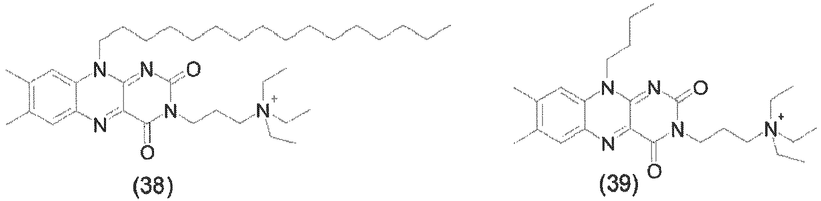

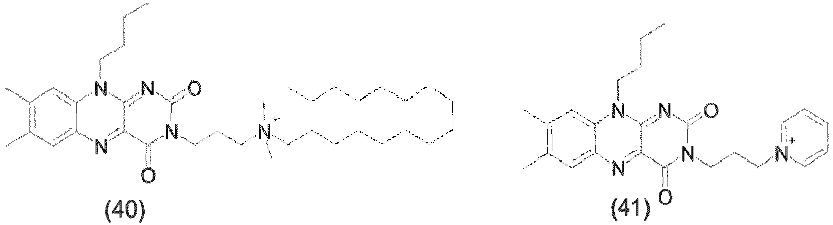

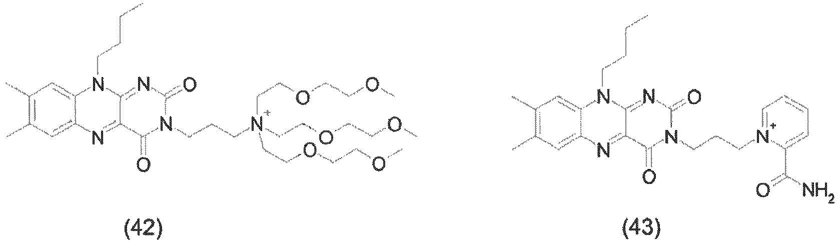

6. Objet revêtu, l'objet étant choisi dans le groupe consistant en les dispositifs médicaux, les emballages de produits alimentaires et les articles hygiéniques, et l'objet étant revêtu d'un composé de formule (1) 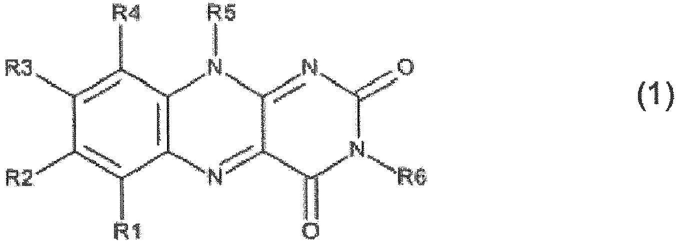 dans laquelle
A) seul un radical R1, R2, R3 ou R4 est un radical organique de formule générale -(C(D)(E))ₕ-X ou (C(D)(E))ₖ-aryl-(C(D)(E))ₗ-X, dans laquelle h est un nombre entier de 1 à 20, et k, l représentent chacun indépendamment de l'autre un nombre entier de 0 à 6, dans laquelle D et E représentent chacun indépendamment de l'autre un atome d'hydrogène, un groupe halogéno, hydroxyle, O-R^{(VIII)}, dans laquelle R^{(VIII)} représente un groupe méthyle, éthyle, n-propyle, n-butyle, phényle ou benzyle, O-C(=O)-R^{(IX)}, dans laquelle R^{(IX)} représente un atome d'hydrogène, un groupe méthyle, éthyle, n-propyle, n-butyle, phényle ou benzyle, ou thiol, et dans laquelle X représente un radical organique n'ayant qu'un atome d'azote quaternaire et aryle un radical aromatique substitué ou non substitué ou hétéroaromatique substitué ou non substitué, qui ne contient pas d'atome d'azote, et
dans laquelle les radicaux R1, R2, R3 ou R4 qui ne sont pas -(C(D)(E))ₕ-X ou (C(D)(E))ₖ-aryl-(C(D)(E))ₗ-X peuvent chacun indépendamment des autres être identiques ou différents, et représentent chacun un atome d'hydrogène, un groupe halogéno, hydroxyle, thiol, nitro, carboxylate, aldéhyde ayant 1 à 20 atomes de carbone, cétone ayant 2 à 20 atomes de carbone, O-alkyle ayant 1 à 20 atomes de carbone, S-alkyle ayant 1 à 20 atomes de carbone, O-alcényle ayant 2 à 20 atomes de carbone, S-alcényle ayant 2 à 20 atomes de carbone, O-aryle ayant 5 à 20 atomes de carbone, S-aryle ayant 5 à 20 atomes de carbone, éther ayant 2 à 20 atomes de carbone, thioéther ayant 2 à 20 atomes de carbone, ester d'acide carboxylique ayant 1 à 20 atomes de carbone, carboxamide ayant 1 à 20 atomes de carbone, thioester ayant 1 à 20 atomes de carbone, alkyle ayant 1 à 20 atomes de carbone, alcényle ayant 2 à 20 atomes de carbone, cycloalkyle ayant 3 à 20 atomes de carbone, cycloalcényle ayant 3 à 20 atomes de carbone, aryle ayant 5 à 20 atomes de carbone ou hétéroaryle ayant 4 à 20 atomes de carbone, qui ne contient aucun atome d'azote, et chacun des radicaux R5 ou R6 est, indépendamment de l'autre, identique ou différent, et représente un atome d'hydrogène, un groupe alkyle ayant 1 à 20 atomes de carbone, alcényle ayant 1 à 20 atomes de carbone, éther ayant 2 à 20 atomes de carbone, thioéther ayant 2 à 20 atomes de carbone, cycloalkyle ayant 3 à 20 atomes de carbone, cycloalcényle ayant 3 à 20 atomes de carbone, aryle ayant 5 à 20 atomes de carbone ou hétéroaryle ayant 4 à 20 atomes de carbone, qui ne contient aucun atome d'azote, et R5 peut être un radical polyol acyclique de formule générale - CH₂(CH(OH))_{g}CH₂OH ou un éther, un ester ou un acétate de celui-ci, g pouvant être un nombre entier de 1 à 10, de préférence de 1 à 4,
ou dans laquelle
B) seul un radical R5 ou R6 représente un radical organique de formule générale -(C(D)(E))ₕ-X ou (C(D)(E))ₖ-aryl-(C(D)(E))ₗ-X, dans laquelle h est un nombre entier de 1 à 20 et k, l représentent chacun indépendamment de l'autre un nombre entier de 0 à 6, dans laquelle D et E représentent chacun indépendamment de l'autre un atome d'hydrogène, un groupe halogéno, hydroxyle, O-R^{(VIII)}, dans laquelle R^{(VIII)} représente un groupe méthyle, éthyle, n-propyle, n-butyle, phényle ou benzyle, O-C(=O)-R^{(IX)}, dans laquelle R^{(IX)} représente un atome d'hydrogène, un groupe méthyle, éthyle, n-propyle, n-butyle, phényle ou benzyle, ou thiol, et dans laquelle X représente un radical organique n'ayant qu'un atome d'azote quaternaire et aryle un radical aromatique substitué ou non substitué ou hétéroaromatique substitué ou non substitué, qui ne contient aucun atome d'azote,
et dans laquelle
le radical R5 ou R6 qui n'est pas -(C(D)(E))ₕ-X ou (C(D)(E))ₖ-aryl-(C(D)(E))ₗ-X représente un atome d'hydrogène, un groupe alkyle ayant 1 à 20 atomes de carbone, alcényle ayant 2 à 20 atomes de carbone, cycloalkyle ayant 3 à 20 atomes de carbone, éther ayant 2 à 20 atomes de carbone, thioéther ayant 2 à 20 atomes de carbone, cycloalcényle ayant 3 à 20 atomes de carbone, aryle ayant 5 à 20 atomes de carbone ou hétéroaryle ayant 4 à 20 atomes de carbone, qui ne contient aucun atome d'azote, et le radical R5 peut être un radical polyol acyclique de formule générale -CH₂(CH(OH))_{g}CH₂OH- ou un éther, un ester ou un acétal de celui-ci, g pouvant être un nombre entier de 1 à 10, de préférence de 1 à 4, et les radicaux R1 à R4 peuvent chacun indépendamment des autres être identiques ou différents, et représentent chacun un atome d'hydrogène, un groupe halogéno, hydroxyle, thiol, nitro, carboxylate, aldéhyde ayant 1 à 20 atomes de carbone, cétone ayant 2 à 20 atomes de carbone, O-alkyle ayant 1 à 20 atomes de carbone, S-alkyle ayant 1 à 20 atomes de carbone, O-alcényle ayant 2 à 20 atomes de carbone, S-alcényle ayant 2 à 20 atomes de carbone, O-aryle ayant 5 à 20 atomes de carbone, S-aryle ayant 5 à 20 atomes de carbone, éther ayant 2 à 20 atomes de carbone, thioéther ayant 2 à 20 atomes de carbone, ester d'acide carboxylique ayant 1 à 20 atomes de carbone, carboxamide ayant 1 à 20 atomes de carbone, thioester ayant 1 à 20 atomes de carbone, alkyle ayant 1 à 20 atomes de carbone, alcényle ayant 2 à 20 atomes de carbone, cycloalkyle ayant 3 à 20 atomes de carbone, cycloalcényle ayant 3 à 20 atomes de carbone, aryle ayant 5 à 20 atomes de carbone ou hétéroaryle ayant 4 à 20 atomes de carbone qui ne contient aucun atome d'azote, et le radical R5 peut être un radical polyol acyclique de formule générale -CH₂(CH(OH))_{g}CH₂OH ou un éther, un ester ou un acétal de celui-ci, g pouvant être un nombre entier de 1 à 10, de préférence de 1 à 4.

7. Objet revêtu selon la revendication 6, dans lequel les radicaux R1à R4, qui chacun indépendamment des autres peuvent être identiques ou différents, représentent chacun un atome d'hydrogène ou un groupe méthyle, et seul un radical R2, R3, R5 ou R6 représente un radical organique de formule générale - (C(D)(E))ₕ-X ou (C(D)(E))ₖ-aryl-(C(D)(E))ₗ-X.

8. Objet revêtu selon l'une des revendications 6 ou 7, dans lequel X est un radical de formule générale (2) 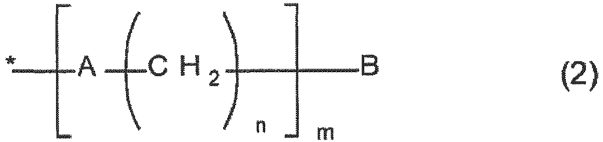 et dans laquelle A représente un atome d'oxygène ou un atome de soufre, et dans laquelle n est un nombre entier de 1 à 8 et m un nombre entier de 0 à 100, et dans laquelle B est un radical de formule (3), (4a), (4b), (5a) ou (5b) : 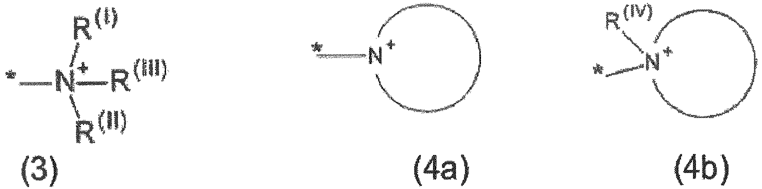 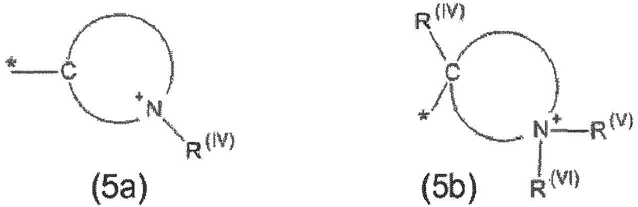 et dans laquelle chacun des radicaux R^{(I)}, R^{(II)}, R^{(III)}, R^{(IV)} et R^{(V)} représente indépendamment des autres un groupe aryle en C1 à C20, alkyle en C1 à C20, qui peut avoir une chaîne droite ou ramifiée, ou un éther en C1 à C20, et le radical de formule (4a) et le radical de formule (5a) 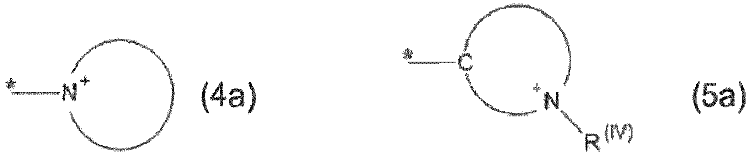 représentent un radical hétérocyclique substitué ou non substitué ayant 5 à 7 atomes nucléaires, qui comprennent au moins un atome de carbone et un atome d'azote, ainsi qu'en option 1 ou 2 atomes d'oxygène ou de soufre, un atome d'azote formant une double liaison, et le radical de formule (4b) et le radical de formule (5b) 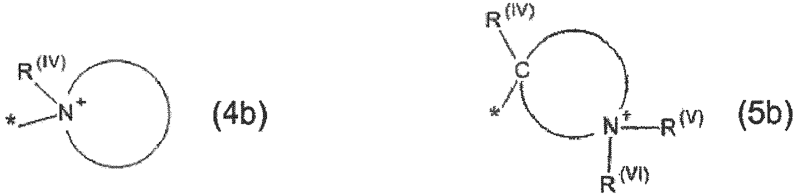 représentent chacun un radical hétérocyclique substitué ou non substitué ayant 5 à 7 atomes nucléaires, qui comprennent au moins un atome de carbone et un atome d'azote, ainsi qu'en option 1 ou 2 atomes d'oxygène ou de soufre, 1 atome d'azote formant une liaison simple.

9. Objet revêtu selon l'une des revendications 6 à 8, dans lequel le composé de formule (1) est choisi dans le groupe consistant en les composés ayant les formules (30) à (43) :

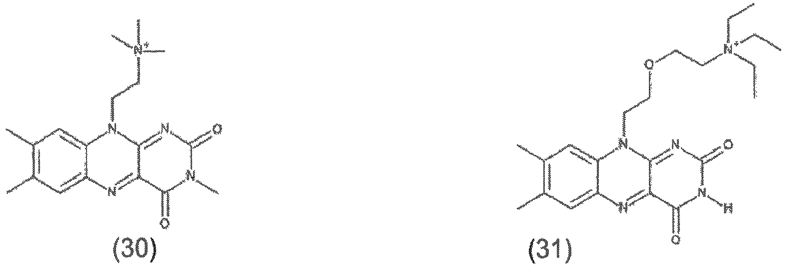

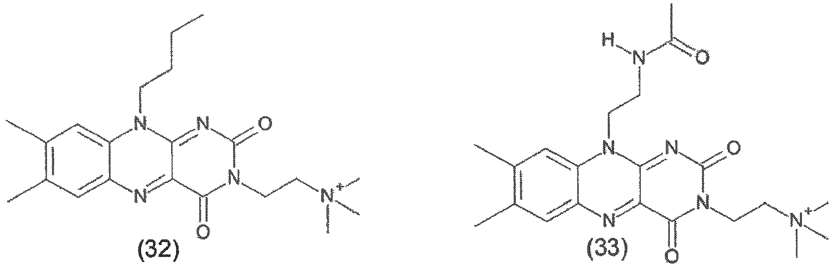

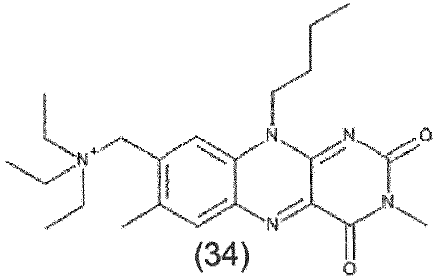

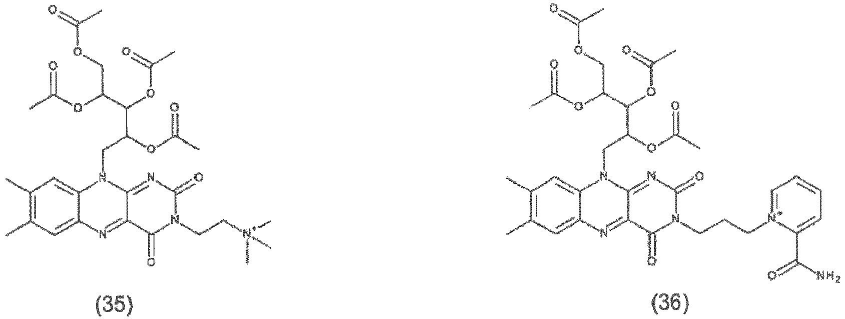

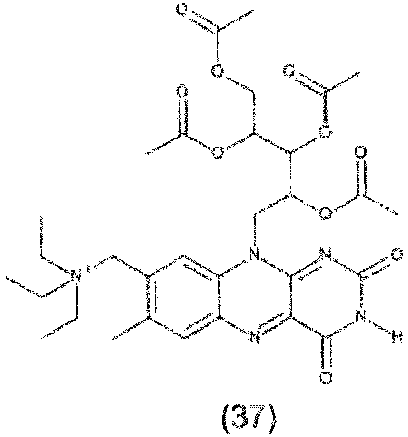

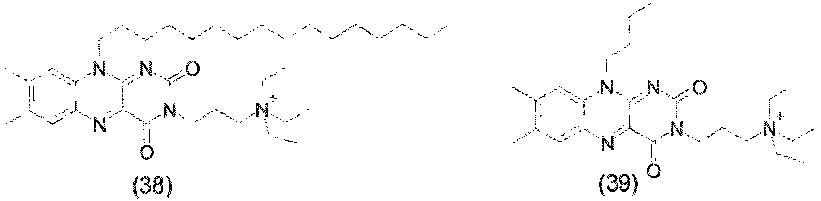

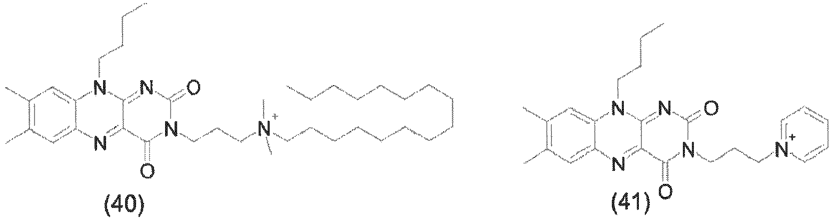

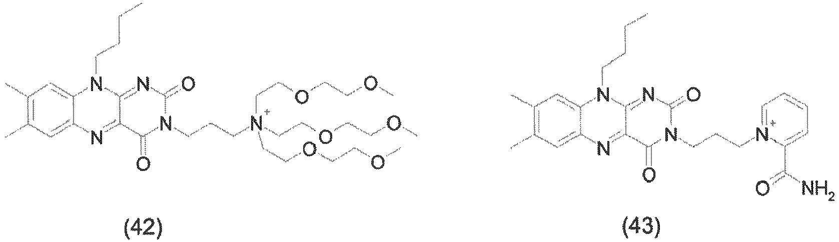

10. Objet revêtu selon l'une des revendications 6 à 9, le dispositif médical étant choisi dans le groupe consistant en les auxiliaires médicaux invasifs, les pansements, les bandages, les empreintes dentaires, les prothèses dentaires, telles que les prothèses, les couronnes ou les implants.

11. Objet revêtu selon la revendication 10, l'auxiliaire médical invasif étant choisi dans le groupe consistant en les cathéters, les sondes creuses, les tubes flexibles et les aiguilles.

12. Utilisation selon l'une des revendications 1 à 5, dans laquelle le dispositif médical est choisi dans le groupe consistant en les auxiliaires médicaux invasifs, les pansements, les bandages, les empreintes dentaires, les prothèses dentaires, telles que les prothèses, les couronnes ou les implants.

13. Utilisation selon la revendication 12, dans laquelle les auxiliaires médicaux invasifs sont choisis dans le groupe consistant en les cathéters, les sondes creuses, les tubes flexibles et les aiguilles.
